# EUROPEAN PATENT APPLICATION

(11) **EP 4 702 950 A1**
(43) Date of publication of application: **04.03.2026**
(21) Application number: 24795811.9
(22) Date of filing: 07.04.2024
(51) Int. Cl.: A61F 2/24, A61F 2/95

(54) **CONTROL MECHANISM, CONTROL HANDLE, DELIVERY SYSTEM AND CONTROL METHOD FOR ARTIFICIAL IMPLANT, AND ARTIFICIAL HEART VALVE**

(30) Priority: 26.04.2023 CN 202310470457
(71) Applicant: Venus MedTech (HangZhou) Inc., Hangzhou, Zhejiang 310051 (CN); Hangzhou Qianjiang Medical and Industrial Cross Innovation Technology Research Institute, Hangzhou, Zhejiang 310052 (CN)
(72) Inventor: LEI, Rongjun, Hangzhou, Zhejiang 310051 (CN); CHEN, Wei, Hangzhou, Zhejiang 310051 (CN); YANG, Lingfeng, Hangzhou, Zhejiang 310051 (CN); WANG, Xiang, Hangzhou, Zhejiang 310051 (CN)
(74) Representative: Metida
(86) International application number: PCT/CN2024/086423
(87) International publication number: WO 2024/222430

(57) **Abstract**

Disclosed **in** this application are a control mechanism, a control handle, a delivery system and a control method for an artificial implant, and an artificial heart valve. The control mechanism includes a base, a pulling wire, and a locking member, wherein the pulling wire is configured with a free end which can pass through the artificial implant or be detached from the artificial implant, the locking member movably engaged with the base, and the locking member cooperates with the free end of the pulling wire to lock the artificial implant. After the control mechanism is optimized, the control of the release or withdrawal process of the artificial implant in the body can be achieved by means of mutual cooperation. Compared with existing structures, the control mechanism is more suitable for working inside the body.

## Description

### TECHNICAL FIELD

This application relates to the technical field of medical devices, and in particular to a control mechanism, a control handle, a delivery system and a control method for an artificial implant, and an artificial heart valve.

### BACKGROUND

With advances in medical technology, artificial implants have been used to treat heart valve disorders. Common treatments generally include surgical valve repair or replacement, or the use of flexible catheters for implantation.

In the interventional techniques, the artificial implant in a compressed and loaded state is mounted on the distal region of a catheter assembly and delivered to a desired location. After radial expansion and release of the artificial implant, the catheter assembly is withdrawn from the body.

For the connection between the artificial implant and the catheter assembly, in the existing technologies, methods such as direct engagement or wire control may be used. The wire-control method uses a flexible member to restrain the artificial implant to the distal region of the catheter assembly and lock the flexible member, maintaining the artificial implant in the compressed state. To release the artificial implant, the flexible member is unlocked, allowing it to loosen and separate from the artificial implant, completing the release of the artificial implant. However, existing methods for locking the flexible member still needs to be improved.

Furthermore, the existing artificial implant includes an outer frame and an inner frame for clamping the original aortic valve, wherein the outer frame is mostly formed seperately, which causes many inconveniences in both surgical operation and positioning.

### SUMMARY

This application provides a control mechanism for an artificial implant, which makes the control of a flexible member (including locking and unlocking) more reasonable.

This application provides a control mechanism for an artificial implant, including:
a base;
a pulling wire having a free end capable of passing through the artificial implant or being detached from the artificial implant;
a locking member movably engaged with the base, the locking member cooperating with the free end of the pulling wire to lock the artificial implant.

The following provides several optional embodiments, which are not intended to be additional limitations of the above-described overall solution but merely to supplement or optimize the above-described overall solution. Assuming there are no technical or logical inconsistencies, each optional embodiment may be combined with the above-described overall solution, or multiple optional embodiments may be combined with each other.

This application also provides a control mechanism for an artificial implant, including:
a base having a locking region;
a pulling wire having a free end that can pass through the artificial implant or be detached from the artificial implant;
a locking member rotatably engaged with the base, the locking member having a positioning portion that moves into or out of the locking region during rotation, the positioning portion cooperating with the free end of the pulling wire to lock the artificial implant.

Optionally, the pulling wire has a control end opposite to the free end, and the control end is movable relative to the base. When the free end is in a first state, the length of the pulling wire exposed outside the base can be adjusted by operating the control end. When the free end is in a second state, the control end can be operated to drive the pulling wire away from the artificial implant.

Optionally, there are multiple pulling wires, the frees end of the pulling wires move independently, while the control ends of the pulling wires move synchronously.

Optionally, the control mechanism further includes a second shaft, and the control ends of the pulling wires are connected to the second shaft.

Optionally, there are multiple locking regions, and, the free end of each pulling wire in the first state corresponds to one of the locking regions.

Optionally, the free end has a wire loop, and when the free end is in the first state, the positioning portion is inserted into the wire loop; and when the free end is in the second state, the positioning portion is disengaged from the wire loop.

Optionally, the wire loop is formed independently or formed by winding the pulling wire.

This application also provides a control mechanism for an artificial implant, including:
a base having a locking region;
a pulling wire having a free end that can pass through the artificial implant or be detached from the artificial implant;
a locking member rotatably engaged with the base, the locking member having a positioning portion that moves into or out of the locking region during rotation, the positioning portion cooperating with the free end of the pulling wire to lock the artificial implant;
wherein the artificial implant has an axial direction, one end of the artificial implant in the axial direction is configured as a wire control end, the wire control end defines holes for extending of the pulling wire; and the artificial implant has the following states based on its degree of deformation:
a folding state, in which the wire control end radially contracts toward the base; and
an expanding state, in which the wire control end radially expands away from the base;
wherein a round-trip path of the same pulling wire winding around the artificial implant is not repeated.

Optionally, the free end of the pulling wire has a first state in which it is restrained to the base and a second state in which it is released from restraint. When the engagement between the locking member and the free end of the pulling wire is released, the restraint on the artificial implant is released.

Optionally, when the artificial implant is in the folding state and the expanding state, the free end of the pulling wire is in the first state.

Optionally, the artificial implant includes a plurality of hollow portions which define the holes and are arranged. In the expanding state, one pulling wire passes through at least two holes.

Optionally, the number of the holes is twice the number of the pulling wires. In the expanding state, one pulling wire corresponds to two holes.

Optionally, the round-trip path of the same pulling wire through the artificial implant is substantially triangular.

Optionally, in the first state, the pulling wire extends out of the base through a current locking region, passes through the artificial implant, and then its free end returns back to the same locking region.

Optionally, the positioning portion is a multi-turn helical structure with axial gaps defined between adjacent turns. In the first state, the pulling wire extends out of the base through a current axial gap, passes through the artificial implant, and then loops over a turn adjacent to the current axial gap.

The base has a distal region and a proximal region opposite to each other, and an axial direction extending between the distal and proximal regions. An interior of the base defines a first cavity, a first opening communicating with the first cavity is defined in a proximal region side of the base, and a second opening communicating with the first cavity is defined in an outer circumference of the base;

the pulling wire passed through the first cavity, one end of the pulling wire extending proximally out of the base through the first opening, and the other end, i.e., the free end of the pulling wire extending out of the base through the second opening for connecting the artificial implant.

Optionally, the locking region is located at the second opening.

Optionally, there are multiple locking regions arranged at intervals along the circumferential direction of the base, and the positioning portion sequentially enters or exits each locking region as the locking member moves.

Optionally, the locking member is rotatably engaged with the base to drive the positioning portion sequentially to enter or exit each locking region.

Optionally, there are multiple second openings arranged at intervals along the circumferential direction of the base, and ribs are formed between adjacent second openings.

Optionally, in the first state, the free end extends out of the base through a corresponding second opening, winds around and passes around the artificial implant, and then returns to the locking region corresponding to the same second opening, or returns to the locking region corresponding to an adjacent second opening.

Optionally, all ribs are converged and fixed at the distal region of the base.

Optionally, all ribs are converged to form an annular-shaped structure.

Optionally, the base defines a locking hole for insertion of the positioning portion.

Optionally, an open side of the locking hole faces the circumferential direction of the base.

Optionally, the locking hole is defined on at least one of the ribs.

Optionally, in the first state, the free end of the pulling wire is located in the current locking region, and two ends of the positioning portion located in the current locking region are respectively inserted into the locking holes on corresponding sides.

Optionally, when the positioning portion moves in a first direction, the free end switches from the second state to the first state; the locking hole extends through the rib in the first direction, and has a forward opening and rearward opening opposite to each other, and the forward opening facing the first direction.

Optionally, there are multiple ribs configured with locking holes, and the positioning portion in whole has a tail end and head end opposite to each other, and the head end is sequentially inserted into and disengaged from the each locking hole as the locking member rotates.

Optionally, all ribs are configured with locking holes.

Optionally, one axial section of the rib is configured as a curved section extending radially inwardly, and the locking holes is defined in the curved section of the corresponding rib.

Optionally, there are one or multiple locking holes defined in the same rib.

Optionally, the multiple locking holes are arranged sequentially along an extension direction of the rib.

Optionally, one side of the rib facing the first cavity is configured as an inner side, and the inner side of each rib is configured with a guide groove for extending of the positioning portion. Optionally, the positioning portion has a curved shape and extends around an axis of the base, and the positioning portion is inserted into and disengaged from the locking hole as the locking member rotates.

Optionally, at least a part of the locking member is configured as a connecting portion positioned within the first cavity, and the control mechanism further includes a first shaft in transmission engagement with the connecting portion for driving the locking member to rotate relative to the base.

Optionally, the pulling wire includes a control end opposite the free end, the control end is movable relative to the base; and the control mechanism further includes a second shaft connected to the control end, and the second shaft is tubular and movably mounted around the first shaft.

Optionally, the positioning portion includes at least one arc-shaped structure matching with the base.

Optionally, the positioning portion is configured as a helical structure.

Optionally, the helical structure is wound at least once.

Optionally, the helical structure includes multiple turns arranged along the axial direction.

Optionally, along a winding direction of the helical structure, the positioning portion as a whole includes a tail end and a head end opposite to each other, and one of the tail and head ends is fixed to the connecting portion.

Optionally, the tail end of the positioning portion is fixed to the connecting portion.

Optionally, the connecting portion is tubular, and the tail end of the positioning portion is wound around and fixed to an outer circumference or a distal region of the connecting portion.

Optionally, the connecting portion is fixed to a distal region of the first shaft, or the connecting portion is axially separable from the distal region of the first shaft.

Optionally, the positioning portion is a multi-turn helical structure with axial gaps between adjacent turns. The helical structure encloses an inner space, and at least one section of the pulling wire is a detour section. In the first state, the detour section is located within the inner space, and two ends of the detour section extend outside the inner space through corresponding axial gaps.

Optionally, in the first state, the pulling wire extends distally from the control end to the outside of the positioning portion, bends through a second axial gap to the inside of the positioning portion, then extends from the second axial gap to the distal first axial gap, passes through the first axial gap from the inside to the outside, and bends to the outside of the positioning portion, and the free end is sleeved onto the positioning portion after winding around and passing through the artificial implant.

Optionally, the free end is sleeved onto a turn of the positioning portion adjacent to the first axial gap or the second axial gap.

This application also provides a control mechanism having a distal region and a proximal region opposite to each other and an axial direction extending between the proximal and distal regions. The control mechanism includes:
an intervention component, including a first intervention component and a second intervention component;
a transmission member, a distal region of which is fixedly connected to the first intervention component;
a control handle, connected to and controlling a proximal region of the transmission member; and
a linkage structure, including two selectively linked mating portions, one of which is fixed to the second intervention component and the other is fixed to the transmission member, such that a linkage state of the two mating portions is switched during movement of the transmission member.

Optionally, along the axial direction, the first intervention component is located at a side of the second intervention component (either the distal region or the proximal region).

Optionally, there is a position between the first intervention component and the second intervention component where they are partially nested relative to each other.

Optionally, the first and second intervention components each include:
an inner shaft assembly for carrying an artificial heart valve before release;
a sheath tube capable of encasing the artificial heart valve before release;
a base connected to the artificial heart valve before release, and the base and the artificial heart valve being positioned axially relative to each other;
a locking member for retaining the heart valve before release against the base; and
a pulling wire connected to the artificial heart valve to control the release of the artificial heart valve.

Optionally, the control mechanism includes a balloon capable of carrying and expanding the artificial heart valve before release.

Optionally, when the transmission member rotates, the two mating portions are linked or unlinked.

Optionally, when the transmission member slides axially, the two mating portions are linked or unlinked.

Optionally, when linked, the two mating portions drive the second intervention component to rotate or move linearly.

Optionally, the two mating portions include a linkage key and a linkage groove that are axially slidable to separate, and the linkage key is rotatably linked with the linkage groove when the linage key is inserted into the linkage groove.

Optionally, the two mating portions include a linkage key and a linkage groove that are rotatable to each other to separate, and the linkage key is axially linked with the linkage groove when the linage key is inserted into the linkage groove.

Optionally, there are multiple linkage keys connected to each other via a tubular component, and the multiple linkage keys are arranged circumferentially along the tubular component.

Optionally, there are multiple linkage grooves connected to each other via a tubular component, and the multiple linkage grooves are arranged circumferentially along the tubular component.

Optionally, the linkage key is set at the proximal region or distal region of the connecting portion.

Optionally, at least a part of the locking member is configured as the connecting portion, and the control mechanism further includes a transmission member that engages with the connecting portion to drive the locking member to rotate relative to the base.

The connecting portion is tubular, and a pipe wall of the connecting portion is configured with a rotary linkage structure that engages with the distal region of the transmission member. When the transmission member moves axially relative to the connecting portion, the rotary linkage structure is engaged or disengaged.

Optionally, the connecting portion is located within the base.

Optionally, the rotary linkage structure includes:
a linkage key radially projecting from one of the connecting portion and the transmission member;
a linkage groove cooperating with the linkage key being provided on the other of the connecting portion and the transmission member, the linkage groove being open at an end surface of the other of the connecting portion and the transmission member.

Optionally, the linkage key is formed by deformation of a part of a pipe wall of the connecting portion.

Optionally, a part of a pipe wall of the connecting portion is concaved to form the linkage key, and an avoidance zone is formed outside the linkage key.

Optionally, a pipe wall of the connecting portion is provided with a pair of cutouts adjacent to a proximal region face, and a radial portion between the pair of cutouts bends inward to form the linkage key.

Optionally, there is at least one linkage key, for example, there are two to four linkage key distributed circumferentially.

Optionally, the positioning portion is a multi-turn helical structure which encloses an interior space, at least a section of the pulling wire extends into the inner space, and an outer circumference of the connecting portion has a radial concave and an avoidance zone corresponding to the position of the detour section.

Optionally, the transmission member is a tubular-shaped first shaft, and the linkage groove is formed in a pipe wall at the distal region of the transmission member.

Optionally, the linkage groove has a flared opening.

This application also provides a control mechanism having a distal region and a proximal region opposite to each other, including:
at least three shafts, including a first shaft, a second shaft, and a third shaft slidably sleeved sequentially from the inside to the outside;
a base connected to a distal region of the third shaft;
a pulling wire connected to a distal region of the second shaft, the pulling wire having a free end that can pass through the artificial implant or be detached from the artificial implant; and
a locking member connected to a distal region of the first shaft, the locking member movably matched with the base, and the locking member cooperating with the free end of the pulling wire to lock the artificial implant.

This application also provides a delivery system including a control handle and the aforementioned control mechanism, wherein the proximal regions of the three shafts are respectively connected to and controlled by the control handle.

This application also provides a control method for an artificial implant based on wire control, including:
providing the aforementioned control mechanism, wherein the free end of a pulling wire passes through the artificial implant and is restrained to the base by the locking member;
wherein the pulling wire has a control end opposite the free end, when the artificial implant is released, the control end is moved to gradually extend a portion of the pulling wire exposed outside the base, allowing the artificial implant to deform radially;
when the artificial implant has deformed to a desired extent, the cooperation between the locking member and the base is released and the free end is thus unlocked; and
moving the control end to withdraw the free end from the artificial implant.

This application provides a control handle for operating a catheter assembly to deploy an artificial implant. The control handle has a distal region and a proximal region opposite to each other and an axial direction extending therebetween; the catheter assembly includes a sheath tube and an inner shaft assembly that slidably disposed within the sheath tube; the artificial implant before release is connected to the inner shaft assembly and wrapped by the sheath tube; during release, the artificial implant is exposed outside the sheath tube;
wherein the control handle includes:
a first handle for connecting to the sheath tube; and
a second handle for connecting to the inner shaft assembly, a distal region of the second handle being movably inserted into a proximal region of the first handle; wherein
before release of the artificial implant, the control handle has a first length; during release of the artificial implant, the second handle further extends into the interior of the first handle to expose the artificial implant outside the sheath tube, and the control handle accordingly has a second length that is less than the first length.

Optionally, the distal region of the second handle is provided a first transmission member that includes:
a first support member to which the proximal region of the sheath tube is fixed; and
a first drive sleeve rotatably mounted around the first support member and threadedly engaged with the first transmission member.

This application also provides a control handle for operating a catheter assembly to deploy an artificial implant. The control handle has a distal region and a proximal region opposite to each other, and an axial direction extending between the proximal and distal regions; the catheter assembly includes a sheath tube and an inner shaft assembly that slidably disposed within the sheath tube; the artificial implant before release is connected to the inner shaft assembly and wrapped by the sheath tube; during release, the artificial implant is exposed outside the sheath;
wherein the control handle includes:
a first handle connected to the sheath tube; and
a second handle including a second support body and a first transmission member, wherein the first transmission member is axially slidably mounted on the first handle, and the distal region of the second support body rotatably matches with and axially links with the first transmission member;
the proximal region of the inner shaft assembly is connected to the second support body, and the distal region of the inner shaft assembly is connected to a wire control assembly for controlling the release of the artificial implant.

Optionally, the inner shaft assembly includes multiple shafts slidably mounted from the inside to the outside. The wire control assembly includes a base, a locking member, and a pulling wire, each of which is connected to a corresponding shaft in a transmission manner. The locking member is movably mounted on the base, and the base is provided with a locking hole that mates with the locking member. One end of the pulling wire is connected to the corresponding shaft, and the other end can pass through the artificial implant and then be restrained to the base by the locking member.

Optionally, the inner shaft assembly includes:
a first shaft, a distal region of which is linked to the locking member and a proximal region of which is movably mounted to the second support body;
a second shaft, a distal region of which is connected to the pulling wire and a proximal region of which is movably mounted to the second support body; and
a third shaft, a distal region of which is fixed to the base and a proximal region of which is fixed to the second support body.

Optionally, the distal region of the sheath tube is provided with a first loading section for enclosing the artificial implant, the first loading section being open toward the distal region.

Optionally, a second loading section is fixed to the distal region of the first shaft, the second loading section being open toward the proximal region and interlocking with the first loading section to enclose the artificial implant.

Optionally, the first shaft is selectively linked with the locking member via a linkage structure, and the linkage structure switches between different linkage states during axial movement of the first shaft.

Optionally, the first handle includes a first support body and a drive sleeve rotatably mounted around the first support body;
a guide groove being provided within the first support body and extending axially, and the first transmission member being slidably mounted within the guide groove and threadedly engaged with the inner wall of the drive sleeve;
a connecting head being provided on the distal region of the second support body, and the first transmission member defining a mounting hole, the connecting head inserted into the mounting hole and rotatably engaged with the mounting hole, and matching structures which are rotationally engaged and axially linked being provided between the connecting head and the inner wall of the mounting hole, engagement structure.

Optionally, a connecting head being provided on the distal region of the second support body, the proximal region of the third shaft is fixed to the connecting head, and the second and first shafts extend through connecting head via the interior of the third shaft.

This application also provides a control handle for operating a catheter assembly to deploy an artificial implant. The control handle has a distal region and a proximal region opposite to each other, and an axial direction extending between the proximal and distal regions; the catheter assembly includes a sheath tube and an inner shaft assembly that slidably disposed within the sheath tube; the artificial implant before release is connected to the inner shaft assembly and wrapped by the sheath tube; during release, the artificial implant is exposed outside the sheath;
wherein the control handle includes:
a first handle for connecting the sheath tube; and
a second handle for connecting the inner shaft assembly, wherein the distal region of the second handle is configured as an insertion section that is movably inserted into the first handle, when the second handle is moved distally relative to the first handle to a limit position, the insertion section is located within the first handle; the inner shaft assembly includes a movable shaft that slidably disposed within the second handle, and the proximal region of the movable shaft has a motion path extending into the interior of the insertion section.

Optionally, an outer wall of the insertion section is provided with distance markings.

This application also provides a control handle for operating a catheter assembly to deploy an artificial implant. The control handle has a distal region and a proximal region opposite to each other, and an axial direction extending between the proximal and distal regions; the catheter assembly includes a sheath tube and an inner shaft assembly that slidably disposed within the sheath tube; the artificial implant before release is connected to the inner shaft assembly and wrapped by the sheath tube; during release, the artificial implant is exposed outside the sheath;
wherein the control handle includes:
a first handle for connecting the sheath tube;
a second handle including a second support body and an inner shaft drive mechanism, the second support body including a sleeve and a guide rail fixed within the sleeve, the distal region of the sleeve being movably inserted into the interior of the first handle, and a support frame being fixed to the proximal region of the sleeve;
wherein the inner shaft drive mechanism includes:
   a plurality of inner cylinders being nested sequentially from the inside to the outside, each cylinder being rotatably mounted in the radial gap between the sleeve and the guide rail, the proximal region of each inner cylinder extending beyond the sleeve and serving as a force-applying component for driving this inner cylinder, the force-applying components being arranged at intervals along the axial direction through the support frame;
   a plurality of second transmission members being axially arranged and slidably mounted on the guide rail, each second transmission member being threadedly engaged with a corresponding inner cylinder, the inner shaft assembly including a plurality of movable shafts, and the proximal region of each movable shaft being axially linked to the second transmission member.

Optionally, the support frame is provided with multiple partition plates which are arranged circumferentially, and mounting zones for mounting the force-applying components are defined between adjacent partition plates.

Optionally, the support frame has a cylindrical outer profile, and within each mounting zone, the sidewall of the cylindrical structure is provided with a plurality of openings arranged at intervals in the circumferential direction to expose the force-applying components, and connecting plates are provided between adjacent openings and connected between the partition plates.

Optionally, the force-applying component and the inner cylinder are formed separately and then fixedly connected together.

This application also provides a control handle for operating a catheter assembly to deploy an artificial implant. The control handle has a distal region and a proximal region opposite to each other, and an axial direction extending between the proximal and distal regions; the catheter assembly includes a sheath tube and an inner shaft assembly that slidably disposed within the sheath tube; the artificial implant before release is connected to the inner shaft assembly and wrapped by the sheath tube; during release, the artificial implant is exposed outside the sheath;
wherein the control handle includes:
a first handle connected to the sheath tube; and
a second handle including a second support body and an inner shaft drive mechanism, wherein the second support body includes a sleeve and a guide rail fixed within the sleeve, and the distal region of the sleeve is movably inserted into the interior of the first handle;
wherein the inner shaft drive mechanism includes:
   an inner shaft, being rotatably mounted in a radial gap between the sleeve and the guide rail, the proximal region of the inner shaft extending beyond the sleeve and serving as a force-applying component for driving the inner shaft;
   a second transmission member, being slidably mounted on the guide rail and threadedly engaged with the inner shaft, wherein the inner shaft assembly includes at least one movable shaft, the proximal region of which is axially linked to the second transmission member.

Optionally, the distal region of the sleeve is provided with a connecting head, the first transmission member defines a mounting hole, the connecting head is inserted into the mounting hole and rotatably engages therein, and limiting structures that cooperates with each other to maintain axial linkage are provided between the connecting head and the inner wall of the mounting hole.

Optionally, the second handle further includes a first transmission member slidably disposed within the first handle, and the distal region of the sleeve is provided with a connecting head that rotatably matches with and axially engages with the first transmission member.

Optionally, the guide rail includes two strips arranged side by side, at least a part of the second transmission member is positioned between the two strips, and the distal regions of the two strips are fixed to each other via the connecting head.

Optionally, the inner shaft assembly includes a fixed shaft, the proximal region of which is fixed to the connecting head, and all movable shafts of the inner shaft assembly are slidably disposed within the fixed shaft.

Optionally, the distal region of the sleeve is sleeved on the outer circumference of the connecting head and secured by a snap fastener.

Optionally, the inner shaft assembly includes:
a fixed shaft, the proximal region of which is fixedly connected to the second support body; and
a plurality of movable shafts that are slidably sleeved inside and outside, each movable shaft being independently configured within the inner shaft drive mechanism; all second transmission members being sequentially arranged along the guide rail, all inner cylinders being sequentially slidably sleeved in the radial direction, and the force-applying components of the inner cylinders being arranged in sequence along the axial direction at the proximal region of the second support body..

Optionally, a blocking member is provided on the guide rail, located between two adjacent second transmission members to limit the sliding travel of each second transmission member.

Optionally, the inner shaft assembly includes, from the innermost to the outermost, a first shaft, a second shaft, and a third shaft, wherein the first shaft and the second shaft serve as movable shafts, and the third shaft serves as a fixed shaft.

Optionally, the inner cylinder includes a first inner cylinder and a second inner cylinder positioned outside the first inner cylinder, each having internal threads for engaging with a second transmission member;
the second transmission member includes two transmission members configured with toothed structures and arranged sequentially from the proximal region to the distal region, wherein the second transmission member at the proximal region engages with the threads of the first inner cylinder and is linked with the first shaft, while the second transmission member at the distal region engages with the threads of the second inner cylinder and is linked with the second shaft.

Optionally, the distal region of the first inner cylinder extends into the second inner cylinder, and a portion of the first inner cylinder extending proximally beyond the second inner cylinder is provided a first annular member serving as a force-applying component;
the distal region of the second inner cylinder extends into the sleeve, and a portion of the second inner cylinder extending proximally beyond the sleeve is provided a second annular member serving as a force-applying component.

Optionally, each inner cylinder and the sleeve are restricted or released from relative rotation by a locking structure, the locking structure including:
an annular body having gear teeth on its inner edge;
an operating button movably mounted on the sleeve, the operating button having a locking portion that engages with the gear teeth;
an elastic member driving the operating button to move outward along a radial direction of the annular body, maintaining the engagement and positioning of the locking member and the gear teeth; and the engagement between the locking member and the gear teeth being released when the operating button is pressed to move inward along the radial direction of the annular body.

This application also provides a control handle for operating a catheter assembly to deploy an artificial implant. The control handle has a distal region and a proximal region opposite to each other, and an axial direction extending between the proximal and distal regions; the catheter assembly includes a sheath tube and an inner shaft assembly that slidably disposed within the sheath tube; the artificial implant before release is connected to the inner shaft assembly and wrapped by the sheath tube; during release, the artificial implant is exposed outside the sheath;
wherein the control handle includes a support body, the catheter assembly includes multiple shafts that are slidably mounted on each other from the inside to the outside, the innermost shaft is a first shaft, a transmission sleeve is connected to the proximal region of the first shaft, the transmission sleeve and the first shaft are slidably engaged in the axial direction and constrained in the circumferential direction;
wherein the proximal region of the support body is rotatably mounted with:
   a third force-applying component fixed to the transmission sleeve to drive the first shaft to rotate; and
   a second force-applying component, in transmission engagement with the proximal region of the first shaft, driving the first shaft to slide axially.

Optionally, a sidewall of the transmission sleeve is provided with a sliding groove that extens axially, and the proximal region of the first shaft is provided with a limiting portion which protrudes radially and outwardly and engages into the sliding groove. When the transmission sleeve and the first shaft slide relative to each other in the axial direction, the limiting portion moves within the sliding groove.

Optionally, a first force-applying component is rotatably mounted on the proximal region of the support body. The first force-applying component engages with the proximal region of the second shaft to drive the second shaft to slide axially. The proximal region of the support body is provided with a support frame, separating the first and second force-applying components, wherein the first force-applying component is located at the distal region of the second force-applying component.

Optionally, a guide rail is fixed within the support body, a second transmission member is slidably mounted on the guide rail in the axial direction, the proximal region of the first shaft is rotatably engaged and axially linked with the second transmission member, an inner cylinder located on the outer circumference of the second transmission member is fixed to the distal region of the second force-applying component, and the inner cylinder engages with the second transmission member in a threaded manner.

Optionally, a reinforcement tube is mounted around an outer circumference of the transmission sleeve.

Optionally, the transmission sleeve and the reinforcement tube have the same length.

This application also provides a control handle for operating a catheter assembly to deploy an artificial implant. The control handle has a distal region and a proximal region opposite to each other, and an axial direction extending between the proximal and distal regions; the catheter assembly includes a sheath tube and an inner shaft assembly that slidably disposed within the sheath tube; the artificial implant before release is connected to the inner shaft assembly and wrapped by the sheath tube; during release, the artificial implant is exposed outside the sheath;
wherein the control handle includes:
a support body to which the catheter assembly is connected;
a transmission member slidably mounted on the support body and connected to a controlled member of the catheter assembly; and
a drive sleeve rotatably mounted on the support body and configured with internal threads, the transmission member and the internal threads of the drive sleeve being threadedly engaged with each other;
wherein the transmission member is configured with a mounting hole for the controlled member to pass through, a radial side of the mounting hole being open to allow the controlled member to be radially inserted and assembled.

Optionally, the support body includes two guide rails arranged side by side, a guide groove being defined between the two guide rails, at least one radial side of the transmission member is configured with a toothed structure extending beyond the guide groove, and the toothed structures on two sides mate with the internal threads of the drive sleeve.

Optionally, two radial sides of the transmission member are configured with toothed structures.

Optionally, the transmission member includes a shoulder which is formed at a portion extending out of the guide groove and adhered to the guide rail.

Optionally, an axial positioning groove is provided on an inner edge of the mounting hole, and a limiting block is fixed to an outer circumference of the proximal region of the controlled member, the limiting block is rotatably inserted into the axial positioning groove.

Optionally, there are at least two toothed structures arranged circumferentially.

Optionally, there are two toothed structures arranged symmetric to each other.

Optionally, there are two toothed structures, namely a first toothed structure and a second toothed structure, corresponding to the first and second guide grooves, respectively, wherein the first guide groove has a greater width than the second guide groove.

Optionally, in terms of axial length, the first toothed structure is greater than or equal to the second toothed structure, and the ratio is in the range of 1 to 2.

This application also provides a locking structure for rotary mating components in a control handle for an artificial implant. The control handle has a distal region and a proximal region opposite to each other, and an axial direction extending between the proximal and distal regions; the catheter assembly includes a sheath tube and an inner shaft assembly slidably mated within the sheath tube; the artificial implant before release is connected to the inner shaft assembly and wrapped by the sheath tube. During release, the artificial implant is exposed outside the sheath tube;
wherein the control handle includes a first component and a second component that are rotatably engaged with each other, and the locking structure includes:
an annular body connected to the first component and rotatably engaged with the second component, gear teeth being formed on an inner edge of the annular body;
an operating button movably mounted on the second component, the operating button being configured with a locking portion that engages with the gear teeth; and
an elastic member driving the operating button to move toward one side along a radial direction of the annular body, maintaining the engagement and positioning of the locking member and the gear teeth; and the engagement between the locking member and the gear teeth being released when the operating button is pressed to move inward toward another side along the radial direction of the annular body.

This application also provides a control handle for operating a catheter assembly to deploy an artificial implant. The control handle has a distal region and a proximal region opposite to each other, and an axial direction extending between the proximal and distal regions; the catheter assembly includes a sheath tube and an inner shaft assembly that slidably disposed within the sheath tube; the artificial implant before release is connected to the inner shaft assembly and wrapped by the sheath tube; during release, the artificial implant is exposed outside the sheath;
wherein the control handle includes a support body;
a bend adjusting drive mechanism mounted to the support body; and
a bend adjusting member, with a first end extending to the distal region of the sheath tube and acting on the sheath tube, and a second end controlled by the bend adjusting drive mechanism.

Optionally, the bend adjusting drive mechanism includes:
a winding wheel rotatably mounted on the support body, the proximal region of the bend adjusting member being made of a flexible material and wound around the winding wheel;
a knob linked with the winding wheel;
a one-way latch structure that interacts with the winding wheel and/or the knob and has relative engaged and disengaged states, in the engaged state, the winding wheel is allowed to rotate in only one direction.

Optionally, a rotary axis of the winding wheel is perpendicular to the axial direction of the control handle.

Optionally, the one-way latch structure includes:
a ratchet; coaxially fixed to the winding wheel;
a limiting member, movably mounted on the support body and having a pawl that engages with the ratchet;
an elastic member, acting on the limiting member to cause the pawl to engage with the ratchet; and
a drive assembly, movably mounted on the support body and matching with the limiting member to drive the limiting member to move, making the one-way latch structure be in the engaged state or disengaged state.

Optionally, the ratchet and the winding wheel are integral.

Optionally, the drive assembly is a toggle button being fixedly connected to the engaged state and extending beyond the control handle.

Optionally, the engaged state and the toggle button are formed integrally or separately.

Optionally, the knob is snap-fitted to the winding wheel and extends through the support body.

This application also provides a connecting structure between a sheath tube and a control handle. The control handle has a distal region and a proximal region opposite to each other, and an axial direction extending between the proximal and distal regions. The catheter assembly includes a sheath tube and an inner shaft assembly that slidably disposed within the sheath tube. The artificial implant before release is connected to the inner shaft assembly and wrapped by the sheath tube. During release, the artificial implant is exposed outside the sheath tube. The connecting structure includes:
a connecting head fixed to the proximal region of the sheath;
an end cap mounted around an outer circumference of the connecting head, anti-rotation structures that cooperate with each other being provided between the end cap and the connecting head; and
a mounting portion located at the distal region of the control handle, the mounting portion defining a mounting channel therein, one radial side of the mounting channel being open, a radial groove being defined on an outer circumference of the mounting portion, the proximal region of the end cap slidably engaged into the radial groove, the proximal region of the sheath tube being engaged into the mounting channel, the mounting portion and the end cap clamping and fixing the connecting heat in the axial direction.

This application also provides a delivery system for operating a catheter assembly to deploy an artificial implant, including a control handle and a catheter assembly. The control handle has a distal region and a proximal region opposite to each other, and an axial direction extending between the proximal and distal regions. The proximal region of the catheter assembly is connected to and controlled by the control handle. The catheter assembly includes:
a second shaft, a pulling wire being connected to a distal region of the second shaft;
a first shaft, a locking member being linked to a distal region of the first shaft;
a third shaft, a base being fixed to a distal region of the third shaft, the locking member movably mounted to the base, the base having a locking hole configured to engage with the locking member;
the pulling wire winding around the artificial implant through the second shaft and then secured to the base by the locking member, the proximal regions of the second shaft and the first shaft being slidably engaged with the third shaft.

The present application provides an artificial heart valve having an axial direction and an inflow side and an outflow side that are opposed to each other in the axial direction. The artificial heart valve includes:
an inner frame being configured as a radially deformable cylindrical structure with a blood flow channel defined therein;
valve leaflets, connected to the inner frame to adjust an opening degree of the blood flow channel; and
an outer frame being integrally formed as an annular structure with multiple arms arranged circumferentially, outflow sides of the arm being fixedly connected to the inner frame, inflow sides of the arms being located around the inner frame, first gaps being defined between the inner frame and the inflow sides of the arms for clamping native tissue;
wherein the outflow sides of two adjacent arms approach each other to form a junction, and the junction extends inwardly to an inner side of the inner frame, being adhered and fixed to an inner wall of the inner frame.

The following provides several optional embodiments, which are not intended to be additional limitations of the above-described overall solution but merely to supplement or optimize it. Each optional embodiment may be combined with the above-described overall solution independently, or with multiple optional embodiments, provided there are no technical or logical inconsistencies.

The present application also provides an artificial heart valve having an axial direction and an inflow side and an outflow side that are opposed to each other in the axial direction. The artificial heart valve includes:
an inner frame being configured as a radially deformable cylindrical structure with a blood flow channel defined therein;
valve leaflets, connected to the inner frame to adjust an opening degree of the blood flow channel; and
an outer frame being integrally formed as an annular structure with multiple arms arranged circumferentially, outflow sides of the arm being fixedly connected to the inner frame, inflow sides of the arms being located around the inner frame, first gaps being defined between the inner frame and the inflow sides of the arms for clamping native tissue;
wherein the arm includes, from the outflow side to the inflow side, a root portion, a middle portion, and a head portion, in a released state, a circumferential span of the arm gradually narrows from the root portion to the head portion, and a separation gap is defined between the middle portion and an outer circumference of the inner frame.

An artificial heart valve has an axial direction, and an inflow side and an outflow side that are opposed to each other in the axial direction. The artificial heart valve includes:
an inner frame being configured as a radially deformable cylindrical structure with a blood flow channel defined therein;
valve leaflets, connected to the inner frame to adjust an opening degree of the blood flow channel; and
an outer frame configured with multiple arms arranged circumferentially, outflow sides of the arm being fixedly connected to the inner frame, inflow sides of the arms being located around the inner frame, first gaps being defined between the inner frame and the inflow sides of the arms for clamping native tissue;
wherein the inner frame and outer frame are integrally formed, and connected to each other through an S-shaped transition portion at the outflow side, and the transition portion has spans in both the circumferential and radial directions of the inner frame.

An artificial heart valve has an axial direction, and an inflow side and an outflow side that are opposed to each other in the axial direction. The artificial heart valve includes:
an inner frame being configured as a radially deformable cylindrical structure with a blood flow channel defined therein;
valve leaflets, connected to the inner frame to adjust an opening degree of the blood flow channel; and
an outer frame being integrally formed as an annular structure with multiple arms arranged circumferentially, outflow sides of the arm being fixedly connected to the inner frame, inflow sides of the arms being located around the inner frame, first gaps being defined between the inner frame and the inflow sides of the arms for clamping native tissue;
wherein the outflow sides of two adjacent arms approach each other to form a junction, the arms are fixed to the inner frame via the junctions, and the outflow side of the junction is provided with a connection lug for cooperating with a delivery system.

An artificial heart valve has an axial direction, and an inflow side and an outflow side that are opposed to each other in the axial direction. The artificial heart valve includes:
an inner frame being configured as a radially deformable cylindrical structure with a blood flow channel defined therein;
valve leaflets, connected to the inner frame to adjust an opening degree of the blood flow channel; and
an outer frame being integrally formed as an annular structure with multiple arms arranged circumferentially, outflow sides of the arm being fixedly connected to the inner frame, inflow sides of the arms being located around the inner frame, first gaps being defined between the inner frame and the inflow sides of the arms for clamping native tissue;
wherein the arm includes, from the outflow side to the inflow side, a root portion, a middle portion, and a head portion, in a released state, the root portion in whole is inclined outward relative to an axis of the inner frame; compared to the transition state, the inclined tendency of the root in the released state increases; and, correspondingly, a radial distance between the head portion and an outer circumference of the inner frame gradually decreases from the transition state to the released state.

An artificial heart valve has an axial direction, and an inflow side and an outflow side that are opposed to each other in the axial direction. The artificial heart valve includes:
an inner frame being configured as a radially deformable cylindrical structure with a blood flow channel defined therein;
valve leaflets, connected to the inner frame to adjust an opening degree of the blood flow channel; and
an outer frame being configured with multiple arms arranged circumferentially, outflow sides of the arm being fixedly connected to the inner frame, inflow sides of the arms being located around the inner frame, first gaps being defined between the inner frame and the inflow sides of the arms for clamping native tissue;
wherein the inner frame has multi-turns of mesh structures arranged along the axial direction, the mesh at the outflow side being more sparse than that at the inflow side; and
wherein one turn of mesh structure on the outflow side of the inner frame includes two parallelograms arranged side by side and sharing a common edge, the common edge extends along the axial direction of the inner frame, opposite edges at two sides of the common edge along a circumferential direction of the inner frame offset toward the outflow side, respectively.

Optionally, according to the degree of radial deformation, the artificial heart valve has:
a compressed state, wherein the inner and outer frames are both radially compressed, and the inflow side of the outer frame approaching the outer circumference of the inner frame;
a transition state, wherein the inflow side of the outer frame radially expands outward relative to the outer circumference of the inner frame, and at least a part of the inner frame remains radially compressed; and
a released state, wherein the inner and outer frames both expand radially.

Optionally, the inner and outer frames switch from the compressed state to the released state through self-expanding.

Optionally, there is a plurality of leaflets cooperating with each other, a number and a circumferential position of each arm correspond to a corresponding leaflet.

Optionally, the leaflets are made of a biomaterial or a polymer material.

Optionally, the outflow sides of two adjacent arms approach each other to form a junction, and the arms are fixed to the inner frame via the junctions.

Optionally, the inner frame has a mesh structure formed by frame bars, and the junction has a bifurcated structure extending toward the outflow side.

Optionally, the bifurcated structure extends along the frame bar of the inner frame.

Optionally, the bifurcated structure has suture holes at the node points.

Optionally, the inner frame and the outer frame are formed separately and then fixed together or formed integrally.

Optionally, the inner frame and the outer frame each are integrally formed by cutting a tubular stock, and the inflow sides of the inner frame and the outer frame are spaced from each other in an axial direction of the tubular stock.

Optionally, the transition section has a first span W1 in a circumferential direction of the inner frame, and a span between adjacent arms at the outflow side is at least twice of W1.

Optionally, the transition section has a second span W2 in a radial direction of the inner frame.

Optionally, the junction is fixed to the outflow side of the inner frame.

Optionally, the outflow side of the junction is configured with a connecting lug for cooperating with a delivery system.

Optionally, the junction and the outflow side of the inner frame are joined together to form a common mesh.

Optionally, the nodes of the common mesh at the outflow side are provided with connecting lugs for cooperating with a delivery system.

Optionally, at least one of two axial ends of the inner frame is provided with a connecting lug for cooperating with a delivery system, and the connecting lug is integrally formed with the inner frame or the outer frame.

Optionally, the connecting lug is T-shaped or has a hole structure for threading a pulling wire.

Optionally, the inner frame has a mesh structure, and the connecting lug is located at a vertex on the outflow side of the mesh structure along the circumferential direction of the inner frame.

Optionally, the inner frame has a mesh structure, multiple mesh vertices are arranged circumferentially on the inflow side of the inner frame, at least two of which tend to extend axially further than other mesh vertices on the inflow side.

Optionally, both the inner frame and the outer frame are provided with integral connecting lugs, and the connecting lugs provided by the inner and outer frames are nested with each other.

Optionally, an end of the connecting lug bends radially and inwardly.

Optionally, in the released state, the inner frame is cylindrical-shaped, and a ratio of an axial length L of the inner frame to a diameter D of the inner frame is (0.8-1.5):1.

Optionally, in the released state, the inner frame is a straight cylinder.

Optionally, the inner frame has multi-turns of mesh structures arranged along the axial direction, and the mesh on the outflow side is concave hexagons, with the portion facing the outflow side concaved inwardly toward the inflow side.

Optionally, the inner frame has multi-turns of mesh structures arranged along the axial direction, and in at least one mesh structure, two opposing nodes of the same mesh in the circumferential direction of the inner frame are staggered along the axial direction of the inner frame.

Optionally, the inner frame has multi-turns of mesh structures arranged along the axial direction, and the mesh structure on the outflow side is more sparse than the mesh structure on the inflow side.

Optionally, the mesh structure of the inner frame on the inflow side is configured with diamond-shaped meshs, and in the circumferential direction, a number of meshs at the inflow side is twice that of the meshs at the outflow side.

Optionally, the head portion is adhered to the outer circumference of the inner frame.

Optionally, the head portion abuts against the outer circumference of the inner frame and there is a preload force between the head portion and the outer circumference of the inner frame.

Optionally, the outer frame is formed by frame bars, and the frame bars of the head portion have undulating shapes.

Optionally, the outer frame is formed by frame bars, and surfaces of the frame bars of the head portion are configured with anti-slip grooves.

Optionally, the axial lengths of the arms are not identical.

Optionally, one of the arms has the smallest axial length.

Optionally, the root portion abuts against the outer circumference, and the axial length of the abutting portion is at least 1/5 of the total length of the arm.

Optionally, an edge of the leaflet is configured with a fixed edge connected to the inner frame and a movable edge that cooperates with the leaflet to control blood flow; and the fixed edge is closer to the inflow side than the arm.

Optionally, an edge of the leaflet is configured with a fixed edge connected to the inner frame and a movable edge that cooperates with the leaflet to control blood flow; the inner frame has a enlarged portion expanding radially outwardly on its outflow side, and the fixed edge is substantially located on the outflow side of the enlarged portion.

Optionally, the inner frame has a mesh structure, and the distance between the bottom of the fixed edge and an edge of the inflow side of the inner frame corresponds to half a mesh.

Optionally, the movable edges of adjacent leaflets converge at the junction of the inner frame, and the inner frame further extends at least one mesh on the outflow side of the junction, serving as an end mesh.

Optionally, an overlapping portion of the inner frame and the outer frame is located on the outflow side of the end mesh.

Optionally, a membrane is fixed within the end mesh.

Optionally, the membrane is formed by splicing adjacent leaflets.

Optionally, the outflow side of the inner frame has multiple sharp corners distributed circumferentially, each of which has an hole structure fixed thereto for threading a pulling wire.

Optionally, the arms are U-shaped, the outflow sides of adjacent arms approach each other to form a junction, and the arms are fixed to the inner frame via the junctions.

Optionally, the inner frame has a mesh structure, and the junctions and the frame bars of the mesh structure are overlapped and fixed to each other.

Optionally, an apex of the junction forms a V-shaped structure facing the outflow side, multiple positioning holes are arranged at the connection of the junction and the arm, corresponding positioning holes are also arranged at corresponding portions of the inner frame, and the inner and outer frames are fixed to each other by binding wires passing through the positioning holes.

The control mechanism of this application can cooperate with each other to control the release or withdrawal process of artificial implants inside the body, and has been further optimized and improved in structure; furthermore, the outer frame of the artificial heart valve is an annular structure formed integrally, which is easy to process and can ensure strength; moreover, improvements have been made to the shape of the inner and outer frames and their connection methods, and corresponding technical effects have been achieved.

### BRIEF DESCRIPTION OF THE DRAWINGS

Fig. 1a is a schematic diagram of a control mechanism constraining an artificial implant according to an embodiment of this application;
Fig. 1b is a schematic diagram of the free end of the pulling wire in Fig. 1a being released from restraint.
Fig. 2a is a schematic diagram of a control mechanism constraining an artificial implant according to an embodiment of this application.
Fig. 2b is a schematic diagram of the free end of the pulling wire in Fig. 2a being released from restraint.
Fig. 3a is a schematic diagram of a control mechanism constraining an artificial implant according to the prior art.
Fig. 3b is a schematic diagram of the free end of the pulling wire in Fig. 3a being released from restraint.
Fig. 4 is a schematic diagram of a control mechanism according to an embodiment of this application with three pulling wires.
Fig. 5 is a schematic diagram of the control mechanism according to an embodiment of this application, wherein each pulling wire is connected to a second shaft.
Figs. 6a and 6b are schematic diagrams of the free ends of the pulling wires according to an embodiment of this application.
Fig. 7 is a schematic diagram of an artificial implant according to one embodiment of this application.
Figs. 8a-8c are schematic diagrams showing the wire control end of the artificial implant according to this application being folded.
Figs. 9a and 9b are schematic diagrams of the threading and withdrawal of the artificial implant according to this application.
Fig. 10 is a three-dimensional diagram of the threading of pulling wires between the artificial implant and the control mechanism according to this application.
Fig. 11a is a schematic diagram of the threading of pulling wires for an artificial implant according to the prior art.
Fig. 11b is a diagram of a retraction route of an artificial implant according to the prior art.

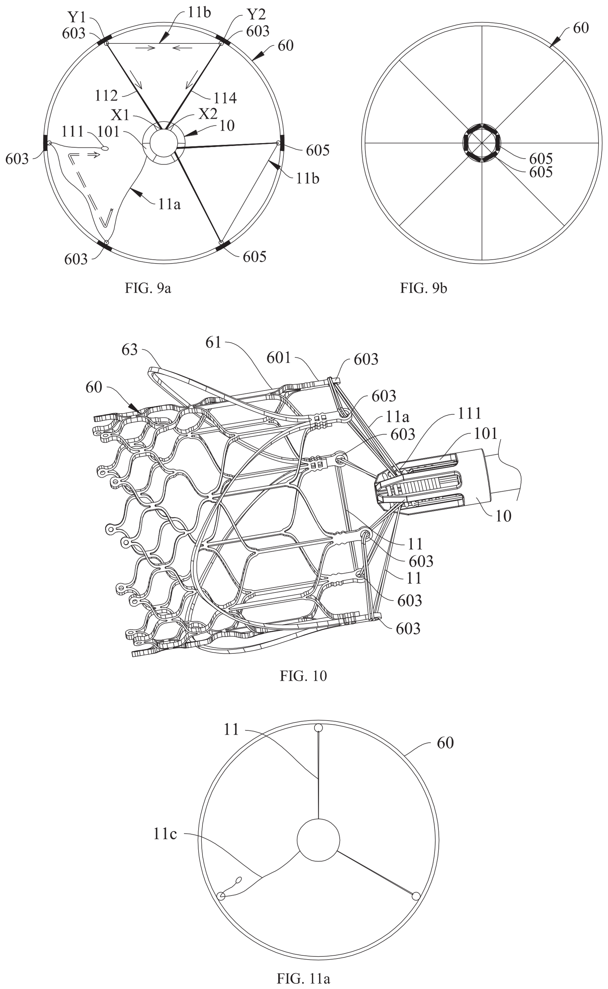

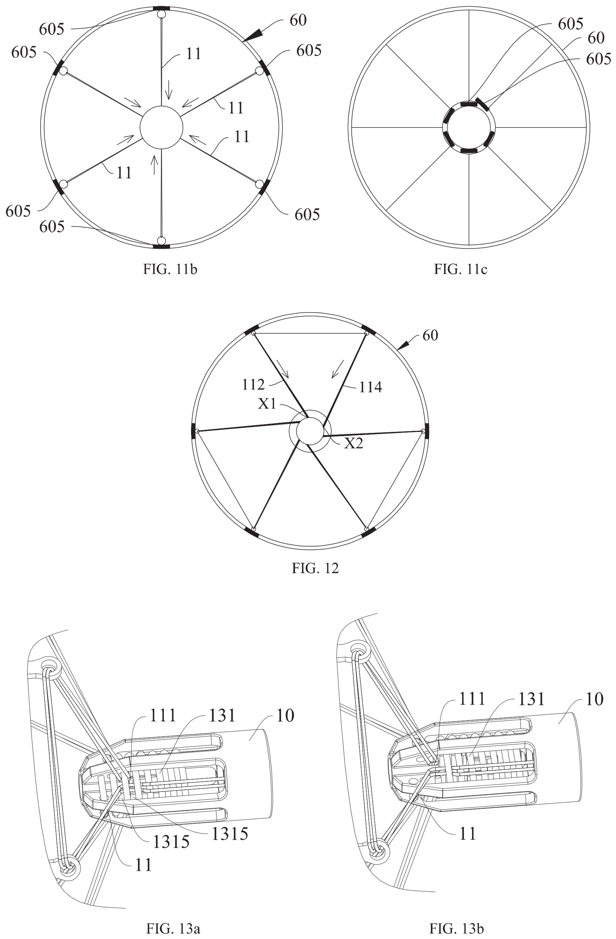

Fig. 11 c is a schematic diagram of the artificial implant in Fig. 11b after completion of retraction.
Fig. 12 is a diagram of the retraction route of the artificial implant according to this application.
Figs. 13a and 13b are schematic diagrams of the locking member in the control mechanism according to this application entering and exiting the locking hole.

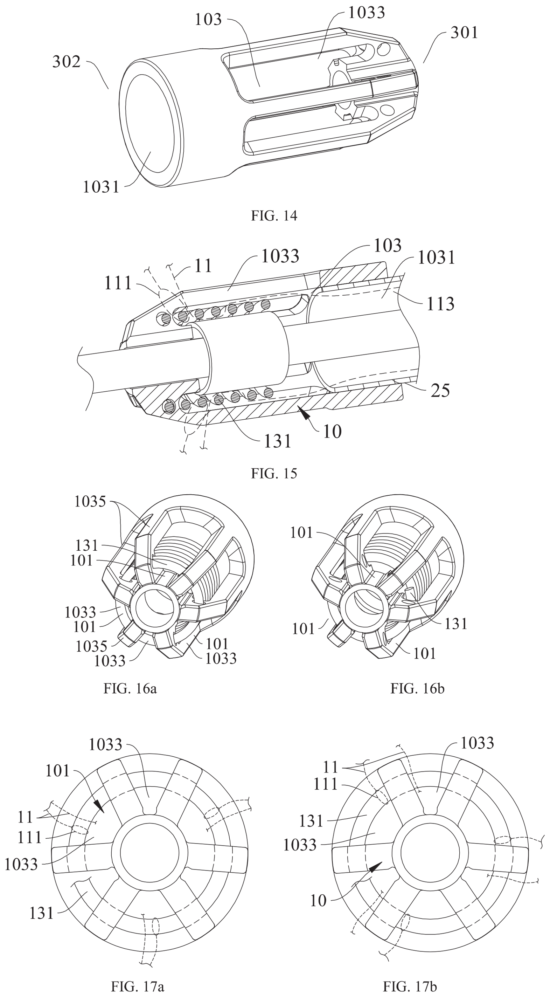

Fig. 14 is a schematic diagram of the base of the control mechanism of this application.
Fig. 15 is a schematic diagram of a half cross-sectional of the base of the control mechanism of this application.
Figs. 16a and 16b are three-dimensional schematic diagrams of the locking member in the control mechanism of this application being engaged into and disengaged from the locking hole.
Figs. 17a to 17c are schematic diagrams of the locking member in the control mechanism of this application where the pulling wires pass through sequentially.

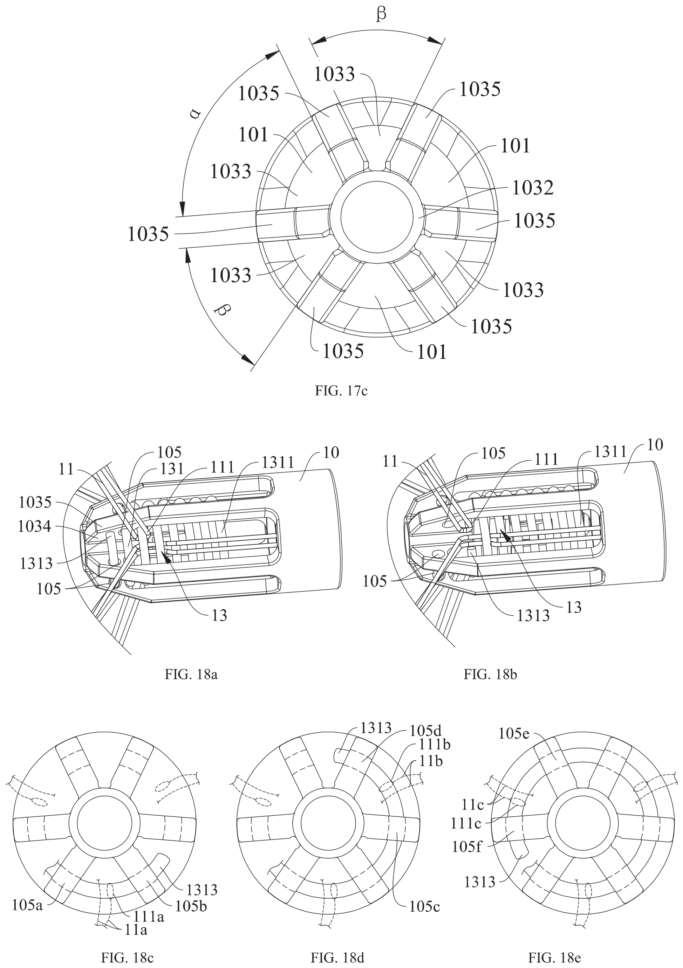

Figs. 18a and 18b are schematic diagrams of the locking member in the control mechanism of this application entering and exiting the locking hole.
Figs. 18c through 18e are schematic diagrams of the locking member in the control mechanism of this application sequentially passing through the locking holes.

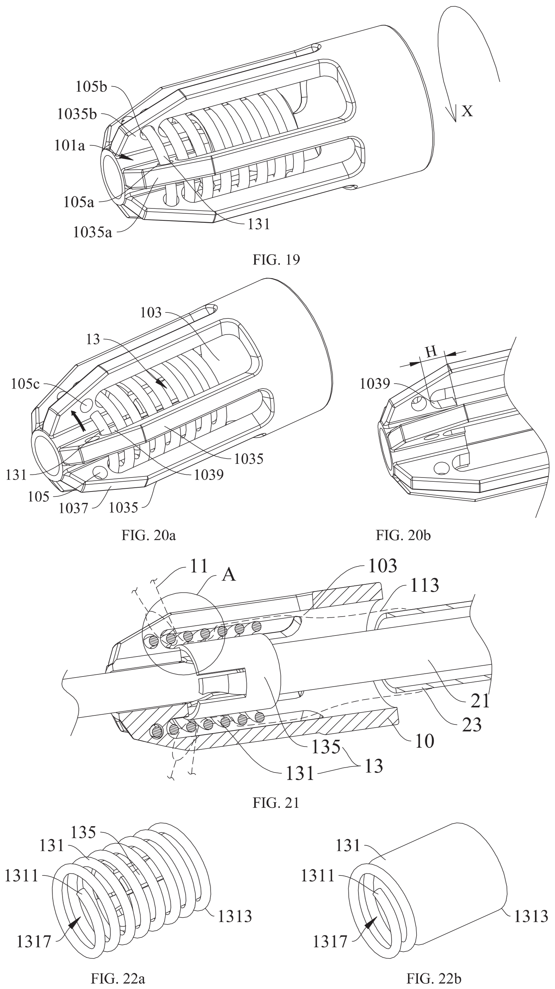

Fig. 19 is a schematic diagram showing the cooperating of the locking member and the base in the control mechanism of this application.
Fig. 20a is a schematic diagram of the process of inserting the locking member into the locking hole of the control mechanism of this application.
Fig. 20b is a schematic diagram of a portion of the base of the control mechanism of this application.
Fig. 21 is a schematic diagram of a half cross-sectional of the wire control end of the base of the control mechanism of this application.
Figs. 22a and 22b are schematic diagrams of the locking member of the control mechanism of this application.

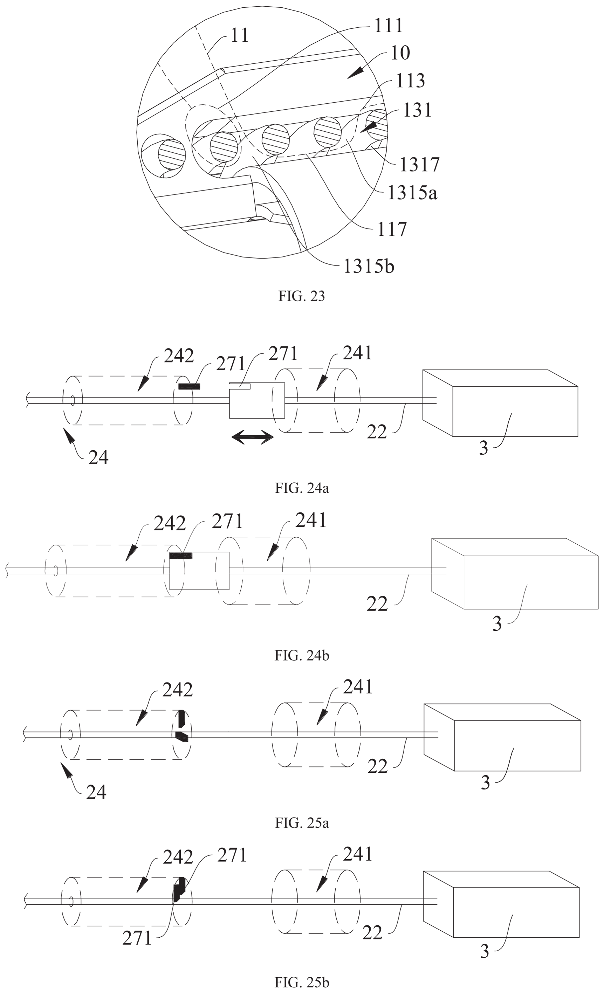

Fig. 23 is a schematic diagram of the path of the pulling wire through the locking member in the control mechanism of this application.
Figs. 24a and 24b are schematic diagrams showing the linkage of the control mechanism of one embodiment of this application.
Figs. 25a and 25b are schematic diagrams showing the linkage of the control mechanism of another embodiment of this application.

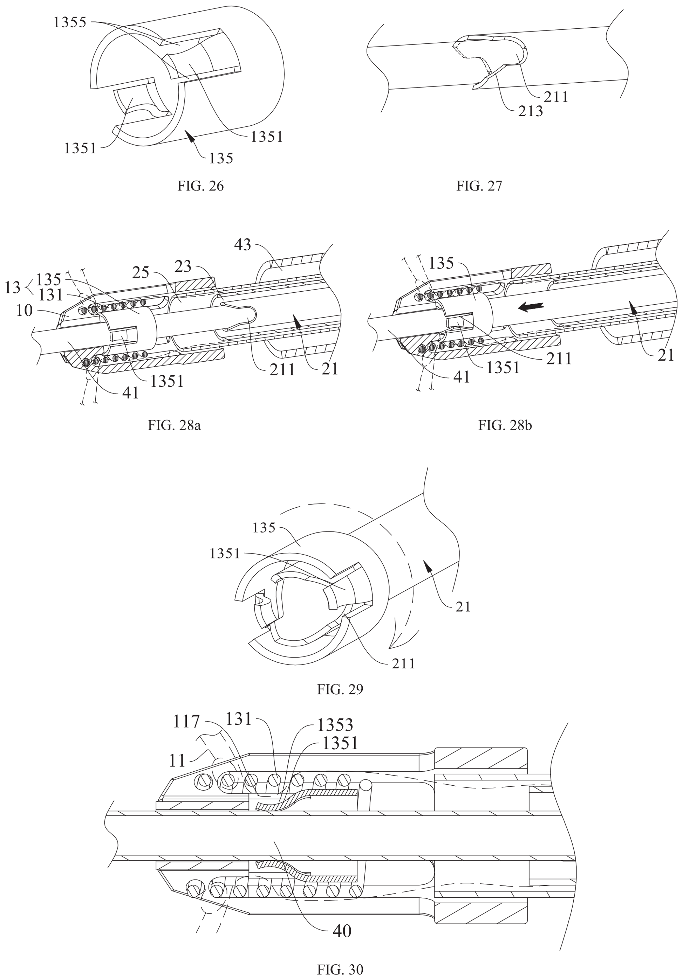

Fig. 26 is a schematic diagram of the connecting portion of the locking member in the control mechanism of this application.
Fig. 27 is a partial, schematic diagram of the distal region of the first shaft in the control mechanism of this application.
Figs. 28a and 28b are schematic, half cross-sectional diagrams showing the engagement and disengagement of the first shaft and the connecting portion of the control mechanism of this application.
Fig. 29 is a schematic diagram of the rotation of the first shaft and the connecting portion after engaged in the control mechanism of this application.
Fig. 30 is a cross-sectional view of the base of the control mechanism of this application.

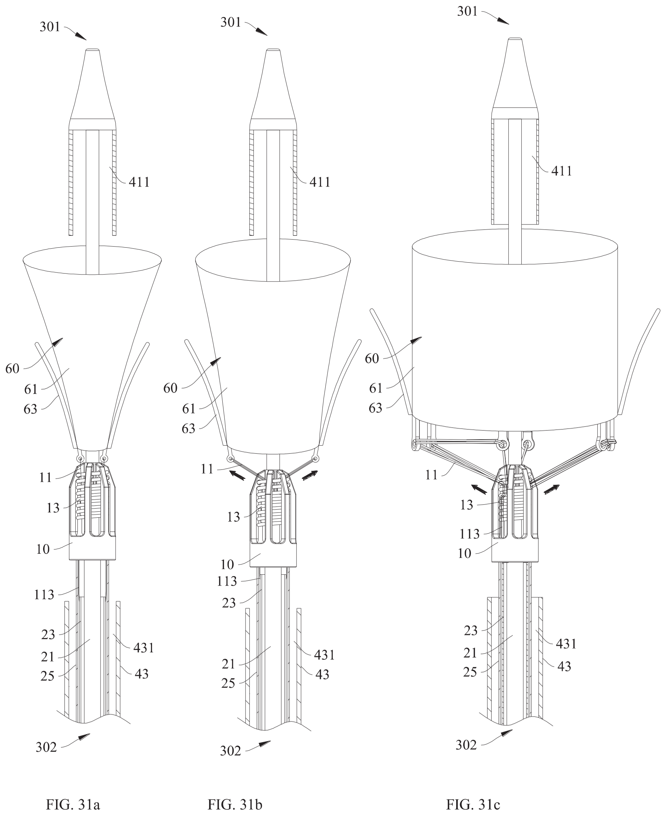

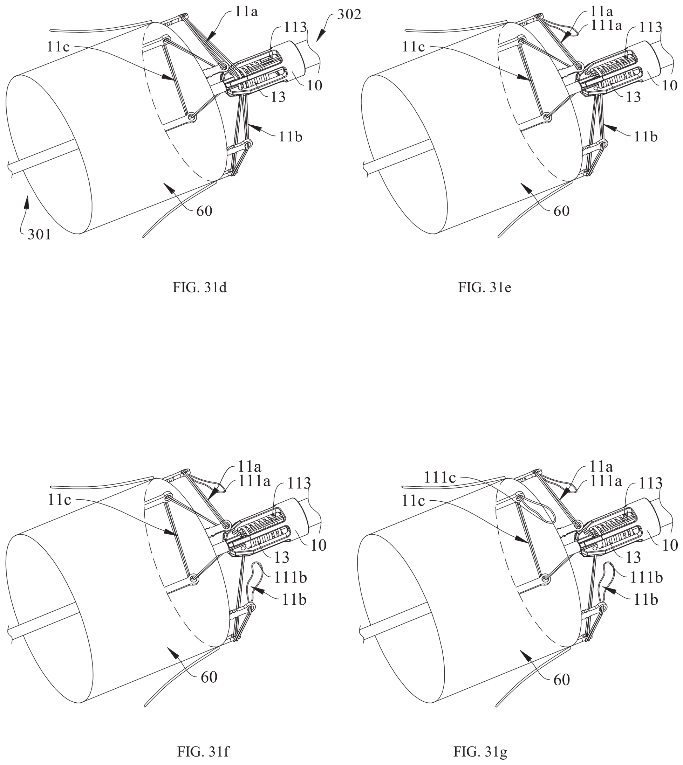

Figs. 31a-31c are schematic diagrams of the release of the artificial implant in the control mechanism of this application, allowing the proximal region to gradually expand.
Figs. 31d-31g are schematic diagrams of the sequential release of the restraints of the pulling wires in the control mechanism of this application.

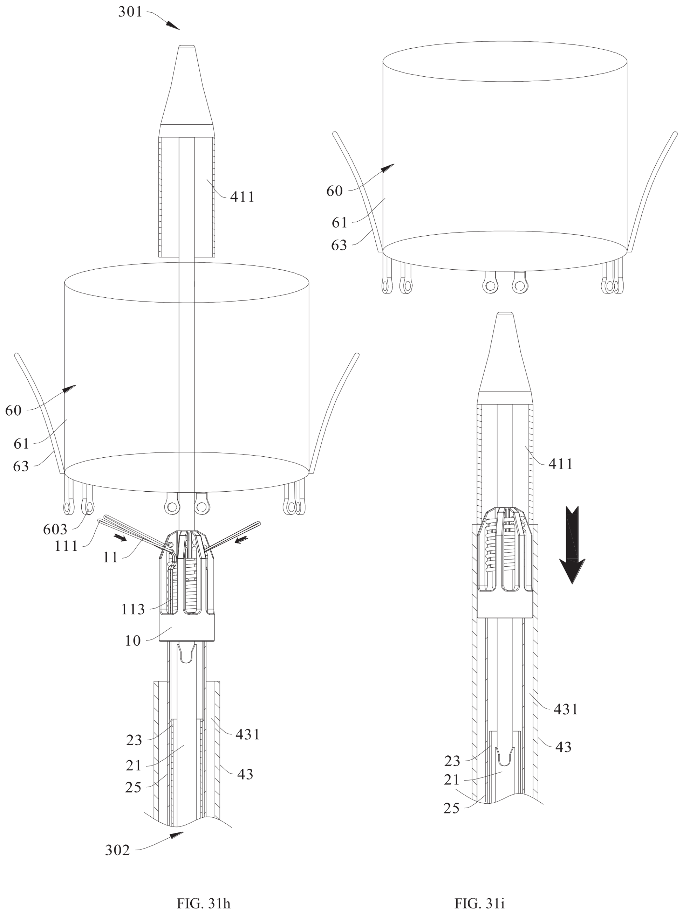

Figs. 31h and 31i are schematic diagrams of the retraction and withdrawal of the pulling wires in the control mechanism of this application.

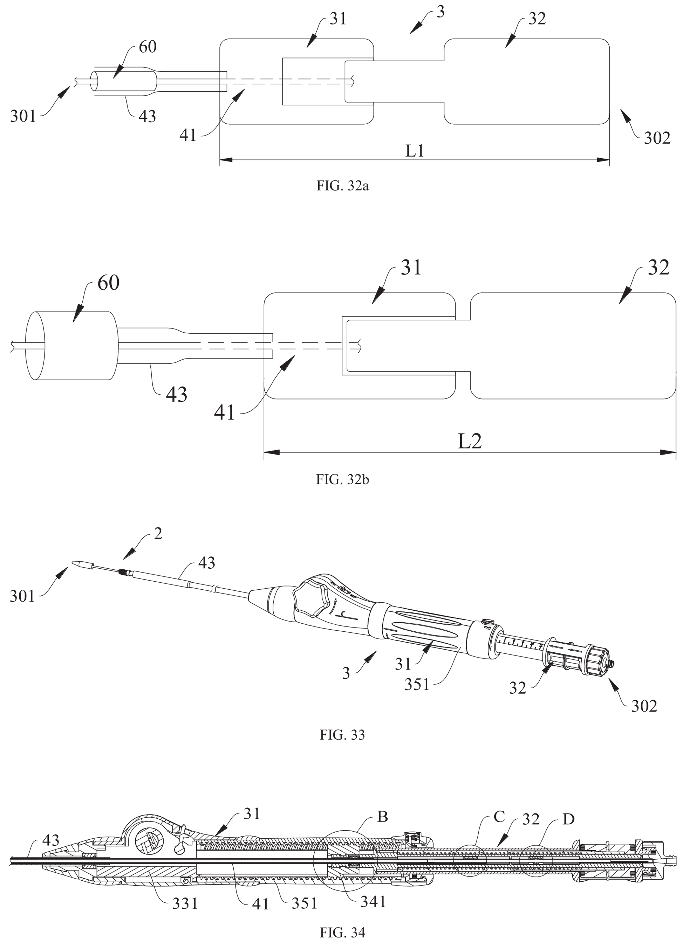

Figs. 32a and 32b are schematic diagrams of the control handle of one embodiment of this application.
Fig. 33 is a schematic diagram of the control handle of one embodiment of this application.
Fig. 34 is a cross-sectional view of the control handle in Fig. 33.

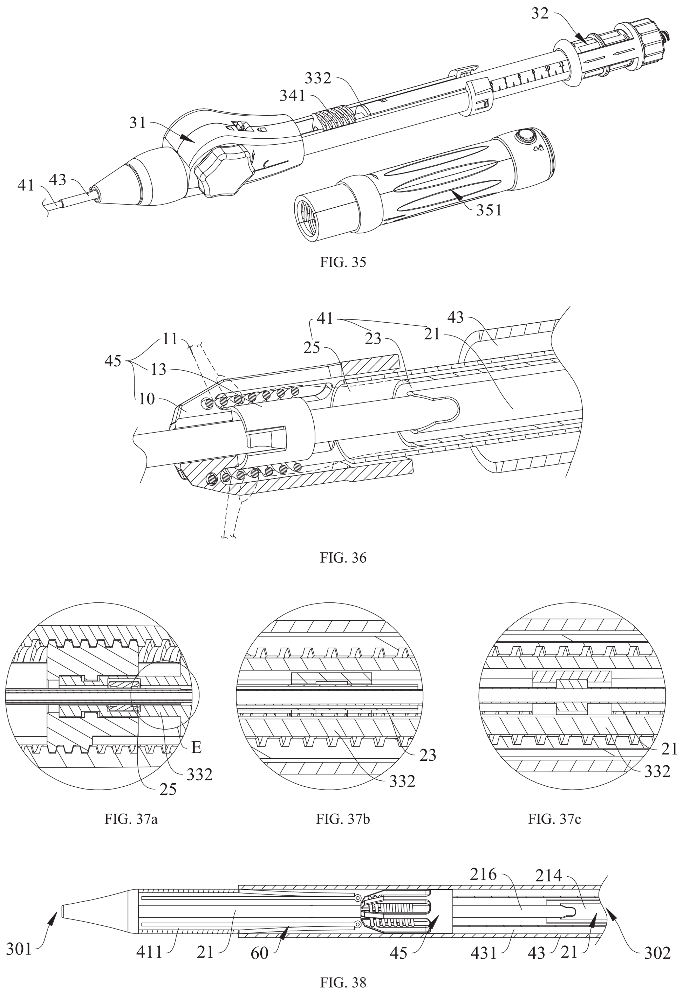

Fig. 35 is an exploded view of the control handle after the first drive sleeve is removed in one embodiment of this application.
Fig. 36 is a schematic diagram of the distal region of the control handle in one embodiment of this application.
Figs. 37a-37c are enlarged views of sections B, C, and D in Fig. 34.
Fig. 38 is a cross-sectional view of the first and second loading sections of the control handle in one embodiment of this application, enclosing an artificial implant.

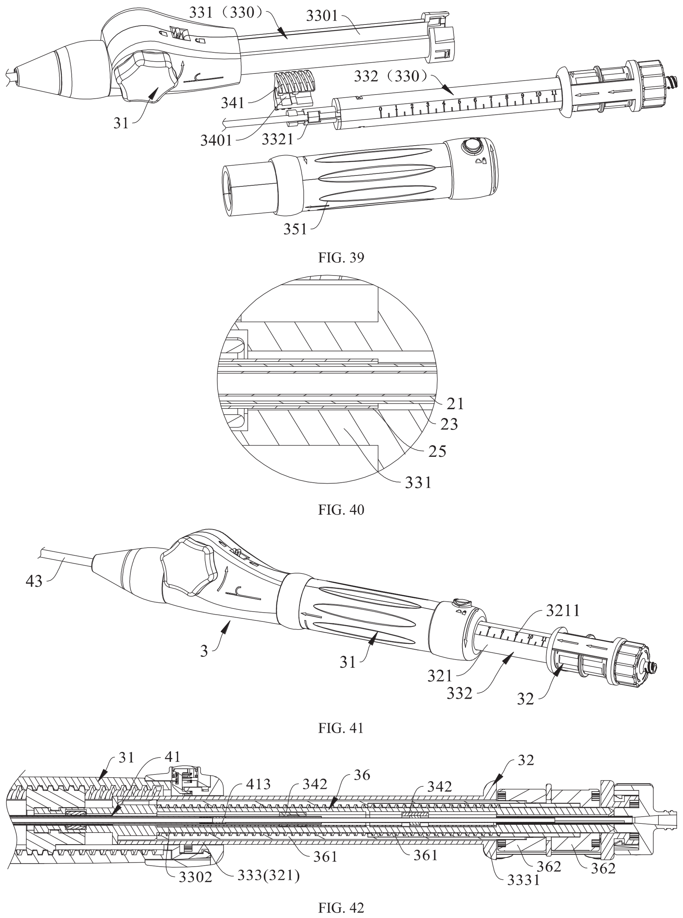

Fig. 39 is an exploded view of the control handle in one embodiment of this application.
Fig. 40 is an enlarged view of section E in Fig. 37a.
Fig. 41 is a schematic diagram of a control handle in another embodiment of this application.
Fig. 42 is a partial, cross-sectional view of the control handle in one embodiment of this application.

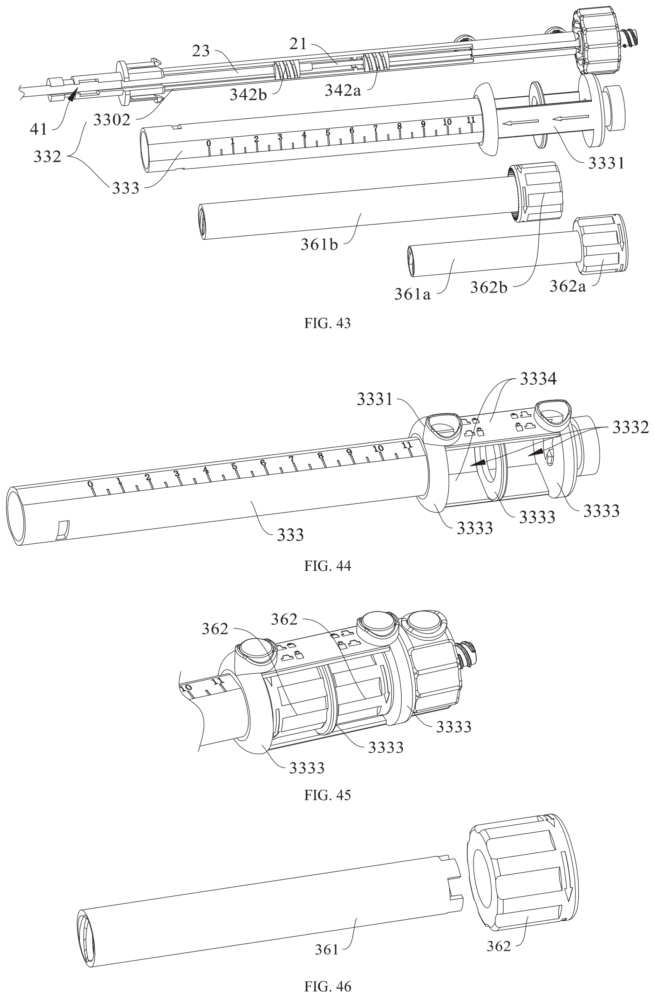

Fig. 43 is a schematic, exploded diagram of a control handle in one embodiment of this application.
Fig. 44 is a schematic diagram of the sleeve in Fig. 43.
Fig. 45 is a schematic diagram of the support frame in Fig. 41.
Fig. 46 is an exploded view of the inner cylinder and the force-applying component in Fig. 43.

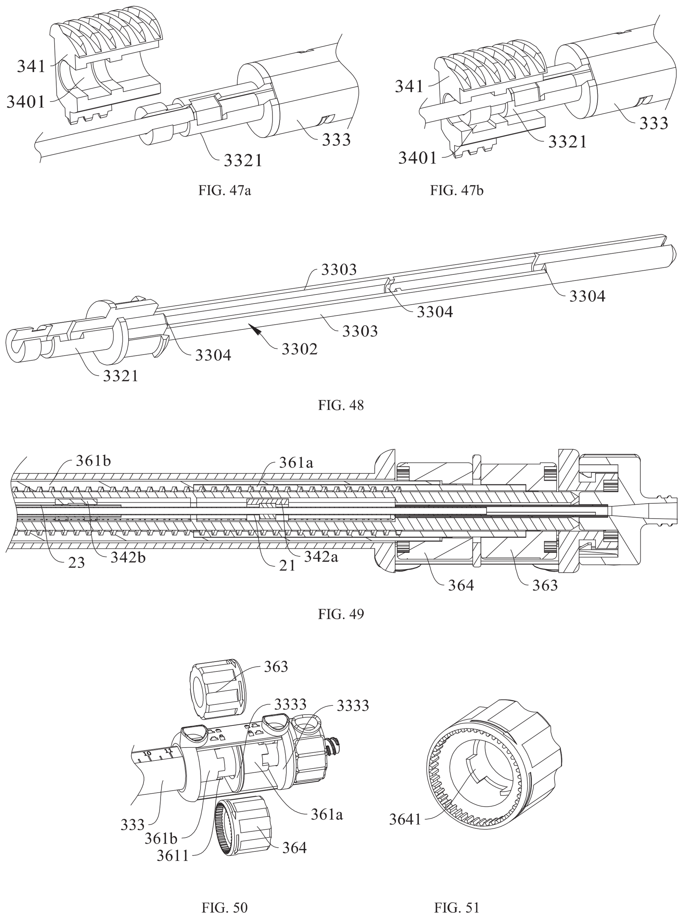

Figs. 47a and 47b are schematic diagrams of the first transmission member and the connecting head in the control handle of one embodiment of this application.
Fig. 48 is a schematic diagram of the guide rail in the control handle of one embodiment of this application.
Fig. 49 is a partial, cross-sectional view of the control handle of one embodiment of this application.
Fig. 50 is an exploded view of the first annular body, the second annular body, and the support frame in the control handle of one embodiment of this application.
Fig. 51 is a schematic diagram of the second annular body in Fig. 50.

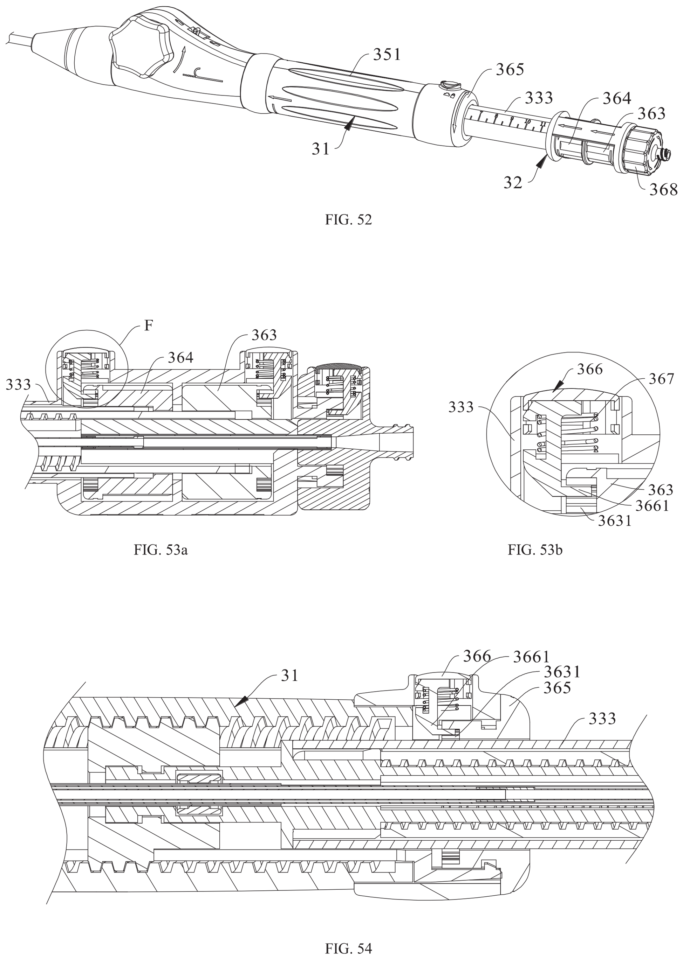

Fig. 52 is a schematic diagram of a control handle of another embodiment of this application.
Fig. 53a is a partial, cross-sectional view of the support frame of the control handle in one embodiment of this application.
Fig. 53b is an enlarged view of section F in Fig. 53a.
Fig. 54 is a partial, cross-sectional view of the third annular body of the control handle in one embodiment of this application.

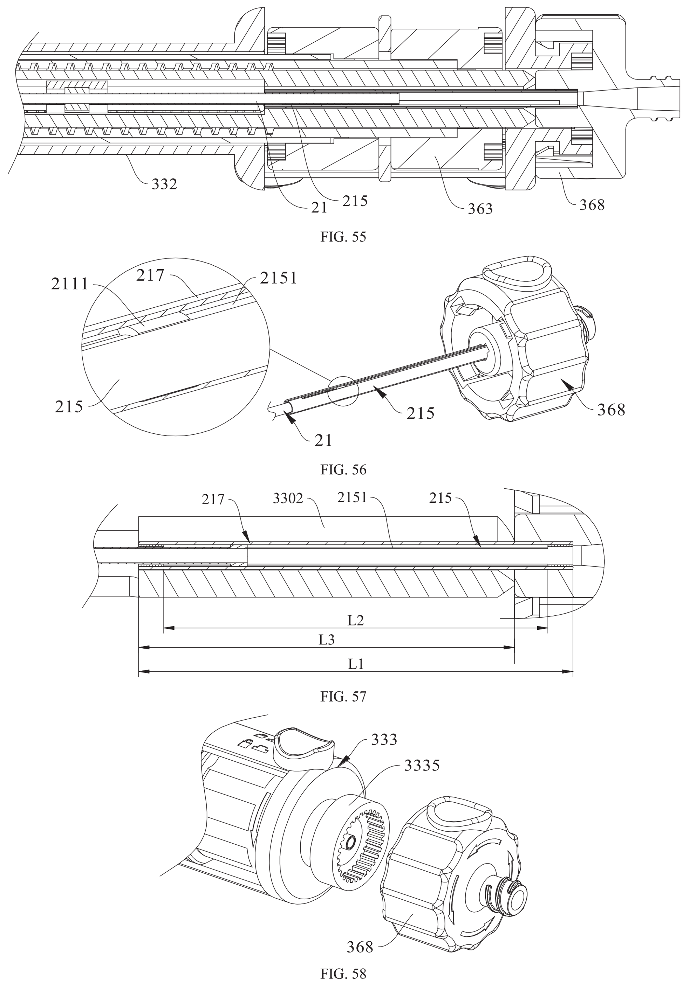

Fig. 55 is a partial, cross-sectional view of the fourth annular body of the control handle in one embodiment of this application.
Fig. 56 is a half cross-sectional view of the first shaft, transmission sleeve, reinforcement tube, and fourth annular body of the control handle in one embodiment of this application.
Fig. 57 is a partial, cross-sectional view of the first shaft, transmission sleeve, reinforcement tube, and fourth annular body of the control handle in one embodiment of this application.
Fig. 58 is a partial, exploded view of the support frame and fourth annular body of the control handle in one embodiment of this application.

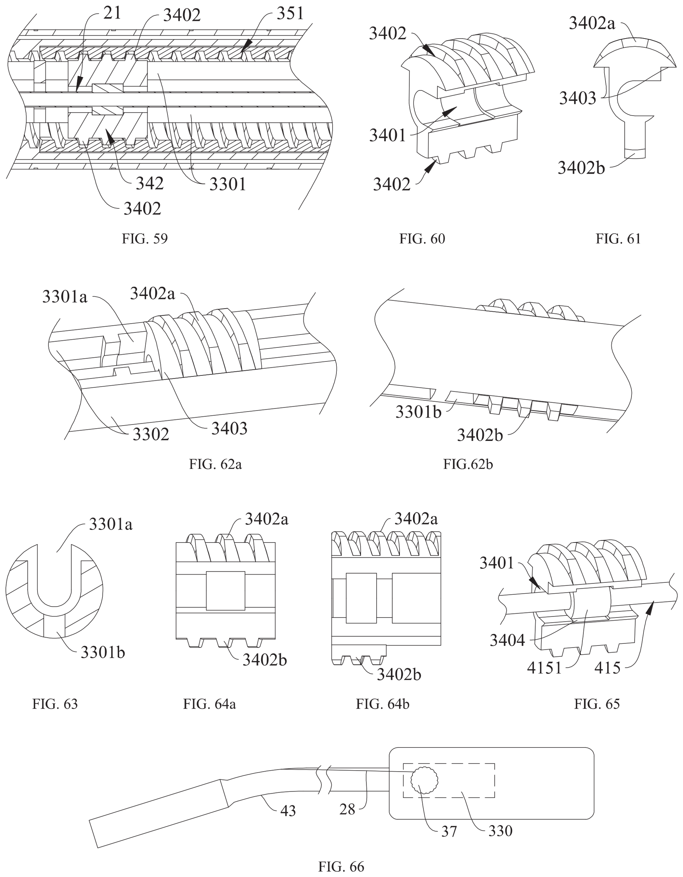

Fig. 59 is a partial, cross-sectional view of the second transmission member in the control handle according to an embodiment of this application.
Fig. 60 is a three-dimensional view of the second transmission member in the control handle according to an embodiment of this application.
Fig. 61 is a front view of the second transmission member in the control handle according to an embodiment of this application.
Figs. 62a and 62b are schematic, partial views showing the cooperation of the second transmission member and the guide rail in the control handle according to an embodiment of this application.
Fig. 63 is a cross-sectional view of the guide rail in the control handle according to an embodiment of this application.
Figs. 64a and 64b are side views of the second transmission member in the control handle according to an embodiment of this application.
Fig. 65 is a schematic view showing the cooperation between the second transmission member and the controlled member in the control handle according to an embodiment of this application.
Fig. 66 is a schematic view of the control handle according to an embodiment of this application regarding the bend adjusting member.

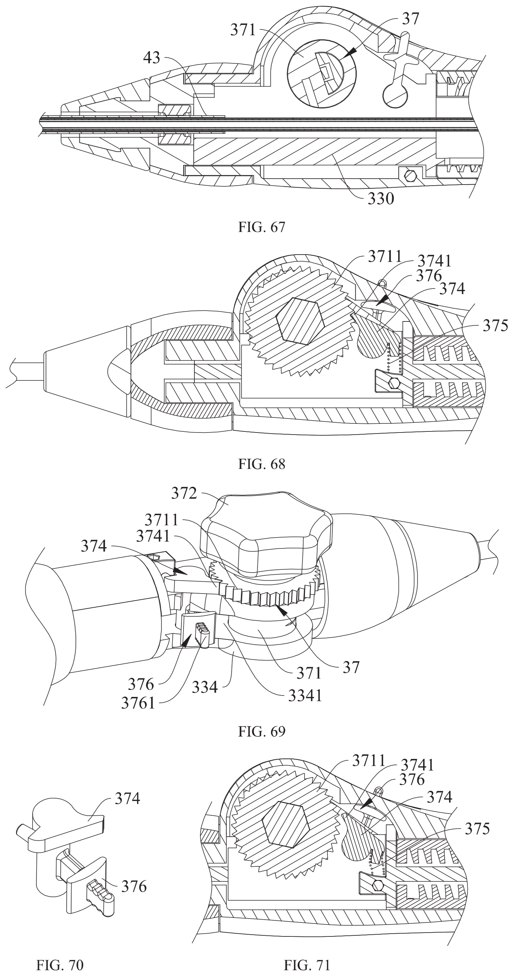

Fig. 67 is a partial, cross-sectional view of the control handle at the bend adjusting mechanism in one embodiment of this application.
Fig. 68 is a partial, cross-sectional view of the control handle at the ratchet in one embodiment of this application.
Fig. 69 is a partial, schematic view of the control handle in one embodiment of this application regarding the bend adjusting mechanism.
Fig. 70 is a schematic view of the limiting member in the control handle in one embodiment of this application.
Fig. 71 is a schematic view of the ratchet in Fig. 68 after release.

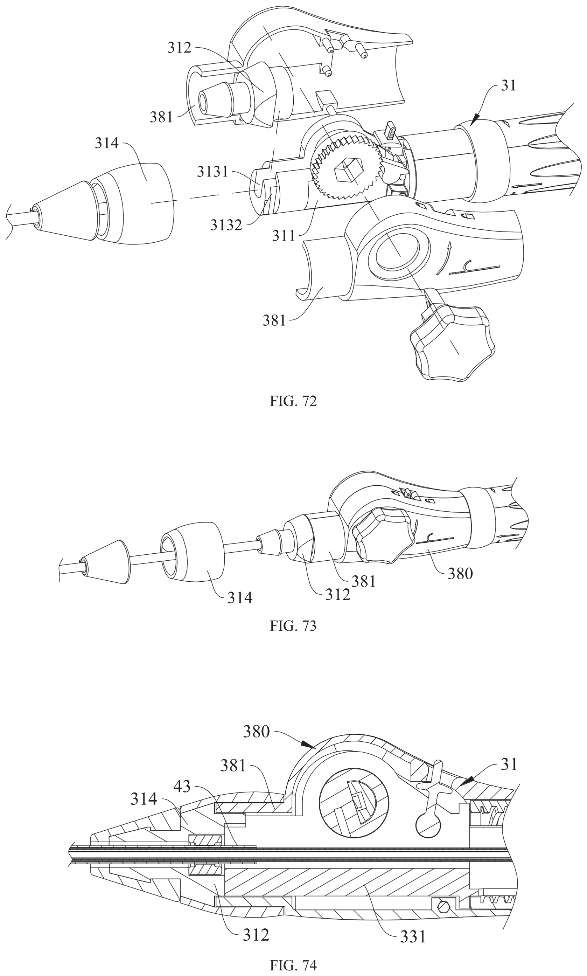

Fig. 72 is an exploded view of the distal region of the control handle in one embodiment of this application.
Fig. 73 is an exploded view of the distal region of the control handle in one embodiment of this application.
Fig. 74 is a partial, cross-sectional view of the proximal region of the sheath tube in the control handle in one embodiment of this application.

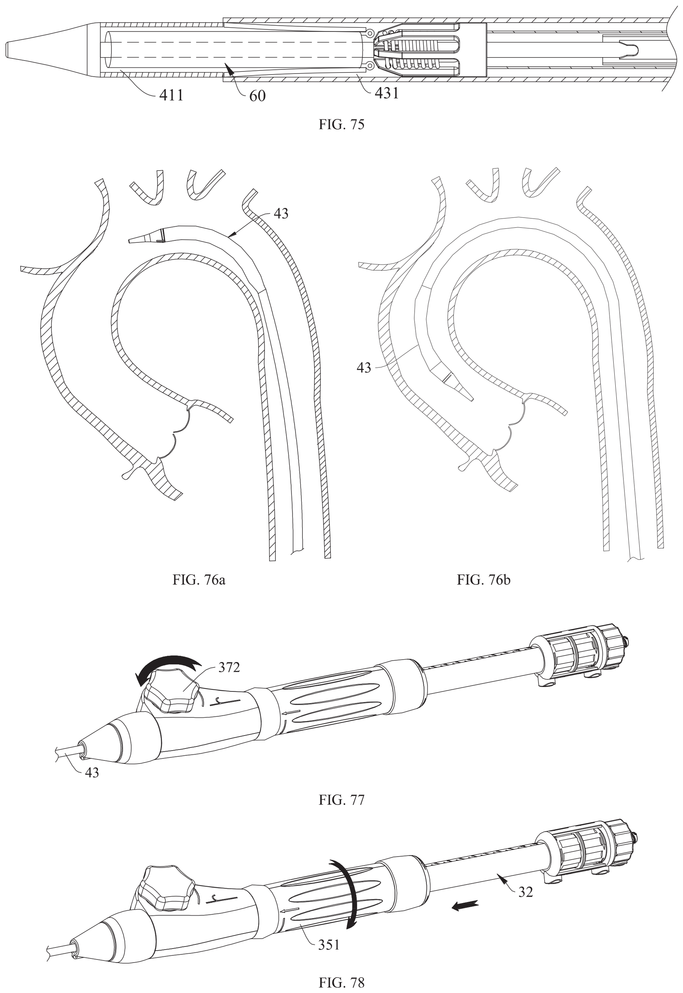

Fig. 75 is a cross-sectional view of an artificial implant in accordance with an embodiment of the present application being wrapped by the first loading section and the second loading section.
Figs. 76a and 76b are schematic diagrams of the delivery system when the distal region is bent.
Fig. 77 is a schematic diagram of an operating knob of the delivery system according to an embodiment of the present application.
Fig. 78 is a schematic diagram showing the delivery system in Fig. 77 operating the first drive sleeve.

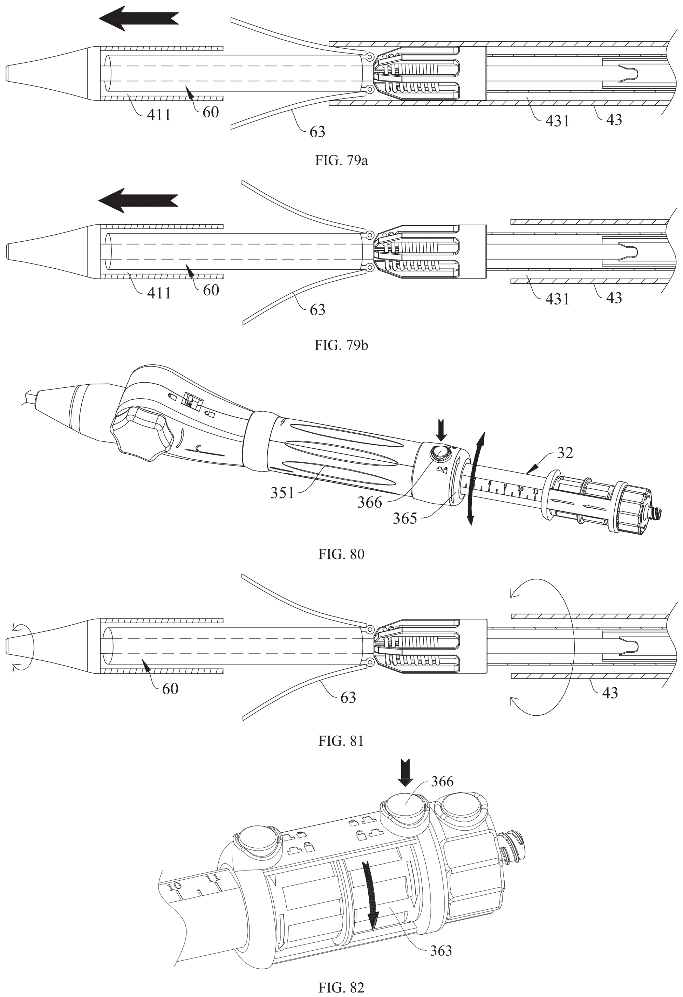

Figs. 79a and 79b are schematic diagrams of the arm of the artificial implant in Fig. 75 being released from restraint.
Fig. 80 is a schematic diagram showing the delivery system in Fig. 77 operating the third annular body.
Fig. 81 is a schematic diagram of adjusting the circumferential position of the artificial implant.
Fig. 82 is a schematic diagram of the delivery system in Fig. 77 operating the first annular body.

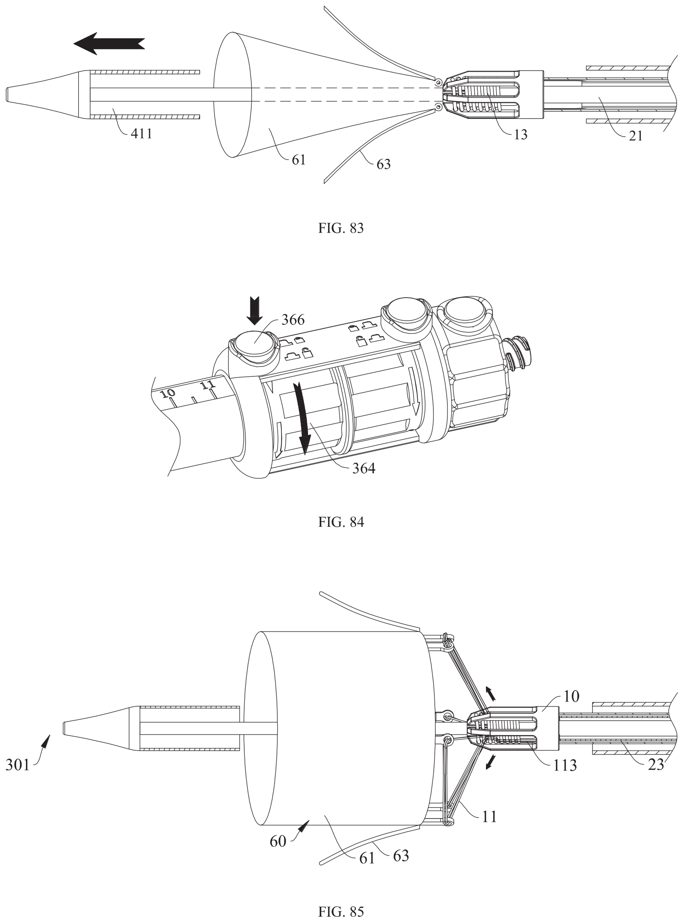

Fig. 83 is a schematic diagram of releasing the restraint of the distal region of the artificial implant.
Fig. 84 is a schematic diagram of the delivery system in Fig. 77 operating the second annular body.
Fig. 85 is a schematic diagram showing the gradual release of the pulling wire, causing the wire control end of the artificial implant to expand gradually.

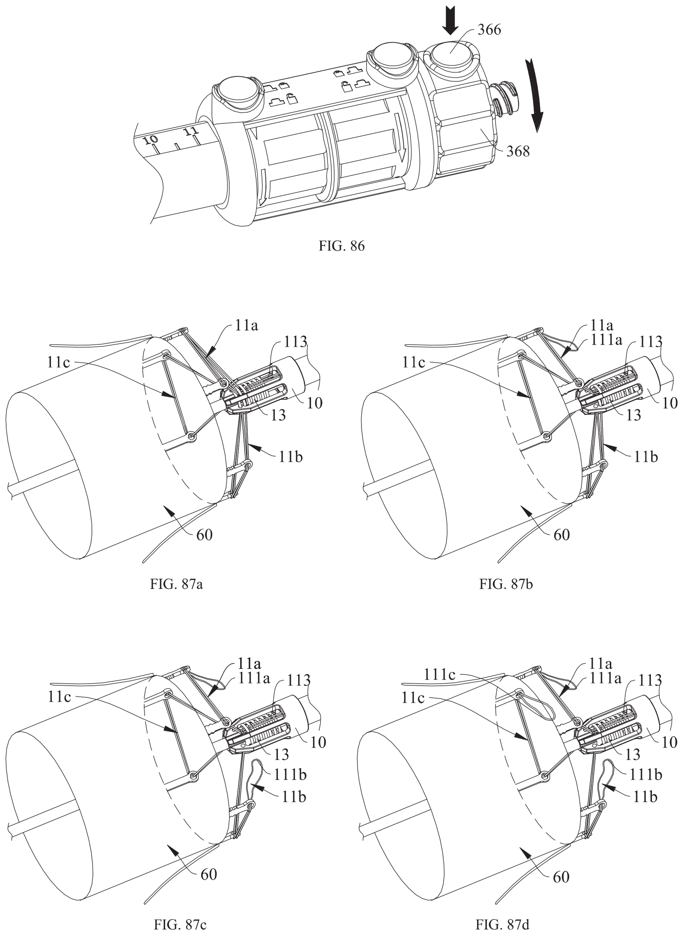

Fig. 86 is a schematic diagram of the delivery system in Fig. 77 operating the fourth ring body.
Figs. 87a to 87d are schematic diagrams showing the pulling wires being detached sequentially from the artificial implant.

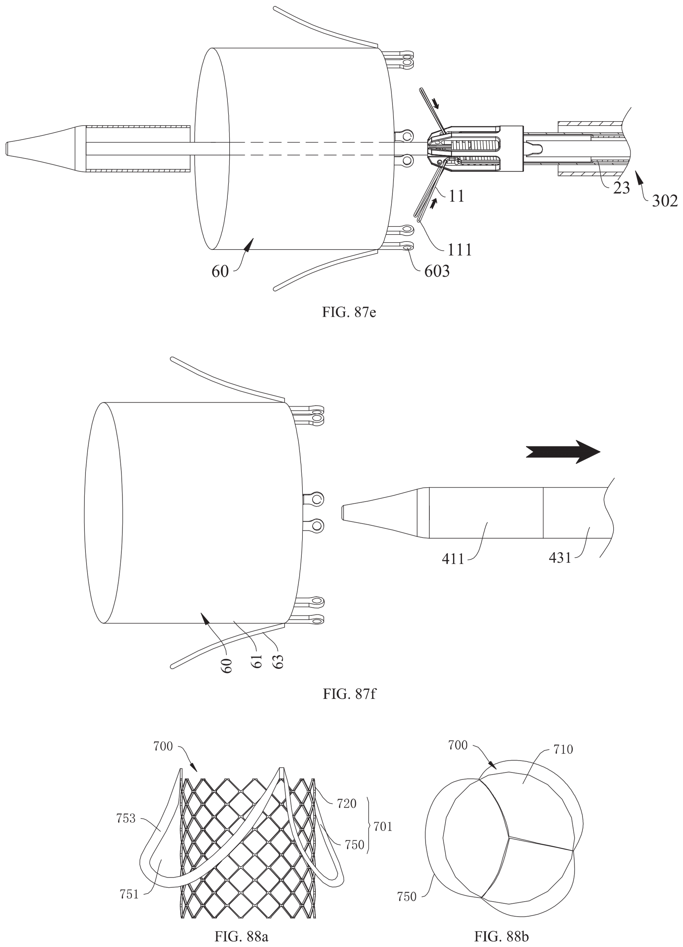

Figs. 87e and 87f are schematic diagrams showing withdrawal of the pulling wires and the first and second loading sections being engaged to withdraw from the body.
Fig. 88a is a schematic diagram of an artificial heart valve in one embodiment.
Fig. 88b is a schematic diagram of the artificial heart valve in Fig. 88a from a top view.

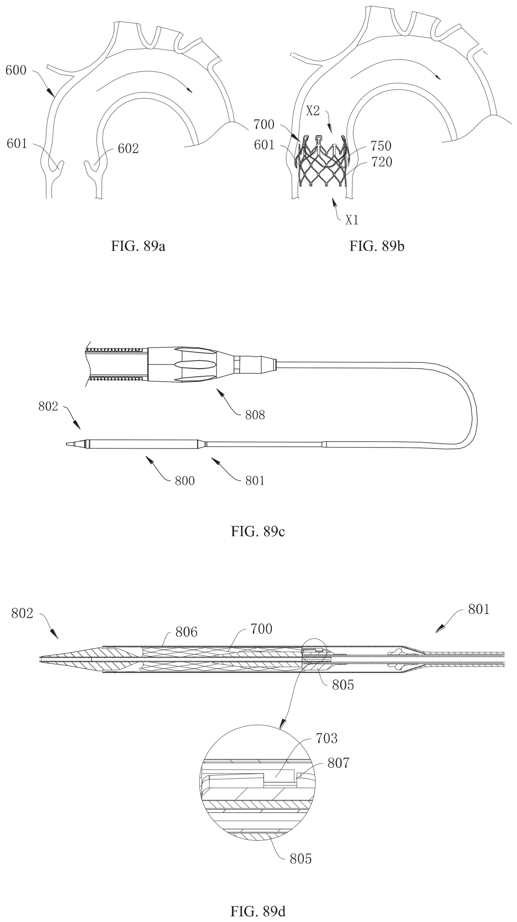

Fig. 89a is a schematic diagram of the aorta.
Fig. 89b is a schematic diagram of the artificial heart valve inserted into the aorta.
Fig. 89c is a schematic diagram of the delivery system.
Fig. 89 d is a schematic diagram of the artificial heart valve within the sheath tube.

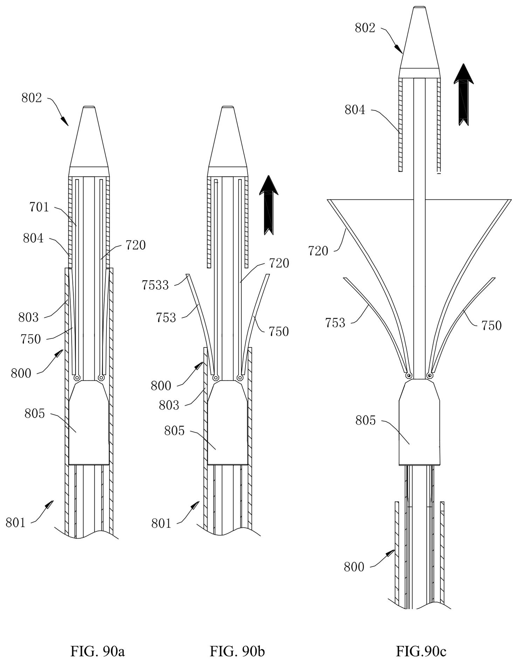

Fig. 90a is a schematic diagram of the artificial heart valve in a compressed state.
Fig. 90b is a schematic diagram of the artificial heart valve in the first stage of the transition state.
Fig. 90c is a schematic diagram of the artificial heart valve in the second stage of the transition state, wherein the distal region of the inner frame is released.

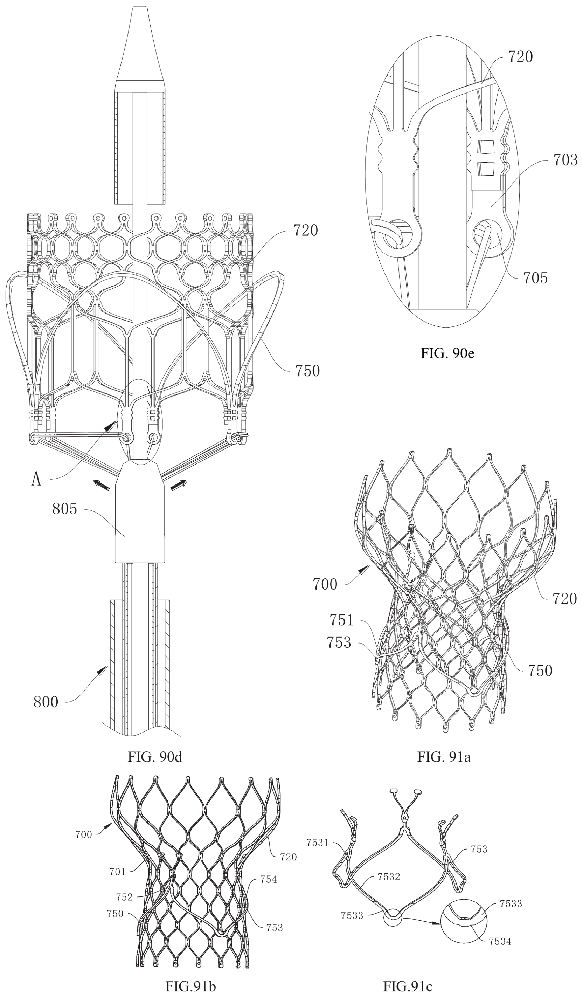

Fig. 90d is a schematic diagram of the artificial heart valve in the released state.
Fig. 90e is an enlarged view of circle A in Fig. 90d.
Fig. 91a is a schematic diagram of the artificial heart valve in one embodiment.
Fig. 91b is a schematic diagram of the artificial heart valve in one embodiment from another aspect.
Fig. 91c is a schematic diagram of the outer frame in Fig. 91a.

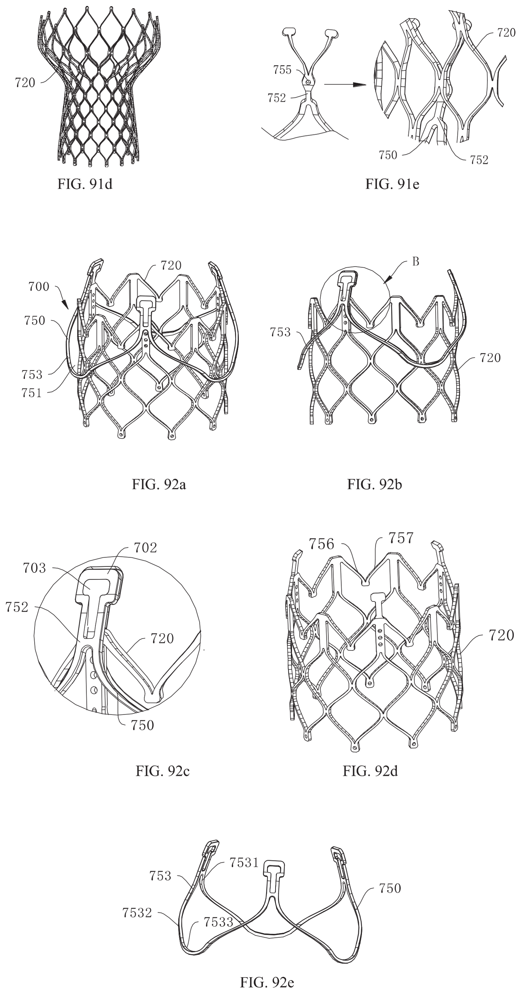

Fig. 91d is a schematic diagram of the inner frame in Fig. 91a.
Fig. 91e is a schematic, partial diagram showing the inner and outer frames in Fig. 91a cooperating with each other.
Fig. 92a is a schematic diagram of an artificial heart valve in one embodiment.
Fig. 92b is a schematic diagram of an artificial heart valve in one embodiment from another aspect.
Fig. 92c is an enlarged view of circle B in Fig. 92b.
Fig. 92d is a schematic diagram of the inner frame in Fig. 92a.
Fig. 92e is a schematic diagram of the outer frame in Fig. 92a.

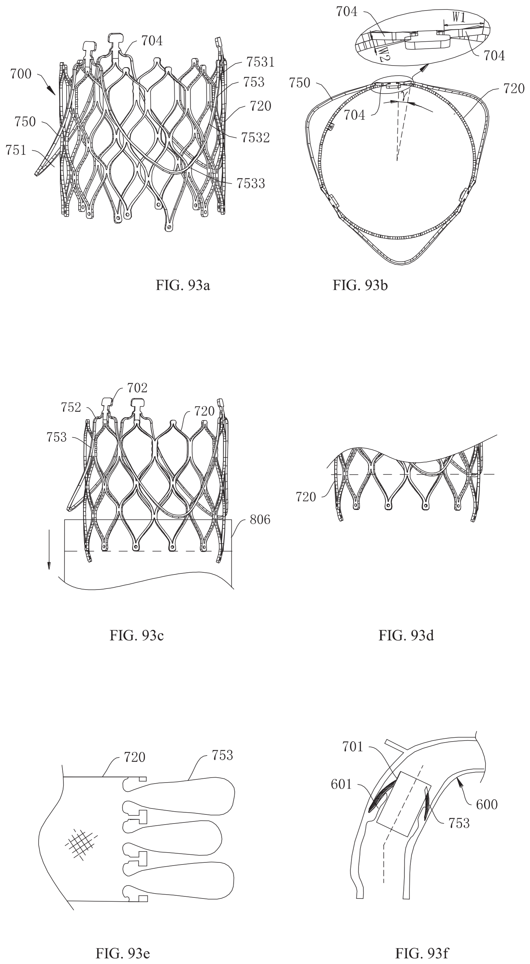

Fig. 93a is a schematic diagram of an artificial heart valve in one embodiment.
Fig. 93b is a schematic diagram of the artificial heart valve in Fig. 93a from a top view.
Fig. 93c is a schematic diagram of an artificial heart valve in one embodiment from another aspect.
Fig. 93d is a schematic, partial diagram of the support points of the inner frame in Fig. 93c.
Fig. 93e is a schematic, partial diagram of an artificial heart valve in one embodiment.
Fig. 93f is a schematic diagram of the artificial heart valve in Fig. 93a inserted into the aorta.

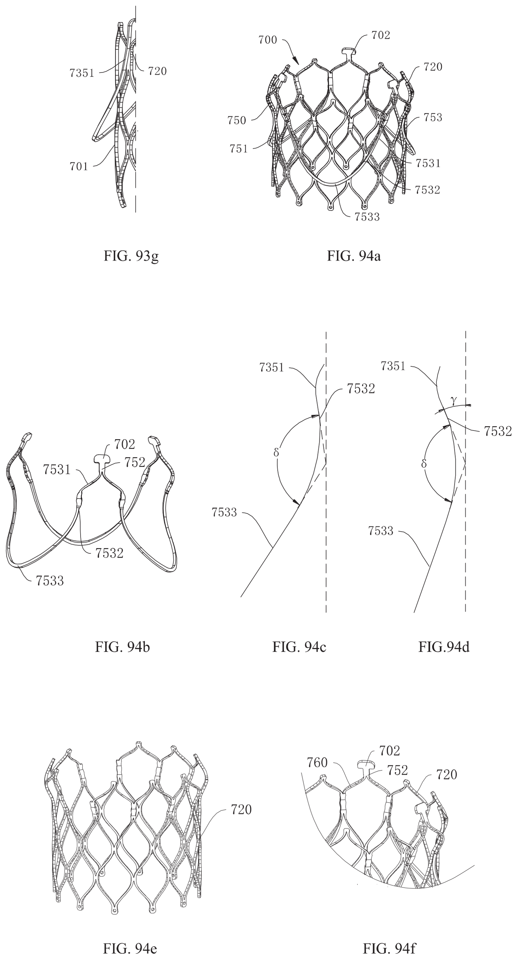

Fig. 93g is a schematic, partial diagram of an artificial heart valve in one embodiment.
Fig. 94a is a schematic diagram of an artificial heart valve in one embodiment.
Fig. 94b is a schematic diagram of the outer frame in Fig. 94a.
Fig. 94c is a schematic diagram of the arms in a compressed state.
Fig. 94d is a schematic diagram of the arms in a released state.
Fig. 94e is a schematic diagram of the inner frame in Fig. 94a.
Fig. 94f is a schematic, partial diagram of an artificial heart valve in one embodiment.

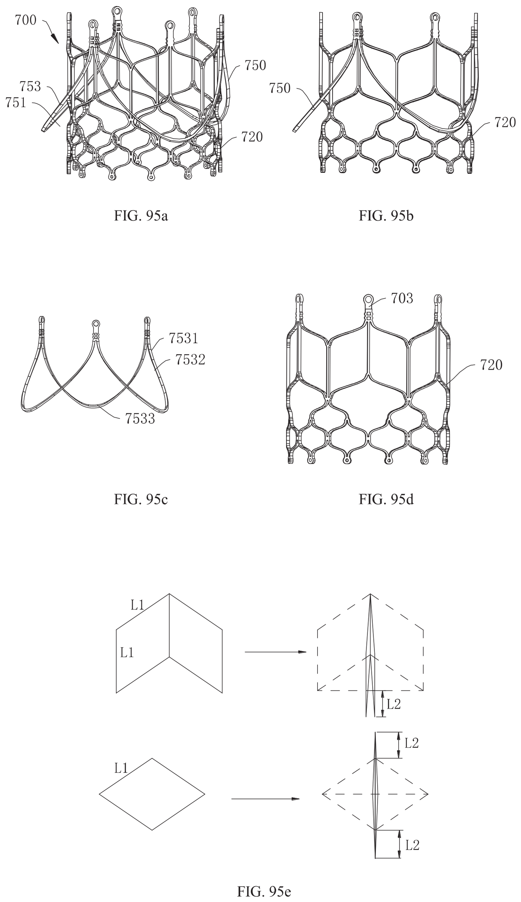

Fig. 95a is a schematic diagram of an artificial heart valve in one embodiment.
Fig. 95b is a schematic diagram of an artificial heart valve in one embodiment from another aspect.
Fig. 95c is a schematic diagram of the outer frame in Fig. 95a.
Fig. 95d is a schematic diagram of the inner frame in Fig. 95a.
Fig. 95e is a schematic diagram showing the parallelogram and rhombus after compression.

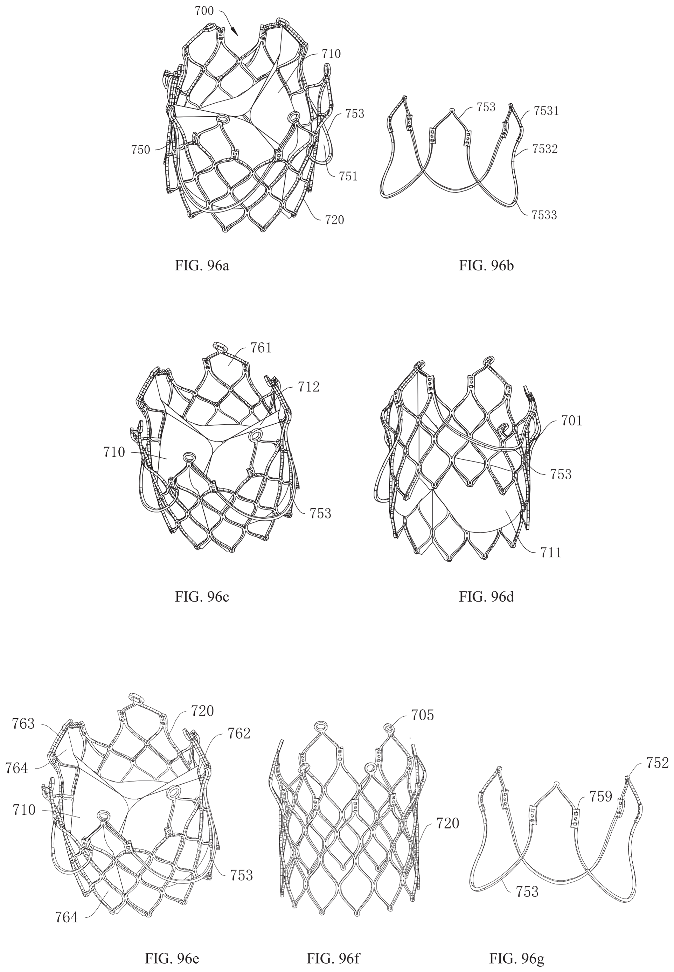

Fig. 96a is a schematic diagram of an artificial heart valve in one embodiment.
Fig. 96b is a schematic diagram of the outer frame in Fig. 96a.
Fig. 96c is a schematic diagram of the artificial heart valve in one embodiment.
Fig. 96d is a schematic diagram of the artificial heart valve in Fig. 96c from another aspect.
Fig. 96e is a schematic diagram of an artificial heart valve in one embodiment.
Fig. 96f is a schematic diagram of the inner frame in Fig. 96a.
Fig. 96g is a schematic diagram of the outer frame in Fig. 96a.

The reference numerals in the drawings are as follows:
10, base; 101/101a/101b, locking region; 103, first cavity; 105/105a/105b/105c/105d/105e/105f, locking hole; 1031, first opening; 1032, annular structure; 1033, second opening; 1034, side wall; 1035/1035a/1035b, rib; 1037, bend section; 1039, guide groove;
11/11a/11b/11c, pulling wire; 111/111a/111b/111c, free end; 112, forward section; 113, control end; 114, backward section; 115, wire loop; 117, detour section;
13, locking member; 131, positioning portion; 135, connecting portion; 1311, tail end; 1313, head end; 1315, axial gap; 1315a, second axial gap; 1315b, first axial gap; 1317, inner space; 1351, linkage key; 1353, avoidance zone; 1355, cutout;
2, catheter assembly; 21, first shaft; 22, transmission member; 23, second shaft; 24, intervention component; 25, third shaft; 28, bend adjusting member; 211, linkage groove; 213, flared structure; 214, first section; 215, transmission sleeve; 216, second section; 217, reinforcement tube; 241, first intervention component; 242, second intervention component; 271, mating portion; 2111, limiting portion; 2151, sliding groove;
3, control handle; 301, distal region; 302, proximal region;
31, first handle; 311, connecting head; 312, end cap; 313, mounting portion; 314, retaining ring; 3131, mounting channel; 3132, radial groove;
32, second handle; 321, insertion section; 3211, distance marker; 330, support body; 3301, guide groove; 3301a, first guide groove; 3301b, second guide groove; 3302, guide rail; 3303, strip-shaped member; 3304, blocking member;
331, first support body; 332, second support body; 3321, connecting head; 333, sleeve; 3331, support frame; 3332, mounting zone; 3333, partition plate; 3334, connecting plate; 3335, connecting seat; 334, mounting seat; 3341, mounting groove;
340, transmission member; 341, first transmission member; 342/342a/342b, second transmission member; 3401, mounting hole; 3402, toothed structure; 3402a, first toothed structure; 3402b, second toothed structure; 3403, shoulder; 3404, axial positioning groove;
351, first drive sleeve;
36, inner shaft drive mechanism; 361, inner cylinder; 361a, first inner cylinder; 361b, second inner cylinder; 3611, retaining groove;
362/362a/362b, force-applying component;
363, first annular body; 364, second annular body; 365, third annular body; 366, operating button; 367, elastic member; 368, fourth annular body; 3631, gear teeth; 3641, retaining ring; 3661, locking portion;
37, bend adjusting drive mechanism; 371, winding wheel; 372, knob; 374, limiting member; 375, elastic member; 376, drive assembly; **3711,** ratchet; 3741, pawl; 3761, toggle button;
380, outer shell; 381, limiting portion;
40, pipe fitting; 41, inner shaft assembly; 411, second loading section; 413, movable shaft; 415, controlled member; 43, sheath tube; 431, first loading section; 4151, limiting block; 45, wire control assembly;
60, artificial implant; 61, inner frame; 63, arm; 601, wire control end; 603, hole; 605, connecting lug;
600, aorta; 601, native valve leaflets; 602, sinus;
700, artificial heart valve; 701, stent; 702/703, connecting lug; 704, transition portion; 705, hole structure; 710, valve leaflets; 711, fixed edge; 712, movable edge; 720, inner frame; 750, outer frame; 751, first gap; 752, junction; 753, arm; 7531, end portion; 7532, middle portion; 7533, head portion; 7534, anti-slip groove; 754, isolation gap; 755, suture hole; 756, first frame bar; 757, second frame bar; 759, positioning hole; 760, common mesh; 761, enlarged portion; 762, joint; 763, end mesh; 764, membrane;
800, catheter assembly; 801, proximal region; 802, distal region; 803/804, loading section; 805, mounting head; 806, sheath tube; 807, locking slot; 808, control handle.

### DESCRIPTION OF THE EMBODIMENTS

The following will provide a clear and complete description of the technical solutions in the embodiments of this application, in combination with the accompanying drawings. Obviously, the described embodiments are only a part of the embodiments of this application, not all of them. Based on the embodiments of this application, all other embodiments obtained by those skilled in the art without creative labor are within the scope of protection of this application.

It should be noted that when a component is referred to as "connected" to another component, it may be connected to another component directly or there may be a middle component connected therebetween. When a component is considered to be "set on" another component, it may be set on another component directly or there may be a middle component set therebetween.

Unless otherwise defined, all technical and scientific terms used in this disclosure have the same meanings as those commonly understood by those skilled in the art belonging to This application. The terms used in the disclosure of this application are only for the purpose of describing specific embodiments and are not intended to limit the scope of this application. The term "and/or" used in this disclosure includes any and all combinations of one or more related listed items.

In this application, the terms "first", "second", etc. are only used for descriptive purposes and should not be understood as indicating or implying relative importance or implying the quantity or order of the indicated technical features. Thus, the features limited to "first" and "second" may explicitly or implicitly include one or more of these features. In the description of this application, the meaning of "multiple/plurality of" refers to at least two, such as two, three, etc., unless otherwise specified.

This disclosure describes an artificial implant and a delivery system for delivering the artificial implant into a human body. The delivery system includes a control handle and a catheter assembly, wherein the control handle may be connected to and control the catheter assembly which is used for performing interventional surgery. The catheter assembly includes a plurality of controlled components, and distal regions of the controlled components cooperate with each other to operate the artificial implant, such as releasing, withdrawing, locking position, adjusting spatial posture, etc. Each controlled component may be configured as a hollow tube, a solid rod, a flexible wire, or a combination of various forms. There are multiple controlled components, and at least two of them (taking the proximal region as an example) can slide relative to each other along the axial direction or rotate relative to each other about the axis. The force applying components on the control handle (i.e., the parts that the user directly operates and contacts), which are used to operate the controlled components, can be connected to the corresponding controlled component directly and fixedly for transmission, or, threads and rack-and-pinion may be used for transmission therebetween.

In the following description, many improvements regarding control handles or local structures can be implemented on the same control handle without obvious technical contradictions, but are not strictly limited to being implemented on the same control handle. For different numbers of controlled components and motion characteristics, or for control handles with further simplified structures, the embodiments may be implemented separately or combined appropriately.

The application site and structure of artificial implants are not strictly limited. Taking artificial heart valves as examples in some drawings or texts, the artificial heart valve generally includes a deformable stent and leaflets connected to the stent. The stent in whole is cylindrical-shaped, with a sidewall thereof being configured as a hollow mesh structure. Unless otherwise specified, the shape or size of the mesh structure is not strictly limited. An interior of the stent is configured as a blood flow channel, and multiple leaflets cooperate with each other to control the degree of opening and closing of the blood flow channel inside the stent. In order to locate within the body, positioning structures that can interact with surrounding native tissues, such as anchor spikes, arms, etc., may be provided around the stent. To prevent leakage, skirts or anti-leakage materials may be provided on the interior and/or exterior of the stent.

According to different expansion modes, the stent is processed using corresponding materials, such as nickel titanium alloy with shape memory and self-expanding ability, or stainless steel material for balloon expansion. The stent itself may be formed by cutting pipes or weaving wires, and the leaflets may be connected to the stent by sewing, bonding, or integral molding.

Taking the self-expanding stent as an example, its expansion and retraction may be controlled by a sheath catheter wrapped around the outer circumference of the stent. According to the different parts of the stent exposed to the sheath catheter, corresponding control may be carried out. The stent may also be controlled by a pulling wire, which passes through the structural gap (or wire hole) of the stent. By adjusting the tension of the pulling wire through the control handle, the degree of expansion of the stent can be changed. When the pulling wire is pulled away from the stent, the stent is allowed to be completely released. Of course, the control of the pulling wire is also achieved through various controlled components in the catheter assembly.

The stent of artificial implant generally has a connecting structure that matches with the catheter assembly to limit positions relative to each other and prevent unnecessary positional deviation during delivery. The artificial implant is in a radially compressed state when delivery, that is, in a loaded state. After releasing the constraint of the catheter assembly and expanding radially in the human body, the stent is in an expanding state. Unless otherwise specified, the shape of the artificial implant is understood as the shape in the expanding state, without considering the local deformation caused by surrounding tissue pressure.

Due to the complexity of the internal of the human body, the catheter assembly often requires bending operations. Corresponding bending components may be configured as tubes or wires, with the distal region thereof acting on the bend adjusting member and the proximal region thereof adjusting the bending amplitude or direction through a control handle.

When used to indicate direction, the proximal region in the disclosure generally refers to the side adjacent to the operator (such as a doctor), and the distal region is the side relatively far away from the operator. Along the intervention path, each component has its own distal and proximal regions that are opposite to each other. In theory, when the catheter assembly and control handle are completely straightened, a straight line between the proximal region and the distal region determines the axial direction, and correspondingly determines the radial direction which is perpendicular to the axial direction and the circumferential direction which is around the axial direction. When used to refer to a structure, the term "end" in the disclosure refers to the endpoint, or a point or region in this direction, or a specific structure connected to that point or region, of the structure.

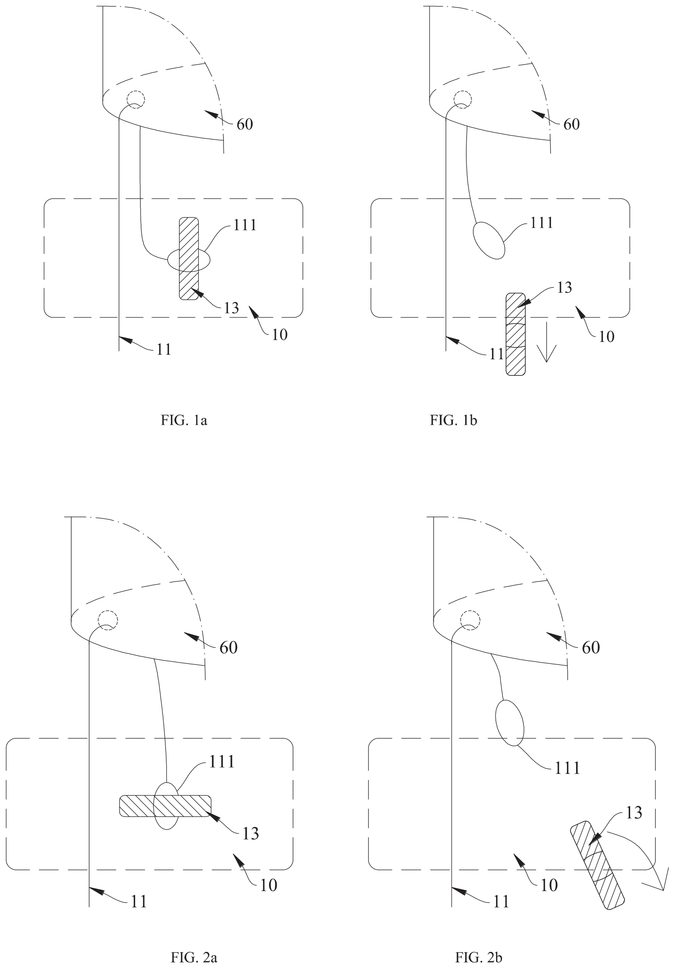

This application provides a control mechanism for an artificial implant, including a base 10, a pulling wire 11, and a locking member 13, wherein the pulling wire 11 has a free end 111 that can pass through the artificial implant or be detached from the artificial implant 60. The free end 111 has a first state (as shown in Fig. 1a) in which it is restricted to the base 10 and a second state (as shown in Fig. 1b) in which the restriction is released. The locking member 13 is movably engaged with the base 10. The locking member 13 cooperates with the free end 111 of the pulling wire 11 to switch the state of the free end 111.

The free end 111 is the end of the pulling wire 11 that first passes through and last exits the artificial implant 60, which may also be understood as the distal end of the pulling wire 11 when straightened. The other end of the pulling wire 11 opposite the free end 111 is a control end, which can be directly fixed to the base 10 or controllably extended proximally.

In the first state, after the free end 111 engages with the locking member 13, the locking member 13 cooperates with the base 10 to prevent the pulling wire from detaching from the locking member 13. In this state, the free end 111 may be understood as being relatively fixed to the locking member 13. In this state, the pulling wire 11 is always connected to the artificial implant 60, and the release process (i.e., the degree of expansion) of the artificial implant is adjusted based on the length of the pulling wire exposed from the base. The release/folding speed of the artificial implant is also controlled based on the rate of change of the pulling wire. If necessary, the pulling wire can also be used to withdraw the artificial implant.

In the second state, the free end 111 disengages from the locking member 13 and the artificial implant 60 in sequence, releasing the interconnection between the artificial implant and the control mechanism. The control mechanism can then be withdrawn from the body, leaving the artificial implant in a predetermined position within the body.

In one embodiment, the control mechanism for the artificial implant includes a base 10, a pulling wire 11, and a locking member 13. The base 10 has a locking region 101. The pulling wire 11 has a free end 111 that can pass through the artificial implant or be detached from the artificial implant 60. The free end 111 has a first state (as shown in Figs. 1a and 2a) where it is restricted in the locking region 101, and a second state (as shown in Figs. 1b and 2b) in which the restriction is released. The locking member 13 rotatably engages with the base 10 and has a positioning portion 131 that moves into or out of the locking region 101 during rotation. The positioning portion 131 cooperates with the free end 111 of the pulling wire 11 to switch the state of the free end 111.

The locking region 101 can be positioned within the overall outer contour of the base 10 to accommodate the free end in the first state. Furthermore, the locking region 101 can be open distally to optimize the extension path of the pulling wire 11.

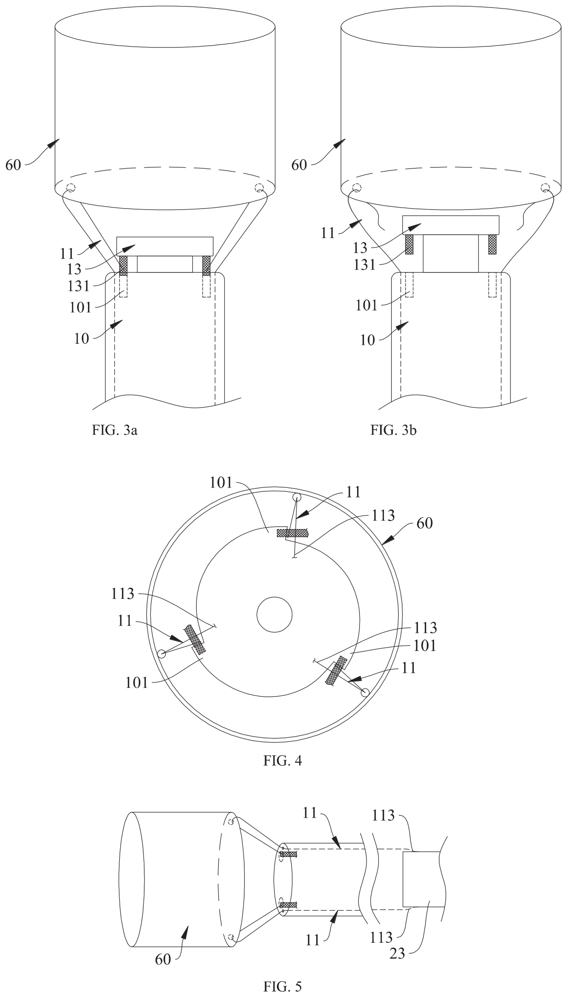

In conventional technology, for example, Figs. 3a and 3b illustrate that the locking member 13 slides axially to make the positioning portion 131 be disengaged from the locking region 101, thereby allowing the pulling wire 11 to be detached from the artificial implant.

In this embodiment, the positioning portion 131, acting as a part of the locking member 13, is displaced at least in the circumferential direction during operation, which can reduce or even eliminate the axial stroke change of the locking member 13, reduce the axial space occupied by related components and even the proximal control handle, being more conducive to the configuration of the transmission mechanism.

The pulling wire 11 has a control end 113 opposite the free end 111. The control end 113 is movable relative to the base 10. When the free end 111 is in the first state, the length of the pulling wire 11 exposed outside the base 10 can be adjusted by operating the control end 113. When the free end 111 is in the second state, the control end 113 can be operated to drive the pulling wire 11 away from the artificial implant 60. The control end 113 can extend to and be controlled by a control handle. The movement manner of the control end 113, specifically such as axial movement, rotation, winding, and etc., can change the length of the pulling wire exposed outside the base 10.

The artificial implant 60 is a cylindrical structure with a corresponding circumferential direction. Multiple pulling wires 11 are configured, and the control ends 113 of the pulling wires 11 can be controlled independently or move synchronously. The positions where the pulling wires 11 interact with the artificial implant 60 are spaced apart along the circumferential direction of the artificial implant 60, which improves the synchronization of the folding and release of the artificial implant 60. For example, as shown in Fig. 4, there are three pulling wires 11, which interact with the artificial implant 60 at three positions and are arranged at intervals along a circumferential direction of the artificial implant.

The control ends 113 of the pulling wire 11 can extend directly to the control handle or be connected to the control handle for transmission through an intermediary component to reduce the risk of multiple pulling wires 11 becoming entangled. For example, as shown in Fig. 5, the control mechanism also includes a second shaft 23, to which the control ends 113 of the pulling wires 11 are connected. Operation of the second shaft 23 synchronizes the control of the pulling wires 11.

Correspondingly, there are multiple locking regions 101, and in the first state, the free end 111 of each pulling wire 11 corresponds to one of the locking regions 101. This prevents interference between the pulling wires 11. The multiple locking regions can be separated by the base structure itself, or additional separation components can be provided on the base.

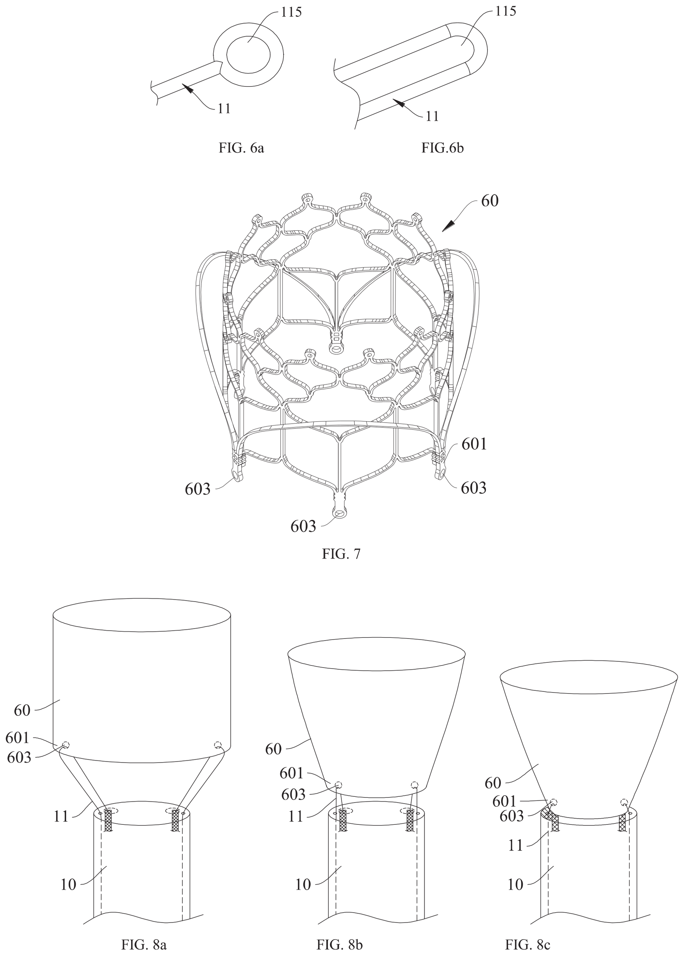

Regarding the cooperation between the free end 111 and the positioning portion 131, in one embodiment, the free end 111 is configured with a wire loop 115. When the free end 111 is in the first state, the positioning portion 131 extends into the wire loop 115. When the free end 111 is in the second state, the positioning portion 131 is removed from the wire loop 115. The wire loop 115 can be configured independently (as shown in Fig. 6a) or can be formed by winding the pulling wire 11 (as shown in Fig. 6b). The pulling wire 11 can be single or multiple strands. For example, the pulling wire 11 can be a single strand extending to the control end 113, or two strands extending in parallel to the control end 113, or two strands extending in parallel for a period of time and then combined to extend to the control end 113.

In one embodiment, this application provides a control mechanism for an artificial implant, including a base 10, a pulling wire 11, and a locking member 13. The pulling wire 11 has a free end 111 that can pass through the artificial implant or be detached from the artificial implant 60. The free end 111 has a first state where it is restricted to the base 10 and a second state in which the restriction is released. The locking member 13 movably engages with the base 10 and cooperates with the free end 111 of the pulling wire 11 to switch the state of the free end 111.

As shown in Fig. 7, the artificial implant 60 has an axial direction, one end of the artificial implant 60 is configured as a wire control end 601, which can be either the distal region or the proximal region of the artificial implant 60. The wire control end 601 has holes 603 for the pulling wires (not shown in the Fig. 7 for clarity) to pass through.

The holes can be formed as follows:
the artificial implant includes a hollow portion configured with the holes, wherein the hollow portion is a type of structural gap within the artificial implant; or
the holes may be formed by direct drilling (e.g., Fig. 7); or
the holes can be independently configured within the artificial implant (e.g., by welding).

The holes 603 are multiple and spaced from each other. In an expanding state, one pulling wire 11 passes through at least two holes 603.

In the first state, the free end 111 of the pulling wire 11 can prevent the artificial implant 60 from completely disengaging from the control mechanism. However, by adjusting the length of the pulling wire exposed outside the base, the artificial implant can be deformed to a certain extent, which may also be understood as that a position of the artificial implant relative to the base can be changed. Therefore, when the free end 111 of the pull wire is maintained in the first state, the artificial implant 60 can still have multiple states according to its own deformation degree, for example:
an expanding state, in which the wire control end 601 radially expands away from the base 10 (as shown in Fig. 8a), and a round-trip path of the same pulling wire 11 winding around the artificial implant 60 does not repeat;
a folding state, in which the wire control end 601 radially folds toward the base 10 (as shown in Fig. 8c);
an intermediate state, in which the wire control end 601 is between the expanding state and folding state (as shown in Fig. 8b).

As shown in Figs. 7-9a, multiple holes 603 are arranged at intervals along the circumferential direction. In the expanding state, the same pulling wire 11 passes through at least two holes 603, which means that one pulling wire 11 can act on two circumferential positions of the wire control end 601. In a preferred embodiment, the number of holes 603 is twice the number of the pulling wires 11, with each pulling wire 11 corresponding to two holes 603. This ensures synchronized contraction and expansion of the wire control end 601 at various points along the circumferential direction of the artificial implant 60, reduces the number of pulling wires, and avoids entanglement and friction at the proximal regions of the pulling wires, finally reducing the space required to accommodate the pulling wires.

For the artificial implant 60, at least two wires are configured. In addition, for the same hole, the pulling wire is only allowed to be threaded once to avoid interference caused by reciprocating threading.

As shown in Fig. 9a, in this state, an extending direction (radial direction) of the pulling wire between the artificial implant 60 and the base 10, i.e., the direction of the pulling force, is perpendicular to the movement direction (circumferential direction) of the locking member for unlocking, making unlocking easier.

For example, as shown in Fig. 9, the round-trip path of the same pulling wire **11** through the artificial implant generally encloses a triangular area. This means that the forward path and the return path do not overlap. Otherwise, if the return path overlaps the forward path, the extension path of the pulling wire 11 will be a single straight line or curve line, which will not enclose a specific area. The pulling wire 11b is a state after threading is completed, and the bolded portions represent the forward path 112 and the return path 114 of the pulling wire 11b.

As shown in Figs. 9a and 10, specifically, a triangular threading path is formed. In the first state, the pulling wire 11a extends through the current locking region 101 out of the base 10, wraps around the artificial implant 60, and then the free end 111 returns back to the same locking region 101. After threading, the pulling wire 11 forms a wire loop 115 at a the position where it warps around the artificial implant. As shown in Fig. 9a, the pulling wire 11b forms two action points (X1 and X2) in the locking region 101, as well as two action points (Y1 and Y2) between the pulling wire 11b and the artificial implant.

Here is an explanation based on the existing technology: in Fig. 11a, three holes 603 arranged at intervals along the circumferential direction are defined in the artificial implant 60, and correspondingly there are three pulling wires. The specific threading path of the pulling wire may refer to that of the pulling wire 11c, being not repeated. During the folding process, the pulling wires exert only radial forces. The artificial implant generates corresponding stresses in the circumferential direction, which react on the pulling wires, gradually increasing the force required to apply the pulling wires and affecting the operational feel.

However, in this embodiment, the pulling wire after threading is shaped as a triangle. In addition to generating radial forces, they also generate forces along the line connecting the two action points (Y1 and Y2) (indicated by the solid arrows in Fig. 9a) to directly overcome the stress changes during the artificial implant's deformation. This results in a smaller change in the overall pulling wire force compared to existing methods, resulting in a more refined operational feel.

In conventional techniques, the spacing between adjacent holes 603 is relatively wide (i.e., the circumferential span is large). The holes 603 are defined in connecting lugs 605 (as shown in Fig. 11b). Adjacent connecting lugs 605 are independently connected to the pulling wires. During the folding process, adjacent connecting lugs 605 may end up misaligned and overlapping (as shown in the bold portion of Fig. 11c), affecting subsequent release. However, the extension path of the pulling wire in this embodiment allows the connecting lugs 605 and the structural gaps to be largely or entirely controlled by the pulling wires, reducing or even eliminating the problem of misalignment and overlapping during folding (as shown in Fig. 9b).

Since the two action points (X1 and X2) are located in the same locking region and adjacent to each other, the return path of the pulling wire forms a roughly equilateral triangle. This means that the bolded forward path 112 and return path 114 in Fig. 9a are of equal length, resulting in the same movement rates for the corresponding forward path 112 and return path 114, improving the synchronization of folding and expansion of the artificial implant 60. If the circumferential span between the two action points (X1 and X2) is large, as shown in Fig. 12, the corresponding forward path 112 and return path 114 have different lengths, resulting in different movement rates and abnormal folding/expansion of the artificial implant.

To improve the synchronization of folding and expansion of the artificial implant, existing technologies require a larger number of pulling wires (e.g., six or more). However, this requires consideration of the spatial arrangement of the pulling wires at the proximal region, which may lead to friction or entanglement due to the close proximity of the pulling wires. The threading method of this embodiment reduces the number of pulling wires required, thereby meeting space requirements.

Regarding the positioning portion 131, there are two axial gaps 1315 defined at two sides of the positioning portion 131 along the axial direction of the base, configured for the radial reciprocating threading of the pulling wire 11 and/or the winding of the free end of the pulling wire 11. In one embodiment, the positioning portion 131 is a multi-turn helical structure with axial gaps 1315 defined between adjacent turns. In the first state, the pulling wire 11 extends out of the base through a current axial gap 1315, wraps around the artificial implant 60, and then the free end sleeves onto a turn adjacent to the current axial gap 1315 (see Fig. 13a).

As shown in Fig. 14, the base 10 has a distal region 301 and a proximal 302 that are opposite to each other, and an axial direction extending between the distal and proximal regions 301 and 302. The base 10 has a first cavity 103 defined therein, a first opening 1031 communicating with the first cavity 103 is defined in a proximal region side of the base 10, and a second opening 1033 communicating with the first cavity 103 is defined in an outer circumference of the base 10.

As shown in Fig. 15, the pulling wire 11 passes through the first cavity 103. One end (i.e., the control end 113) of the pulling wire 11 extends proximally out of the base 10 through the first opening 1031. The other end (i.e., the free end 111) of the pulling wire 11 extends out of the base 10 through the second opening 1033 for connecting the artificial implant.

In this embodiment, referring to Figs. 16a and 16b, the locking region 101 is located at the second opening. Along the circumferential direction of the base 10, there are multiple locking regions 101 arranged at intervals. The positioning portion 131 sequentially enters or exits each locking region 101 as the locking member 13 moves.

In one embodiment, as shown in Fig. 15, the control mechanism further includes a third shaft 25 fixedly connected to the base 10. The proximal region of the third shaft 25 extends to and can be controlled by a control handle. The third shaft 25 maintains the base 10 relatively fixed in the circumferential direction, allowing the positioning portion 131 to rotate relative to the base 10.

In one embodiment, multiple separating members are arranged on the base 10 circumferentially. Adjacent separating members define a second opening 1033. Consequently, multiple second openings 1033 are arranged at intervals along the circumferential direction of the base 10. The separating members are configured as ribs 1035, as shown in Figs. 16a and 16b. The strip-shaped structure minimizes its circumferential span, and thus maximizing the circumferential span of the second opening. This facilitates the extending of the pulling wires. For example, the pulling wire can extend out of a locking region and then return to the same locking region; or the pulling wire can extend out of a locking region and then return to an adjacent locking region, ensuring that the two action points are sufficiently close. The ribs also serve as reinforcements to maintain the basic strength requirements of the base.

In the first state, the free end 111 extends out of the base 10 through the corresponding second opening 1033, wind around and passes through the artificial implant 60, and then returns to the locking region 101 corresponding to the same second opening 1033 (Fig. 17a), or returns to a position circumferentially adjacent to the second opening 1033 (Fig. 17b).

The position circumferentially adjacent to the second opening 1033 may be another adjacent locking region or a separate channel for extending of the pulling wire. This channel may be formed using the same structural principles as the locking region. For example, there are six second openings 1033, three of which are spaced apart from each other are configured for the extending of the free ends of the pulling wires out of the base, while the remaining three of which are spaced apart from each other serve as the locking regions. For example, as shown in Fig. 17c, the circumferential span of the locking region 101 is α, and the circumferential span of the second opening 1033 is β, wherein α/β = 1.1-2.

All of the ribs 1035 are converged and fixed at the distal region of the base 10, forming an annular structure 1032. At the proximal region of the base 10, all of the ribs 1035 are continuously distributed circumferentially to form a cylindrical structure.

In one embodiment, the base 10 defines a locking hole 105 for inserting the positioning portion 131 therein. The positioning portion 131 includes:
a locking state, in which the positioning portion 131 is inserted into the locking hole 105, preventing the free end 111 of the pulling wire 11 from disengaging (as shown in Fig18a);
an unlocking state, in which the positioning portion 131 exits the locking hole 105, allowing the free end 111 of the pulling wire 11 to disengage (as shown in Fig18b).

The locking region 101a is formed between two ribs 1035a and 1035b. In the first state, the free end 111 of the pulling wire 11 is located in the locking region 101. Locking holes 105a and 105b are defined on opposite sides of the two ribs. Alternatively, the locking holes can extend through the ribs. A section of the positioning portion 131 is located in the locking region 101a, configured with two ends of this section being inserted into the locking holes 105a and 105b, respectively.

Based on the moving direction of the locking member 13, an open direction of the locking hole 105 is oriented circumferentially of the base 10.

As shown in Figs. 18a to 19, when the positioning portion 131 moves in the first direction (as indicated by the X in Fig. 19), the free end 111 switches from the second state to the first state. The locking hole 105 extends through the rib 1035 along the first direction. The locking hole 105 has a forward opening and a rearward opening opposite to each other, wherein the forward opening faces the first direction.

There are multiple ribs 1035 configured with locking holes 105. The positioning portion 131 has a tail end 1311 and a head end 1313 opposite to each other. The head end 1313 are sequentially inserted into (as shown in Fig. 18a) or disengaged from (as shown in Fig. 18b) each locking holes 105 as the locking member 13 rotates. Taking the sequential insertion of the positioning portion as an example, as shown in Figs. 18c-18e, the head end 1313 sequentially engages into the locking hole 105a, the free end 111a, the locking hole 105b, the locking hole 105c, the free end 111b, the locking hole 105d, the locking hole 105e, the free end 111c, and the locking hole 105f. Sequential disengagement is the reverse of sequential engagement.

In a preferred embodiment, all of the ribs 1035 are configured with locking holes 105, allowing the positioning portion 131 to rotate through them sequentially and providing a certain degree of movement guidance. During the rotational insertion of the locking member into each locking hole, the head end 1313 finally abuts against the sidewall 1034 of one rib 1035 (as shown in Fig. 18a), or engages into the corresponding locking hole, thereby acting as a stop.

When the positioning portion 131 is configured as a multi-turn helical structure, the locking holes 105 of each rib 1035 are positioned to align with the positioning portion 131. There are one or multiple locking holes 105 on each rib 1035. The multiple locking holes 105 are arranged sequentially along the extension direction of the rib 1035.

A side of the rib 1035 facing the first cavity 103 is configured as an inner side. The inner side of each rib 1035 is provided with a guide groove 1039, through which the positioning portion 131 extends. The axially penetrating guide groove 1039 allows the positioning portion 131 to rotate through, and the proximal region of the guide groove 1039 is open for the positioning portion 131 to be positioned and inserted into the first locking hole 105c after the locking member 13 is rotated (as shown in FIG. 20a ). In one embodiment, the depths of the guide grooves corresponding to the ribs (H in Fig. 20b) are different, ensuring that the helical positioning portion 131 is properly guided.

Referring to Fig. 20a, in one embodiment, one axial section of the rib 1035 is a bend section 1037 protruding radially and inwardly. The locking hole 105 is located on the bend section 1037 of the corresponding rib 1035. The bend section 1037 is located at the distal region, forming a chamfered or rounded corner on the distal region side of the base, which serves as a guide for the folding of the artificial implant.

Taking into account the rotational characteristics of the locking member and the arrangement of the pulling wires in the circumferential distribution, the positioning portion 131 is rod-shaped, which may be straight rod-shaped or curved rod-shaped. There is only one rod, which mates with the free ends 111 of all the pulling wires 11. This eliminates axial dimensional variations in the control mechanism while retaining the original locking member's ability to mate with the pulling wires individually.

In one embodiment, the base 10 defines a locking hole 105, the opening of which faces the circumference of the base 10. The positioning portion 131 is curved and extends around the base axis. The positioning portion 131 is inserted into and disengaged from the locking hole 105 as the locking member 13 rotates. The bend shape fully utilizes the circumferential space, maintains the necessary length of the locking member, and allows the same locking member to control the free ends of all pulling wires.

As shown in Fig. 21, at least a part of the locking member 13 is configured as a connecting portion 135 located within the first cavity 103. The control mechanism also includes a first shaft 21 that is in driving engagement with the connecting portion 135 to drive the locking member 13 relative to the base 10. The proximal region of the first shaft 21 is connected to and controlled by the control handle.

In one embodiment, the pulling wire 11 has a control end 113 opposite to the free end 111. The control end 113 is movable relative to the base 10. The control mechanism also includes a second shaft 23 connected to the control end 113. The second shaft 23 is tubular and movably mounted around the first shaft 21. The movement of the second shaft 23 drives the pulling wire 11 to gradually contract or expand the artificial implant.

In some embodiments, the positioning portion 131 includes at least one arc-shaped structure that mates with the base. For example, as shown in Figs. 21-23, the positioning portion 131 is a helical structure that winds at least once. For example, the helical structure may be a multi-turn structure, with the turns arranged axially (around the circumference of the base). The generatrix of the helical structure can be an oblique line (away from or closer to the axis of the base) or a straight line. For example, the generatrix of the helical structure is a straight line, which can avoid increasing the radial dimension or reducing the space between the connecting portion 135 and the first shaft 21.

Along the winding direction of the helical structure, the positioning portion 131 generally has a tail end 1311 and a head end 1313. One of the tail end 1311 and a head end 1313is fixed to the connecting portion 135. This fixing method includes separately formed and then connected by, such as welding or assembly, or integral molding. The other end of the positioning portion 131 is relatively free.

In this embodiment, the head end 1313 of the positioning portion 131 mates with the base 10 and the pulling wire, while the tail end 1311 of the positioning portion 131 is fixed to the connecting portion 135, for example, at the outer circumference or distal region of the connecting portion 135. The connection method may be direct welding of the ends or welding after partial overlap.

The connecting portion 135 is tubular, with an interior being used to pass through other components, such as the first shaft 21. To enhance connection strength and avoid the extension of other components, the positioning portion 131 can be spirally wound around the outer circumference of the connecting portion 135. The connecting portion 135 and a part of the positioning portion 131 near the tail end 1311, are located within the first cavity 103 and welded to each other.

The connecting portion 135 is fixed to the distal region of the first shaft 21, or can be axially separated from the distal region of the first shaft 21.

As shown in Figs. 21-23, in one embodiment, the positioning portion 131 is configured as a multi-turn helical structure with axial gaps 1315 between adjacent turns. The helical structure encloses an inner space 1317. At least one section of the pulling wire 11 forms a detour section 117. In the first state, the detour section 117 is located within the inner space 1317, with two ends of the detour section 117 extending outside the inner space 1317 through corresponding axial gaps 1315 (as shown in Fig. 20). The detour section 117 and the positioning portion 131 form a force-bearing point (a first force-bearing point). The free end 111 is sleeved onto a turn of the positioning portion 131 adjacent to the first axial gap 1315b, serving as a second force-bearing point, causing the second shaft 23 to act on the artificial implant to fold.

The positioning portion 131 has at least one and a half turns or more, for example, more than two turns as shown in Fig. 22a, or approximately one and a half turns as shown in Fig. 22b.

Combining Figs. 18 and 20, in the first state, the pulling wire 11 extends distally from the control end 113 to the outside of the positioning portion 131, extends through the second axial gap 1315a and bends to the inner side of the positioning portion 131, and then extends through the second axial gap 1315a to the first axial gap 1315b at the distal region, and then extends inwardly through the first axial gap 1315b and bends to the outside of the positioning portion 131. After winding around the artificial implant, the free end 111 is sleeved onto the positioning portion 131. The detour section 117 is located between the first axial gap 1315b and the second axial gap 1315a, wherein a part of the pulling wire 11 extending from the control end 113 to the second axial gap 1315a is located between the positioning portion 131 and the base 10, as far away from the first shaft 21 as possible, thereby reducing the influence on the fit between the first shaft 21 and the connecting portion 135, and facilitating the fixed connection between the connecting portion 135 and the positioning portion 131.

In another embodiment, this application further provides a control mechanism having a distal region and a proximal region that are opposite to each other and an axial direction extending between the proximal and distal regions. The control mechanism includes:
an intervention component 24, including a first intervention component 241 and a second intervention component 242;
a transmission member 22, a distal region of which is fixedly connected to the first intervention component 241;
a control handle 3, which connects to and controls the proximal region of the transmission member 22; and
a linkage structure including two selectively linked mating portions 271, one of which is fixed to the second intervention component 242 and the other is fixed to the transmission member 22. Movement of the transmission member 22 switches the linkage state of the two mating portions.

In the linkage state, the two mating portions drive the second intervention component 242 to rotate or move linearly. For example, when the transmission member 22 slides axially, the two mating portions are linked or unlinked. As shown in Fig24a, the two mating portions 271 are unlinked, and the transmission member 22 slides axially to make the two mating portions 271 switch to the linkage state as shown in Fig24b, whereby the transmission member 22 can drive the second intervention component 242 to rotate.

Alternatively, when the transmission member 22 rotates, the two mating portions are linked or unlinked. As shown in Fig. 25a, the two mating portions 271 are unlinked, and rotation of the transmission member 22 causes the two mating portions 271 to switch to the linkage state as shown in Fig. 25b, allowing the transmission member 22 to drive the second intervention component 242 to slide.

The two mating portions are configured as a linkage key and a linkage groove which be separated by axial sliding. When the linkage key is inserted into the linkage groove, they are rotationally linked to each other. Alternatively, the two mating portions are configured as a linkage key and a linkage groove which be separated by relative rotation. When the linkage key is inserted into the linkage groove, they are linked to each other axially.

There are multiple linkage keys, and one of the intervention components is partially tubular. The multiple linkage keys are arranged circumferentially along the tubular portion, correspondingly, there are multiple linkage grooves.

Along the axial direction, the first intervention component 241 is located at one side (proximal side) of the second intervention component 242. In certain initial or linkage states, the first intervention component 241 and the second intervention component 242 may partially nest within each other.

In accordance with common application scenarios, the first and second intervention components each include the following components:
an inner shaft assembly, used to carry an artificial implant (e.g., artificial heart valve) before release;
an outer sheath, capable of encasing the artificial heart valve before release;
a base, connected to and axially constrained by the artificial heart valve before release;
a locking member, used to secure the artificial heart valve before release to the base;
a balloon body, capable of carry and expanding the artificial heart valve before release;
a pulling wire, connected to the artificial heart valve to control the release of the artificial heart valve.

Referring to Figs. 26-28b, in combination with the structure of the locking member 13 described above, in one embodiment, the locking member 13 serves as the second intervention component, and the first shaft 21 serves as the first intervention component. At least a part of the locking member 13 is configured as a connecting portion 135. The first shaft 21 matches with the connecting portion 135 in a transmission manner to dsrive the locking member 13 relative to the base 10. The connecting portion 135 is tubular, and its wall is configured with a rotary linkage structure that engages with the distal region of the first shaft. The rotary linkage structure engages or disengages when the first shaft 21 moves axially relative to the connecting portion 135. The connecting portion 135 is located within the base 10, which facilitates the combination with the first shaft 21 in terms of radial space.

The rotary linkage structure includes two selectively engaged portions:
a linkage key 1351, protruding radially from one of the connecting portion 135 and the first shaft 21; and
a linkage groove 211 matching with the linkage key 1351, wherein the linkage groove 211 is provided on the other one of the connecting portion 135 and the first shaft 21, opening onto an end surface of the other one. For example, as shown in Figs. 28a and 28b, a linkage key 1351 is provided on the connecting portion 135, and a linkage groove 211 is provided on the first shaft 21. The linkage key 1351 slides axially to switch between the two states. The rotational linkage between the two portions is manifested by a sidewall of the linkage groove 211 abutting against a sidewall of the linkage key 1351 in the circumferential direction (as shown in Fig. 29).

Preferably, as shown in Fig. 30, an open side of the linkage groove 211 is provided with a flared structure 213 to guide the insertion of the linkage key 1351. The linkage groove 211 is provided at the distal region of the first shaft 21. As shown in Figs. 28a, 28b, and 30, a tubular member 40 extending out of the base is fixed to the distal region of the first shaft 21. The linkage key 1351 is mounted around and abuts against the outer circumference of the tubular member 40, maintaining radial alignment between the locking member 13 and the base 10 and facilitating insertion into the linkage groove 211.

In one embodiment, as shown in Fig. 30, the linkage key 1351 is formed by deformation of a part of the tubular wall of the connecting portion 135. An avoidance zone 1353 is formed outside the linkage key 1351. This avoidance zone exists between the linkage key 1351 and the positioning portion 131. The detour section 117 of the pulling wire 11 can be placed within the avoidance zone 1353, facilitating the threading of the pulling wire 11.

The specific formation of the linkage key 1351 includes: as shown in Fig. 26, a pipe wall of the connecting portion 135 is configured with a pair of cutouts 1355 at a position near the proximal region face, and a part between the pair of cutouts 1355 bends radially and inwardly to form the linkage key 1351. The connecting portion 135 is a cylindrical structure, and the linkage key 1351 is sheet-shaped.

When the two mating portions are in the linkage state, the proximal region face of the first shaft 21 abuts against the base 10. The linkage key 1351 is located at either the proximal or distal region of the connecting portion 135.

In one embodiment, there are two to four linkage keys 1351 distributed circumferentially. For example, Fig. 26 shows two linkage keys 1351.

An embodiment of this application also provides a control mechanism having a distal region and a proximal region opposite to each other. The control mechanism includes:
at least three shafts, including a first shaft, a second shaft slidably mounted around the first shaft, and a third shaft slidably mounted around the second shaft;
a base connected to the distal region of the third shaft;
a pulling wire connected to the distal region of the second shaft, the pulling wire having a free end that can pass through the artificial implant or be detached from the artificial implant and a free end that has a first state in which it is restrained to the base and a second state in which it is released; and
a locking member connected to the distal region of the first shaft, the locking member movably matching with the base and cooperating with the free end of the pulling wire to switch the state of the free end.

For other structural details and operating methods of the control mechanism of this embodiment, please refer to other embodiments. For example, in the first state, the release process of the artificial implant is controlled by adjusting the length of the pulling wire exposed outside the base. The number of pulling wires can be multiple and the pulling wires are controlled by a control handle via a second shaft. In the second state, after the locking member is unlocked, the movement of the second shaft drives the pulling wire to detach from the artificial implant. In another embodiment, the proximal region of the pulling wire extends to and is directly controlled by the control handle.

An embodiment of this application also provides a delivery system, including a control handle and the control mechanisms described in the aforementioned embodiments, wherein the proximal regions of the three shafts are respectively connected to and controlled by the control handle.

The control handle includes a support body, on which drive mechanisms are respectively configured for the three shafts. To facilitate the delivery and retraction of the artificial implant, the control handle is also connected to a sheath tube that can enclose and release the artificial implant. To enhance positioning, in some embodiments, the artificial implant may configured as a structure similar to that shown in Fig. 10, which includes an inner frame 61 and arms 63 fixed to the outer circumference of the inner frame. After being released, each arm 63 extends into a corresponding valve sinus to prevent the artificial implant from shifting due to blood flow. During release, the arms are released first, and the inner frame is fully released after it has accurately entered the valve sinus. Accordingly, the distal region of the sheath tube includes a first loading section for enclosing the artificial implant. The first loading section opens toward the distal region. A second loading section is fixed to the distal region of the first shaft. The second loading section opens toward the proximal region and interlocks with the first loading section to enclose the artificial implant.

An embodiment of this application also provides a control method for an artificial implant based on wire control, including:
providing a control mechanism according to the aforementioned embodiment, wherein the free end of a pulling wire passes through the artificial implant and is restrained to the base by a locking member; wherein
the pulling wire has a control end opposite the free end, when the artificial implant is released, the control end moves to the distal region, causing a portion of the pulling wire exposed outside the base to be gradually lengthened, thereby allowing radial deformation of the artificial implant;
when the artificial implant deforms to a desired extent, the cooperation between the locking member and the base are unlocked to release the free end;
the control end moving to the proximal region, causing the free end to be detached from the artificial implant.

### Detailed description is provided with reference to the accompanying drawings:

After the artificial implant is delivered to a predetermined location within the body, the components within the sheath tube are first pushed distally to advance the entire implant, or the sheath tube is moved proximally to expose the arms and control mechanism. At this situation, the proximal region of the artificial implant is controlled by the control mechanism, while the distal region of the artificial implant is constrained by the second loading section 411.

As shown in Fig. 31a, the first shaft 21 is then pushed to release the restraint on the artificial implant from the second loading section. That is, the distal region of the artificial implant is released and thus expands radially and outwardly. During the movement of the first shaft to the distal region, the coupling with the connecting portion is achieved by rotating the linkage structure, that is, it is linked with the locking member for releasing and the proximal region of the artificial implant.

As shown in Fig. 31b, while the third shaft 25 remains stationary, the second shaft 23 is pushed to the distal region, causing the control end 113 of the pulling wire 11 to move toward the distal region 301. This makes a portion of the pulling wire 11 exposed outside the base be gradually lengthened, allowing the artificial implant 60 (primarily the proximal portion) to gradually expand upon release. The pulling wire outside the base 10 will be gradually lengthened in the direction of the arrow. The control end 113 continues to move until the artificial implant 60 is in the expanding state (i.e., the desired extent) as shown in Fig. 31c. Once the desired fit with the surrounding tissue is confirmed, the pulling wire can be released.

To release the pulling wires, as shown in Figs. 31d-31g, the first shaft 21 is rotated to drive the locking member 13 to rotate relative to the base 10, sequentially releasing the restraints on the pulling wires 11a, 11b, and 11c. As shown, the corresponding free ends 111a, 111b, and 111c are detached from the locking member 13.

Then, as shown in Fig. 31h, the second shaft 23 is withdrawn proximally, causing the control end 113 of each pulling wire to move toward the proximal region 302, which make the free ends be detached from the corresponding holes 603, i.e., detached completely form the artificial implant. Finally, as shown in Fig. 31i, the second loading section 411 is reengaged with the first loading section 431, and the control mechanism is withdrawn from the body.

In addition, before the pull wire is unlocked, an withdrawal operation can be performed as needed, and the order can be according to the corresponding operations shown in Figs 31c, 31b and 31a: driving the control end 113 of the pulling wire to move proximally, folding the proximal region of the artificial implant through the pull wire, and then moving the remaining components in the sheath tube proximally back into the sheath tube as needed, or pushing the sheath tube toward the distal region to accommodate the artificial implant.

It should be noted that the position of the axis in the drawings is not restrictive; the control end can be long or short, or the pulling wire can be directly connected to the control handle for easy retraction and extension.

For control of the pulling wire, the control end of the pulling wire may extend to and be directly controlled by the control handle.

The aforementioned control handle can be a conventional control handle. This disclosure provides another control handle for operating a catheter assembly to deploy an artificial implant. For details, please referring to the following description:

Referring to Figs. 32a and 32b, the control handle 3 has a distal region 301 and a proximal region 302 opposite to each other, and an axial direction extending between the proximal and distal regions. The catheter assembly 4 includes a sheath tube 43 and an inner shaft assembly 41 that slidably disposed within the sheath tube. Prior to release, the artificial implant is coupled to the inner shaft assembly 41 and enclosed within the sheath tube 43. When released, the artificial implant 60 is exposed outside the sheath tube 43.

The control handle 3 includes:
a first handle 31 for connecting to the sheath tube 43;
a second handle 32 for connecting to the inner shaft assembly, with the distal region of the second handle 32 movably inserted into the proximal region of the first handle 31.

Before the artificial implant 60 is released, the control handle 3 has a first length L1, as shown in Fig. 32a. During release, the second handle 32 extends further into the interior of the first handle 31, making the artificial implant 60 to be exposed outside the sheath tube 43. Accordingly, the control handle 3 has a second length L2, as shown in Fig. 32b, which is less than the first length L1.

The first handle 31 and the second handle 32 are axially movable relative to each other. As the artificial implant 60 is released, the distal region of the second handle 32 gradually extends into the interior of the first handle 31, gradually reducing the overall length of the control handle 3 until reaching the final second length, where the artificial implant 60 is fully exposed from the sheath tube 43, thereby the artificial implant 60 being fully released, or at least allowing for full release.

The relative movement of the first handle and the second handle along the axial direction can be achieved by either threaded transmission or direct transmission. In addition, although the two handles move relative to each other, during the operation, the first handle is basically fixed relative to the interventional path, and only the second handle is delivered distally, that is, the sheath tube remains fixed, and the internal inner shaft assembly and the artificial implant move distally. Compared with the method of withdrawing the sheath tube proximally, the distal region of the sheath tube is more stable, which is conducive to maintaining the direction of the distal region of the inner shaft assembly and avoiding radial deviation during the release process.

As shown in Figs. 33 and 34, the distal region of the second handle 32 is provided with a first transmission member 341. The first handle 31 includes:
a first support body 331, to which the proximal region of the sheath tube 43 is fixed;
a first drive sleeve 351, being rotatably mounted around the first support body 331, engaging with the first transmission member 341 in a threaded manner, the first drive sleeve 351 acting as a force-applying component to drive the second handle 32 (i.e., the inner shaft assembly 41) to slide axially.

For ease of understanding and subsequent explanation, the force-applying component is the component that the operator holds or moves to apply force, including:
a drive sleeve which is an internally threaded force-applying component that drives the second support body to slide as a whole, such as the first drive sleeve 351;
an annular body which is a rotatable force-applying component, and will be described in detail below.

This application also provides another control handle, as shown in Figs. 33 and 35. The control handle includes:
a first handle 31, configured for connecting the sheath tube 43;
a second handle 32, including a second support body 332 and a first transmission member 341, wherein the first transmission member 341 is axially slidably mounted on the first handle 31, and the distal region of the second support body 332 is rotatably engaged with the first transmission member 341 and axially linked;
an inner shaft assembly 41, with a proximal region being connected to the second support body 332 and a distal region being connected to a wire control assembly for controlling the release of the artificial implant.

The wire control assembly allows for more flexible operation of the artificial implant, controlling the release process and enabling retraction as needed. The entire wire control assembly as a whole is controlled by a second handle, which can both slide axially and rotate relative to the first handle. This multi-degree-of-freedom relative motion further adjusts the implant's spatial posture during release, ensuring proper alignment with surrounding tissue.

As shown in Figs. 36-37c, the inner shaft assembly 41 includes multiple shafts (wires, tubes, etc.) that are slidably mounted on each other from the inside to the outside. The wire control assembly 45 includes a base 10, a locking member 13, and a pulling wire 11, each of which is connected to a corresponding shaft. The locking member 13 is movably mounted on the base 10 and has a locking hole that mates with the locking member 13. One end of the pulling wire 11 is connected to the corresponding shaft, while the other end can pass through the artificial implant 60 and then be secured to the base 10 by the locking member 13. The specific connection relationships and structural features of the various components of the wire control assembly refer to the previous embodiments and will not be further described here.

The connection and control of the various components of the wire control assembly with their corresponding shafts are as follows:

The inner shaft assembly 41 includes:
a first shaft 21, with a locking member 13 at its distal region and a proximal region movably mounted on the second support body 332;
a second shaft 23, with a pulling wire 11 connected to its distal region and a proximal region movably mounted on the second support body 332; and
a third shaft 25, with a base 10 fixed to its distal region and a proximal region fixed to the second support body 332.

As shown in Fig. 38 (with the pulling wire omitted), the distal region of the sheath tube 43 has a first loading section 431 for enclosing the artificial implant 60. The first loading section 431 is open toward the distal region. A second loading section 411 is fixed to the distal region 301 of the first shaft 21. The second loading section 411 is open toward the proximal region 302 and interlocks with the first loading section 431 to enclose the artificial implant 60.

The artificial implant 60, taking an artificial aortic valve as an example, includes an inner frame, an outer frame, valve leaflets, and arms arranged on the outer frame. The artificial implant 60 has three states:
a compressed state, in which the first loading section 431 wraps around the outer frame and a proximal portion of the inner frame, while the second loading section 411 wraps around and restrains a distal portion of the inner frame.
a transition state, i.e., the first stage, in which the first loading section 431 moves relative to the artificial implant 60 to release the arms first, the prosthetic aortic valve as a whole enters a transition state to wait for adjustment of the alignment of each arm with the valve sinus and into position. In the first stage, if the proximal portion of the inner frame is completely exposed, it can be kept restrained by wire control to avoid excessive radial deformation that affects position adjustment, or the stroke of the first loading section 431 can be controlled so that the arm is released first (at least its head and most of the adjacent areas are released), and the proximal portion of the inner frame is still in a wrapped and restrained state;
a releasing state, i.e., the second stage, in which the second loading section 411 moves relative to the artificial implant 60 to release the distal portion of the inner frame.

In combination with the first shaft 21 of the aforementioned embodiment, the first shaft 21 icludes a first section 214 and a second section 216 (corresponding to the aforementioned tubular member 40) nested within each other. The proximal region of the first section 214 extends to and is controlled by the control handle 3. The proximal region of the second section 216 is fixed to the inner side of the first section 214, such as by adhesive bonding. The distal region of the second section 216 extends forward and is provided with a second loading section 411.

The first shaft 21 selectively engages the locking element 13 via a linkage structure. The linkage structure switches between different linkage states during axial movement of the first shaft 211. A linkage groove of the linkage structure is provided at the distal region of the first section 214, and the second section 216 extends into the first section 214 by a distance greater than the groove depth of the linkage groove to maintain the structural strength of the linkage groove.

In another embodiment, the diameter of the first shaft is uniform, and the linkage groove is provided only where linkage is required.

As shown in Fig. 39, in one embodiment, the first handle 31 includes a first support body 331 and a first drive sleeve 351 rotatably mounted on the outer circumference of the first support body.

An axially extending guide groove 3301 is provided within the first support body 331. A first transmission member 341 is slidably mounted within the guide groove 3301 and threadedly engages with the inner wall of the first drive sleeve 351.

A connecting head 3321 is provided at the distal region of the second support body 332. The first transmission member 341 defines a mounting hole 3401. The connecting head 3321 is inserted into the mounting hole 3401 and rotatably engages with it. A rotationally engaged and axially linked coupling structure is also provided between the connecting head 3321 and the inner wall of the mounting hole 3401.

By rotating the second handle 32, the second support body 332 is driven to rotate relative to the first support body 331, causing the wire control assembly at the distal region to rotate synchronously with the artificial implant to adjust the artificial implant's spatial posture.

Considering the radial arrangement order of the three shafts, the proximal regions of the three shafts extend axially as follows: as shown in Fig. 41, the proximal region of the third shaft 25 is fixed to the connecting head 3321, and the second shaft 23 and the first shaft 21 extend through the connecting head 3321 via the interior of the third shaft 25.

This application also provides another control handle, as shown in Figs. 40-42. The control handle 3 includes:
a first handle 31, which is used to connect to the sheath tube 43; and
a second handle 32, which is used to connect to the inner shaft assembly 41, wherein the distal region of the second handle 32 is configured as an insertion section 321 that is movably inserted into the first handle 31. When the second handle 32 moves distally relative to the first handle 31 to its limit position, the insertion section 321 is located within the first handle 31. The inner shaft assembly 41 includes a movable shaft 413 that is slidably engaged with the second handle 32. The movement path of the proximal region of the movable shaft 413 extends to the interior of the insertion section 321.

The movement path of the proximal region of the movable shaft 413 utilizes the interior space of the insertion section 321, minimizing the impact on the overall length of the control handle.

The insertion section 321 slides with the first handle 31 and provides sliding support for the second handle 32. During the sliding motion of the second handle 32, the outer circumference of the insertion section 321 is exposed. As shown in Fig. 43, the outer wall of the insertion section 321 is marked with distance markings 3211 to indicate the sliding travel of the second handle 32.

The movable shaft 413 may include the first shaft 21 and the second shaft 23 of the aforementioned embodiment.

This application provides another control handle, as shown in Figs. 41-43, including:
a first handle 31, which is used to connect to a sheath tube 43;
a second handle 32, which includes a second support body 332 and an inner shaft drive mechanism 36, the second support body 332 including a sleeve 333 and a guide rail 3302 fixed within the sleeve 333, a distal region of the sleeve 333 being movably inserted into an interior of the first handle (serving as an insertion section 321), and a proximal region of the sleeve 333 being fixed to a support frame 3331;
the inner shaft drive mechanism including:
   a plurality of inner cylinders 361 sleeved sequentially from the inside to the outside, each of which is rotatably mounted in a radial gap between the sleeve 333 and the guide rail 3302, the proximal regions of the inner cylinders 361 extending out of the sleeve 333, with the parts extending out of the sleeve serving as force-applying components for driving the inner cylinders 361, the force-applying components 362 being arranged in sequence along the axial direction through the support frame 3331; and
   a plurality of second transmission members 342 arranged axially and slidably mounted on the guide rail 3302, each second transmission member 342 engaging with a corresponding inner cylinder 361 in a threaded manner, the inner shaft assembly 41 including multiple movable shafts 413, the proximal region of each movable shaft 413 being axially linked to a corresponding second transmission member 342.

Each inner cylinder 361, sleeve 333, and guide rail 3302 are nested within one another, partially or completely overlapping in the radial direction, reducing the axial length of the second handle. The sleeve 333 and guide rail 3302 are inserted into the first handle 31, reducing the axial length of the control handle. The overlap distance is set based on the sliding travel of the corresponding movable shaft.

As shown in Figs. 43 to 45, the support frame 3331 is provided with multiple axially arranged partition plates 3333. Mounting zones 3332 are formed between adjacent partition plates 3333 for mounting the force-applying components 362, preventing mutual movement and operational interference between the force-applying components.

The support frame 3331 has a cylindrical outer contour for easy handling. Within the same mounting zone, a sidewall of the cylindrical structure is provided with multiple openings which are arranged at intervals in the circumferential direction to expose the force-applying components 362. Connecting plates 3334 are provided between adjacent openings to connect the partition plates 3333, so as to ensure the structural strength of the support frame 3331.

In Figs. 42 and 43, there are two second transmission members 342a and 342b connected to the first shaft 21 and the second shaft 23, respectively. Correspondingly, two inner cylinders 361a and 361b, and two force-applying components 362a (serving as the second force-applying component) and 362b (serving as the first force-applying component) are provided.

As shown in Figs. 45 and 46, the force-applying component 362 is cylindrical-shaped. The force-applying component 362 and the inner cylinder 361 are formed separately and then fixedly connected together. The radial dimension of the force-applying component 362 is smaller than that of the support frame 3331. This reduces the possibility of misoperation of the force-applying component while also facilitating operation, for example, the support frame 3331 can be held by the palm of the hand and the force-applying component can be rotated by the finger. Preferably, the radial dimension of the force-applying component 362 is smaller than the radial dimension of the front and rear partitions 3333.

Another method to prevent misoperation is to provide a corresponding locking structure to limit the movement of the force-applying component.

The outer circumference of the sleeve 333 is provided with the distance markings described in the aforementioned embodiment. The sleeve 333 and the guide rail 3302 are fixedly connected at the distal region and spaced apart at the proximal region, allowing each inner cylinder to be inserted and installed from the proximal region.

This application provides another control handle, as shown in Figs. 41 to 43. The control handle includes:
a first handle 31, which is used to connect to a sheath tube 43;
a second handle 32, which includes a second support body 332 and an inner shaft drive mechanism 36, the second support body 332 including a sleeve 333 and a guide rail 3302 fixed within the sleeve 333, a distal region of the sleeve 333 being movably inserted into the interior of the first handle;
the inner shaft drive mechanism 36 including:
   an inner cylinder 361, rotatably mounted within a radial gap between the sleeve 333 and the guide rail 3302, a proximal region of the inner cylinder 361 extending beyond the sleeve 333and serving as a force-applying component 362 for driving the inner cylinder 361; and
   a second transmission member 342, slidably mounted on the guide rail 3302 and threadedly engaged with the inner cylinder 361, the inner shaft assembly 41 including at least one movable shaft, a proximal region of which is axially linked to the second transmission member 342.

As shown in Figs. 47a and 47b, the distal region of the sleeve 333 is provided with a connecting head 3321. The first transmission member 341 defines a mounting hole 3401. The connecting head 3321 is rotatably inserted into the mounting hole 3401. A limit structure is provided between the connecting head 3321 and an inner wall of the mounting hole 3401 to maintain axial linkage. The limit structure includes end surfaces of the connecting head 3321 and the mounting hole 3401 that abut against with each other in the axial direction.

As shown in Figs. 47a, 47b, and 48, the guide rail 3302 and sleeve 333 are fixedly connected by a snap fastener. The connecting head 3321 is fixed to the distal region of the guide rail 3302, wherein the two components can be formed separately and then fixed together or integrally formed. The guide rail 3302 consists of two strip-shaped members 3303 arranged side by side, forming a slide groove (i.e., the aforementioned guide groove 3301) between them. At least a part of the second transmission member 342 is positioned between the two strip-shaped members 3303. The distal regions of the two strip-shaped members 3303 are fixed to each other via the connecting head 3321. The distal region of the sleeve 333 is mounted around the outer circumference of the connecting head 3321 and is fixed by a snap fastener. The proximal regions of the two strip-shaped members 3303 are fixed to each other, maintaining a stable spacing.

The inner shaft assembly includes:
a fixed shaft, a proximal region of which is fixedly connected to the second support body; and
a plurality of movable shafts being slidably sleeved inwardly and outwardly. All movable shafts in the inner shaft assembly slide through the interior of the fixed shaft and are controlled by the inner shaft drive mechanism. All second transmission members are arranged sequentially along the guide rail. All inner cylinders 361 slide and nest radially in sequence. The force-applying components of the inner cylinders are arranged axially at the distal region of the second support body 332.

From the inside to the outside, the inner shaft assembly 41 includes, in order, the first shaft 21, the second shaft 23, and the third shaft 25. The first and second shafts 21 and 23 are movable shafts, while the third shaft 25 is fixed. For example, as shown in Fig. 47b, the proximal region of the third shaft 25 is fixed to the connecting head 3321.

As shown in Fig. 48, a blocking member 3304 is provided on the guide rail 3302. The blocking member 3304 is located between two adjacent second transmission members to limit the sliding travel of the second transmission members. When the second transmission member reaches its limit position, it is stopped by an end surface of the blocking member 3304.

As shown in Fig. 49, the inner cylinder includes a first inner cylinder 361a and a second inner cylinder 361b located outside the first inner cylinder 361a. Both the first and second inner cylinders have internal threads that mate with the second transmission member.

The second transmission member includes two toothed structure arranged in sequence from the proximal region to the distal region. The second transmission member 342a at the proximal region is threadedly engaged with the first inner cylinder 361a and linked to the first shaft 21, while the second transmission member 342b at the distal region is threadedly engaged with the second inner cylinder 361b and linked to the second shaft 23.

The first and second shafts 21 and 23 are rotationally engaged and axially linked with the corresponding second transmission members 342. Specifically, the first inner cylinder 361a rotates, the second transmission member 342a slides axially, driving the first shaft 21 to slide; and the second inner cylinder 361b rotates, the second transmission member 342b slides axially, driving the second shaft 23 to slide.

As shown in Figs. 49 and 50, the distal region of the first inner cylinder 361a extends into the second inner cylinder 361b, and the proximal region of the first inner cylinder 361a extending beyond the second inner cylinder carries a first annular body 363, which serves as a force-applying component. The distal region of the second inner cylinder 361b extends into the sleeve 333, and the proximal region of the first second cylinder 361b extending beyond the sleeve carries a second annular body 364, which serves as a force-applying component. In other words, the inner cylinder and force-applying component are formed separately and then assembled together. During assembly, the first inner cylinder 361a is inserted into the second inner cylinder 361b, with the proximal region extending beyond the second inner cylinder 361b extending. The distal regions of two inner cylinders are then inserted into the sleeve 333, with the proximal regions extending out to the support frame 3331, and finally surrounded by the first annular body 363 and the second annular body 364.

A snap-fit structure is provided between the annular body and the inner cylinder to maintain the two in relative circumferential position. For example, as shown in Figs. 50 and 51, taking the second inner cylinder as an example, one of the second inner cylinder and the second annular body is provided with a retaining groove 3611, and the other is provided with a retaining ring 3641 that cooperates with the retaining groove 3611. The partition plates 3333 of the support frame abut against at least one axial end of the annular body to prevent the annular body from separating from the inner cylinder.

In one embodiment, a locking structure for rotationally mating components in a control handle of an artificial implant is provided, wherein the control handle includes a first component and a second component that are rotationally mated, and the locking structure includes:
an annular body connected to the first component and rotationally mated with the second component, gear teeth being formed on an inner edge of the annular body;
an operating button movably mounted on the second component, the operating button having a locking portion that matches with the gear teeth; and
an elastic member driving the operating button to move toward one side along the radial direction of the annular body to keep the locking portion and the gear teeth engaged and positioned. When the operating button is forced to move toward the other side along the radial direction of the annular body, the locking portion and the gear teeth are released.

As shown in Figs. 52, 53a, and 53b, the locking structure can be used to restrict or release relative rotation between the inner cylinder 361 and the sleeve 333. The first component is the inner cylinder 361, the second component is the sleeve 333, and the annular body is either the first annular body 363 or the second annular body 364.

In the drawings, taking the second annular body 364 as an example, the inner edge of the annular body is provided with gear teeth 3631. The operating button 366 is movably mounted on the sleeve 333 and configured with the locking portion 3661 that engages with the gear teeth 3631. The elastic member 367 is used to drive the operating button 366 to maintain the locking portion 3661 in engagement with the gear teeth 3631. The operating button is pressed to move radially and inwardly along the annular body, and the elastic member drives the operating button to move radially and outwardly along the annular body.

When locked, the inner cylinder 361 and the sleeve 333 are circumferentially fixed relative to each other, which can be understood as that the annular body is constrained by the sleeve and cannot rotate, or the annular body rotates synchronously with the sleeve 333. When unlocked, the annular body can independently drive the inner cylinder 361 to rotate relative to the sleeve 333. This locking structure is applicable to any structure with an annular body in this application.

The locking structure can also be used between the first handle 31 and the second handle 32. A third annular body 365 is provided at the proximal region of the first handle 31. The third annular body is mounted around the outer circumference of the sleeve 333, and synchronous rotation of the third annular body and the sleeve 333 is achieved through the matched cross-sectional shapes (non-circular) of the them. A locking structure is provided between the first handle 31 and the third annular body 365. The third annular body 365, the operating button 366, the elastic member 367, and etc., can be seen in Fig. 52 and 54, wherein the specific structure and matching method are the same as those of the above-mentioned locking structure.

In another embodiment, a control handle is provided, including a support body, a catheter assembly including multiple shafts slidably nested inside and outside, wherein the first shaft 21 is located at the innermost layer, a transmission sleeve 215 is connected to the proximal region of the first shaft 21, the transmission sleeve 215 and the first shaft 21 are slidably engaged in the axial direction and fixed in the circumferential direction;
the proximal region of the support body being rotatably mounted with:
a third force-applying component, which is fixed to the transmission sleeve 215, for driving the of the first shaft 21 to rotate; and
a second force-applying component, cooperating with the first shaft 21 to drive the first shaft 21 to slide in the axial direction.

The first shaft 21 can slide axially relative to the transmission sleeve 215 to achieve separation or combination of the distal region of the first shaft. The transmission sleeve 215 and the first shaft 21 are relatively fixed in the circumferential direction, and the two rotate differently under the drive of the third force-applying component to release or maintain the restriction on the pulling wire.

As shown in Fig. 55, the support body is the second support body 332 in the aforementioned embodiment, the second force-applying component is the first annular body 363, and the third force-applying component is the fourth annular body 368.

As shown in Fig. 56, the sidewall of the transmission sleeve 215 is provided with a sliding groove 2151 extending axially. The proximal region of the first shaft 21 is provided with a limiting member 2111 that protrudes radially and outwardly and engages into the sliding groove 2151. When the transmission sleeve 215 and the first shaft 21 slide axially relative to each other, the limiting member 2111 moves within the sliding groove 2151. When the transmission sleeve 215 rotates, a sidewall of the sliding groove abuts against the limiting member 2111, causing the first shaft 21 to rotate synchronously.

As shown in Fig. 57, the axial length of the transmission sleeve 215 is L1, and the axial length of the sliding groove 2151 is L2, wherein L2/L1 = 0.8-0.9. This satisfies the sliding travel of the first shaft 21 and minimizes the length of the transmission sleeve 215. If the transmission sleeve is too long, either its distal region extends further distally, which will inevitably affect the movement stroke of the second transmission member, or it extends backward, which will correspondingly increase the axial size of the control handle.

In one embodiment, a reinforcement tube 217 is mounted around the outer circumference of the transmission sleeve 215. The length of the reinforcement tube 217 is the same as that of the transmission sleeve 215. The proximal regions of the two strip-shaped members of the guide rail 3302 are axially connected, forming a mounting zone matching with the reinforcement tube 217. The axial length of the mounting zone is L3, wherein L1/L3 = 0.7-0.8.

The fourth annular body 368 is disposed at the proximal region of the sleeve 333 of the aforementioned embodiment. Specifically, as shown in Fig. 58, the proximal region of the sleeve 333 has a connecting seat 3335 extending axially therefrom and rotatably cooperating with the fourth annular body. The fourth annular body 368 is snap-connected to the connecting seat 3335, and a locking structure of the aforementioned embodiment is arranged between the fourth annular body 368 and the connecting seat 3335.

The proximal region of the fourth annular body 368 is provided with a connecting head, such as a Luer connecting head, which is communicated with an interior of the first shaft 21 for passing guide wires or injecting fluid, etc. The reinforcement tube 217 and the transmission sleeve 215 are inserted into and sealed with the fourth annular body 368.

In another embodiment, a control handle is provided, including:
a support body 330 to which the catheter assembly 4 is connected;
a transmission member 340 slidably mounted on the support body 330 and connected to one of the controlled members 415 in the catheter assembly 4;
a drive sleeve (i.e., the first drive sleeve 351) rotatably mounted on the support body 330 and configured with internal threads, the transmission member 340 being threadedly engaged with the internal threads of the drive sleeve;
a mounting hole for the controlled element 415 to pass through being defined in the transmission member 340, one radial side of the mounting hole being open to allow the controlled element 415 to be radially inserted and assembled.

The first drive sleeve 351 rotates to drive the transmission member 340 to move the controlled element 415 axially, thereby performing a corresponding operation on the distal region of the controlled element.

During assembly, the controlled element 415 is inserted into the transmission member 340 through the mounting hole 3401 and as a whole is mounted on the support body 330, and the support body 330 then closes the mounting hole 3401 to prevent the controlled element from being removed.

The controlled element 415 includes the first shaft 21, second shaft 23, and third shaft 25 of the aforementioned embodiment. The transmission member 340 includes a first transmission member 341 and a second transmission member 342, and the support body 330 includes a first support body 331 and a second support body 332. For ease of description, unless otherwise specified, in the following disclosure, the support body is the second support body, the transmission member is the second transmission member, and the controlled member is the second shaft or the first shaft, which will be described in detail with reference to the accompanying drawings.

As shown in Figs. 59 and 60, the support body includes two parallel guide rails 3302, with a guide groove 3301 defined between them. The transmission member 340 is provided with toothed structures 3402 extending out of the guide groove 3301 on its two radial sides, and the toothed structures 3402 (i.e., external threads) on two sides cooperate with the internal threads of the drive sleeve. The guide rail 3302 on at least one side closes the mounting hole 3401.

As shown in Figs. 59 to 62b, there are at least two toothed structures 3402 arranged circumferentially, and the number of the guide grooves 3301 corresponds to that of the toothed structures 3402. This ensures smoother transmission between the drive sleeve and the transmission member, reducing problems such as slippage and jamming during threaded transmission. In the illustrations, there are two toothed structures 3402 and two guide grooves 3301, both positioned opposite to each other.

The transmission member, where it extends out of the guide groove 3301, is provided with shoulders 3403 that mate with the guide rails 3302. The shoulders 3403 extend along the width of the transmission member and mate with the two guide rails 3302, serving as assembly limits and assisting in guiding movement during threaded transmission.

In the illustration, there are two toothed structures 3402, positioned opposite each other (symmetrically), namely a first toothed structure 3402a and a second toothed structure 3402b. The first toothed structure 3402a is connected to the shoulder 3403, increasing the threaded transmission area with the drive sleeve.

As shown in Figs. 61 and 63, the width of the first toothed structure 3402a is greater than that of the second toothed structure 3402b. Correspondingly, the width of the first guide groove 3301a is greater than that of the second guide groove 3301b. This facilitates the insertion and installation of the transmission member through the first guide groove 3301a and reduces the amount of slotting required in the support body, accordingly increasing the structural strength of the support body.

To facilitate installation, the first drive sleeve can be two shells that interlock with each other and engage with the transmission member, or it can be a cylindrical sleeve that axially extends outside the support body and engages with the transmission member.

In terms of axial dimension, the first toothed structure 3402a is equal to or larger than the second toothed structure 3402b, with the ratio being 1 to 2. For example, in Fig. 64a, the ratio is 1; in Fig. 64b, the ratio is 2.

As shown in Fig. 65, an axial positioning groove 3404 is provided on the inner edge of the mounting hole 3401, and a limiting block 4151 is fixed to the outer circumference of the proximal region of the control panel 415. The limiting block 4151 is rotatably inserted into the axial positioning groove 3404, thereby achieving rotational fit and axial linkage.

This disclosure provides another control handle, which includes a support body 330,
a bend adjusting drive mechanism 37 connected to the support body 330;
a bend adjusting member 28, one end of which extends to the distal region of the sheath tube 43 and acts on the sheath tube 43, and the other end of which is controlled by the bend adjusting drive mechanism.

The distal region of the bend adjusting member can be fixed to the distal region of the sheath tube 43 or movable relative to it. The bend adjusting member may be a wire, a tube, or a combination of a wire and a tube. For example, the bend adjusting member may be a bend adjusting wire fixed to the sheath tube, causing the sheath to bend; or the bend adjusting member may include a bend adjusting tube and a bend adjusting wire connected to the bend adjusting tube, wherein the bend adjusting wire drives the bend adjusting tube to bend the sheath tube.

As shown in Fig. 66, the distal region of the bend adjusting member 28 is fixed to the sheath tube 43, while the proximal region of the bend adjusting member 28 is controlled by the bend adjusting drive mechanism 37 and moves relative to it, causing the distal region of the bend adjusting member 28 to bend the sheath tube 43, thereby changing the travel path of the distal region of the delivery system.

As shown in Figs. 67-71, the bend adjusting drive mechanism includes:
a winding wheel 371 being rotatably mounted on the support body 330, wherein the proximal region of the bend adjusting member 28 is made of a flexible material and is wound around the winding wheel 371;
a knob 372 linked to the winding wheel 371;
a one-way latch structure, which interacts with the winding wheel 371 and/or the knob 372 and has a limiting state and a releasing state, wherein, in the limiting state, the winding wheel 371 is allowed to rotate only in one direction.

The bending degree is related to the amount of change in the bend adjusting member 28. At the proximal region, the bend adjusting member 28 rotates and winds around, reducing the axial length of the control handle 3 and increasing the bending degree. The one-way latch structure is in a limited position when no force is applied, which prevents accidental activation of the knob and limiting its rotation to maintain the directional orientation of the sheath tube 43.

The rotation axis of the winding wheel 371 is perpendicular to the axial direction of the control handle. The winding wheel 371 rotates in a first direction for bend adjusting, and rotates in a second direction under the action of the bend adjusting member 28 to reset. The winding wheel 371 rotates unidirectional in the first direction, and the one-way latch structure prevents the winding wheel 371 from rotating in the second direction until it is disengaged.

In one embodiment, as shown in the figures, the one-way latch structure includes:
a ratchet 3711, coaxially fixed to the winding wheel 371, for example, the ratchet 3711and the winding wheel 371 being an integral structure;
a limiting member 374, movably mounted on the support body 330 and having a pawl 3741 that engages with the ratchet;
an elastic member 375, acting on the limiting member 374 to cause the pawl 3741 to engage with the ratchet 3711; and
a drive assembly 376, movably mounted on the support body 330 and cooperating with the limiting member 374, configured for driving the limiting member 374 to move, making the one-way latch structure be locked or unlocked.

The drive assembly 376 at least partially extends out of the support body 330 to the control handle for operator operation.

As shown in the drawings, the limiting member 374 is rotatably mounted on the support body 330. The drive assembly 376 is configured as a toggle button 3761 fixed to the limiting member 374. The limiting member 374 and the toggle button 3761 may be formed integrally or formed separately and then fixed together. During normal operation, the one-way latch structure is switched to the released state by toggling the toggle button 3761, and when the toggle button 3761 is released and reset under the action of the elastic member 375, it switches to the limiting state.

If the limiting member 374 fails to return to its original position, for example due to a failure of the elastic member 375, this uncontrolled state can also be determined by the toggle button 3761 failing to return to its original position after release, resulting in a free-moving state. In this case, the toggle button 3761 is controlled to directly act on the limiting member 374 to control the one-way locking mechanism. This serves as an emergency measure to improve surgical safety.

The front end of the support body 330 is provided with a mounting seat 334, which has a mounting groove 3341 for the bend adjusting member 28 to extend into. One side of the mounting groove 3341 is open, allowing for the placement of the limiting member and the drive assembly.

The winding wheel 371 is partially inserted into the support body 330, and an accommodating groove is defined in a portion of the winding wheel 371 that is inserted into the support body 330 for winding of the bend adjusting member 28. The winding wheel 371 partially extends outside the support body 330 and is fixedly connected to the ratchet 3711. Correspondingly, the drive assembly is configured as a toggle button 3761 and partially received in the mounting groove, and the limiting member 374 is located outside the support body 330.

The knob 372 is connected to the winding wheel 371 snap-fit connection and passes through the support body 330.

This disclosure provides a connecting structure between a sheath tube 43 and a control handle, as shown in Figs. 72-74. The connecting structure includes:
a connecting head 311, fixed to the proximal region of the sheath tube 43;
an end cap 312 mounted around an outer circumference of the connecting head 311, wherein anti-rotation structures that cooperate with each other are provided between the end cap 312 and the connecting head 311; and
a mounting portion 313, located at the distal region of the control handle (i.e., the aforementioned first handle 31), the mounting portion 313 defining a mounting channel 3131 being open radially at one side, an outer circumference of the mounting portion 313 being configured with a radial groove 3132, a proximal region of the end cap 312 being slidably engaged into the mounting groove 3132, and the mounting portion 313 and the end cap 312 clamping and fixing the connecting head 311 in the axial direction.

The anti-rotation structures include a radial groove 3132 and a retaining groove formed on the end cap 312 and mating with the radial groove 3132. The radial groove 3132 and the retaining groove are plug-fitted in the radial direction to limit the circumferential and axial movements.

As shown in Fig. 72, the control handle includes a support body (the aforementioned first support body) on which the connecting head 311 is mounted, and an outer shell 380 mounted around the support body. The outer shell 380 includes two interlocking halves. The outer shell 380 includes a limiting portion 381 that surrounds the connecting portion between the end cap 312 and the connecting head 311, preventing the end cap 312 from slipping off the connecting head 311.

As shown in Figs. 73 and 74, the control handle includes a limiting portion 381 mounted around the outer shell and a retaining ring 314 mounted around the end cap 312, which limits the separation of the two halves of the outer shell 380. A limiting structure is provided between the retaining ring 314 and the end cap 312 to limit the circumferential rotation of the retaining ring 314.

This disclosure provides a delivery system for operating a catheter assembly to deploy an artificial implant, including a control handle and a catheter assembly. The control handle has a distal region and a proximal region opposite to each other, and an axial line extending between the proximal and distal regions. The proximal region of the catheter assembly is connected to and controlled by the control handle. The catheter assembly includes:
a second shaft, a pulling wire connected to a distal region of the second shaft;
a first shaft, a locking member linked to a distal region of the first shaft; and
a third shaft, a base fixed to a distal region of the third shaft, and the locking member movably mounted to the base, the base configured with a locking hole matching with the locking member;
wherein the pulling wire is bound to the base by the locking member after winding around the artificial implant through the second shaft, and the proximal regions of the second shaft and the first shaft are slidably matched with the third shaft.

The specific structures of the catheter assembly and the control handle may refer to the aforementioned embodiments. The following describes the operation of the delivery system in detail, specifically combining variations of the control handle outside the body and the catheter assembly inside the body:

The distal region of the delivery system is extended into the body. In this case, the artificial implant 60 is enclosed by the first loading section 431 and the second loading section 411 (as shown in Fig. 75).

When the distal region portion reaches the position as shown in Fig. 76a, the knob 372 is rotated in the first direction, and the distal region of the bend adjusting member acts on the sheath tube 43 to bend it, as shown in Fig. 77. During this process, the knob 372 is constantly adjusted. When the bending amplitude is too large, the toggle button is turned while simultaneously the knob is rotated in the second direction to reduce the amplitude. Continue adjusting until the position is passed and the predetermined position as shown in Fig. 76b is reached.

After reaching the predetermined position, as shown in Fig. 78, the first drive sleeve 351 is rotated to move the second handle 32 distally. Inside the body, as shown in Figs. 79a and 79b, the remaining portion of the catheter assembly, excluding the sheath tube 43, moves distally, the artificial implant 60 is freed from the restraint of the first loading segment 431, and its outer frame and/or arms 63 expand and release. At this time, the distal region of the inner frame 61 is constrained by the second loading segment 411, and the proximal region is constrained by the wire control assembly and is basically in a compressed state.

The spatial posture of the artificial implant is adjusted, including the axial direction (in the direction indicated by the distal region of the sheath) and the circumferential direction. As shown in Fig. 80, the specific operation includes: pressing the operating button 366 on the third annular body 365 to release the locking structure, rotating the second handle 32, and observe with external equipment to determine whether the arms are correctly positioned after release. In the body, as shown in Fig. 81, the catheter assembly, except for the sheath tube 43, rotates synchronously with the second handle. After confirmation, the operating button is released and the first drive sleeve 351 is rotated to make the arms extend into the corresponding sinuses.

As shown in Fig. 82, when the operating button 366 corresponding to the first annular body 363 is pressed to release the restriction of the locking structure, the first annular body 363 is rotated, and the first shaft 21 is driven to move distally to the first stroke, as shown in Fig. 83, inside the body, the inner frame 61 is freed from the restraint from the second loading section 411 and released.

When the first annular body 363 is continued to rotate to drive the first shaft 21 to move distally to the second stroke, the distal region of the first shaft 21 is combined with the locking member 13. Here, the second transmission member connected to the first shaft 21 may be restrained by the guide rail, or the distal region face of the first shaft 21 may be restrained by the base, releasing the operating button to limit the rotation of the first annular body 363.

The release of the wire control end of the artificial implants begins with:

As shown in Fig. 84, press he operating button 366 corresponding to the second annular body 364 to release the restriction of the locking structure, rotate the second annular body 364, push the second shaft 23 toward the distal region, and move the control end 113 of the pulling wire 11 toward the distal region 301, so that the part of the pulling wire 11 exposed outside the base is gradually lengthened, allowing the artificial implant 60 (mainly the proximal portion) to gradually expand outward after release, and the pulling wire outside the base 10 will be gradually lengthened in the direction of the arrow. The control end 113 continues to move until reach the state as shown in Fig. 85, the artificial implant 60 is in the releasing state (i.e., the expected amplitude). After confirming that it is in the expected fit with the surrounding tissue, the operating button 366 is released to limit the rotation of the second annular body 364 and prepare to release the pull wire, or perform withdrawal operation of the artificial implant.

When releasing the pulling wire, as shown in Fig. 86, the operating button 366 corresponding to the fourth annular body 368 is pressed to release the restriction of the locking structure, the fourth annular body 368 is rotated, and the first shaft is driven to rotate. As shown in Fig. 87a to Fig. 87d, the locking member 13 is driven to rotate and disengaged from the locking region, and the restrictions of the pulling wire 11a, 11b, and 11c are released in turn, and the corresponding free ends 111a, 111b, and 111c in the drawings are completely detached from the locking member 13.

Next, the operating button 366 corresponding to the second annular body 364 is pressed to release the locking structure and rotate the second annular body 364. The second shaft 23 is driven to retract proximally, as shown in Fig. 87e, causing the control ends 113 of the pulling wires to move toward the proximal region 302, allowing the free ends to disengage from the corresponding holes 603, i.e., the free ends are completely detached from the artificial implant 60;

The first drive sleeve 351 is rotated to move the second handle proximally, as shown in Fig. 87f, causing the second loading section 411 to engage with the first loading section 431 and then be withdrawn from the body.

Referring to Figs. 88a to 89d, the artificial implant 60 in the present application, taking an artificial heart valve 700 as an example, generally includes a deformable stent 701 and leaflets 710 connected to the stent 701. The stent 701 is processed using corresponding materials according to different release modes, such as nickel-titanium alloy with shape memory that can self-expand in the body, or stainless steel that can be released by ball expansion, etc. The stent 701 can be formed by cutting a tube or weaving a wire, and the leaflets 710 can be connected to the stent 701 by sewing, bonding or integral molding.

The stent 701 is generally cylindrical, with a side wall being configured as a hollow mesh structure. Unless otherwise stated, the shape or size of the mesh structure is not strictly limited. In order to position it in the body, a positioning structure that can interact with the surrounding native tissue can be set on the circumference of the stent 701. In this application, the stent 701 includes an inner frame 720 and an outer frame 750, and the outer frame 750 is mainly used as a positioning structure. In order to prevent peripheral leakage, a skirt or anti-peripheral leakage material can be set on the inner and/or outer sides of the inner frame 720.

During surgery, a delivery system is generally used to deliver and operate artificial implants. The delivery system generally includes a control handle 808 and a catheter assembly 800, which includes multiple controlled members. The catheter assembly 800, or the entire delivery system, has a proximal region 801 located near the operator, and a distal region 802 opposite to the proximal region 801. The distal regions 802 of the controlled members cooperate with each other to operate the artificial implant, such as releasing, withdrawal, positioning, and adjusting its spatial posture. The proximal region 801 of each controlled member is connected to and controlled by the control handle 808. vThe controlled member may be a hollow tube, a solid rod, a flexible wire, or a combination of these. There are multiple controlled members, and at least two (for example, the proximal region 801) of them can slide relative to each other along the axial direction or rotate relative to each other about the axis. The force-applying members (the portion directly contacted by the user) on the control handle 808 (the part that the user directly operates and contacts), being used to operate the controlled members, can be directly fixed to the corresponding controlled members for transmission, or can be transmitted by means of threads, gear and racks, etc..

Referring to Figs. 89a to 89d, in order to facilitate understanding and illustrate the structural features of the present application, the following embodiments take aortic intervention as examples. The aortic valve 600 has three native leaflets 601, with sinuses 602 located between each leaflet 601 and the surrounding vascular wall. According to normal blood flow, the artificial heart valve 700 has an axial direction and inflow and outflow sides opposite to each other in the axial direction, wherein X1 represents the inflow side, X2 represents the outflow side, and the direction of the arrow represents the blood flow direction.

In one embodiment of the present application, the artificial heart valve 700 includes an inner frame 720, leaflets 710 and an outer frame 750. The outer frame 750 can be positioned in the body at the location of the sinus 602. The inner frame 720 and the outer frame 750 can accommodate the native leaflets 601. The outer frame 750 can also be pressed against the bottom of the sinus 602 to play an axial and circumferential positioning role, thereby preventing the artificial heart valve 700 from being displaced under the action of the blood flow.

The inner frame 720 and outer frame 750 can be formed separately and then fixed together or integrally formed. The inner frame 720 and the outer frame 750 are integrally formed by cutting tubular blanks. Along the axial direction of the tubular blank, the inflow sides of the inner frame 720 and outer frame 750 are spaced apart from each other. The inner frame 720 is a radially deformable cylindrical structure with a blood flow channel defined therein. The leaflets 710 are connected to the inner frame 720 to adjust the degree of opening of the blood flow channel. The outer frame 750 is an integral annular structure, including multiple arms 753 arranged along the circumferential direction. The outflow side of each arm 753 is fixedly connected to the inner frame 720, and the inflow side is located around the inner frame 720 and forms a first gap 751 for clamping native tissue with the inner frame 720. In this embodiment, the native tissue specifically refers to the native leaflet 601.

The leaflets 710 include multiple leaflets that cooperate with each other, and the number and circumferential position of the arms 753 match the corresponding leaflets 710. In this embodiment, the number of leaflets 710 and arms 753 are both three, wherein the leaflets 710 are made of biomaterial or polymer material.

The outer frame 750 is an integral annular structure that can be integrally formed during processing, ensuring support strength and simplifying assembly with the inner frame 720.

In this embodiment, taking the self-expansion release of a prosthetic heart valve as an example, the release and retraction of the prosthetic heart valve can be controlled by a sheath tube 806 mounted around the stent 701. The stent 701 can be controlled by varying the portion of stent 701 which it is exposed outside of sheath tube 806. Alternatively, the stent 701 can be controlled by a pulling wire. That is, the pulling wire passes through structural gaps (or wire holes) in the stent 701, and a degree of expansion of the stent can be adjusted by adjusting the length of the pulling wire exposed outside the control mechanism. When the pulling wire is removed from stent 701, the stent 701 is fully released. Of course, the control of the pulling wire can also accomplished through various controlled components within the catheter assembly 800. To further fix the stent, the catheter assembly 800 includes a mounting head 805 that mates with stent 701. The outer circumference of mounting head 805 is configured with a locking slot 807. When the stent 701 is disengaged from the locking slot 807 of the mounting head 805, it is fully released.

Referring to Figs. 90a-90d, the artificial heart valve 700 is delivered to a designated position or withdrawn via a catheter assembly 800, specifically, released or withdrawn via a sheath tube806. Depending on the degree of radial deformation, the artificial heart valve 700 has the following states:
a compressed state: both the inner frame 720 and the outer frame 750 are radially compressed and fit within the sheath tube 806, with the inflow side of the outer frame 750 close to the outer circumference of the inner frame 720;
a transitional state: the inflow side of the outer frame 750 expands radially outward relative to the outer circumference of the inner frame 720, while at least a portion of the inner frame 720 remains radially compressed. In this state, a gap is formed between the outer frame 750 and the outer wall of the inner frame 720, allowing the native leaflets 601 to enter;
a releasing state: both the inner frame 720 and the outer frame 750 expand radially to approach a predetermined shape formed by heat setting. In the body, at least one native leaflet 601 is typically clamped between the outer frame 750 and the inner frame 720.

In this embodiment, both the inner frame 720 and the outer frame 750 switch from the compressed state to the releasing state through self-expansion.. To fully utilize the positioning function of the outer frame 750, the delivery system can be configured with at least two catheter assemblies 800, each carrying a loading section at its distal region. The open sides of the two loading sections face each other. When the loading sections are snap-fitted with each other, the artificial implant is enclosed. The loading section is the sheath tube 806 mentioned above. The loading section 803 at the proximal region 801 encloses the outer frame 750 and the proximal region portion of the inner frame 720, while the loading section 804 at the distal region 802 encloses and constrains the distal region portion of the inner frame 720. Releasing of the inner frame includes at least two stages.

In the first stage, the loading section 803 at the proximal region 801 moves relative to the artificial implant, exposing and releasing the arms 753 first. The artificial heart valve 700 as a whole enters the transitional state. After the arms 753 are aligned and positioned with the sinuses 602, the next stage proceeds. During the first stage, if the proximal portion of the inner frame 720 is fully exposed, a wire control method can be used to maintain restraint to prevent excessive radial deformation that could affect position adjustment. Alternatively, the loading section can be controlled to release the arms 753 first (at least the heads 7533 and most of the adjacent area), while the proximal portion of the inner frame 720 remains wrapped and restrained.

In the second stage, the loading section 804 at the distal region 802 moves relative to the artificial implant, releasing the distal region portion of the inner frame 720. This releases the restraints on the proximal region 801 of the inner frame 720, allowing the entire artificial implant to enter the releasing state.

Referring to Fig. 90e, the artificial heart valve 700 generally includes a connecting structure that mates with the catheter assembly 800. For example, at least one of the axial ends of the inner frame 720 may include a connecting lug 703 that mates with the delivery system. The connecting lug 703 may be integrally formed with the inner frame 720 or the outer frame 750. The connecting lugs may be T-shaped or have holes 705 for threading the pulling wires. The T-shaped connecting lugs can be used to engage with the locking slots 807 in the compressed state, while holes 705 can be used directly to secure the pulling wires or mate with the locking slots 807 with corresponding constructions.

The connecting lugs configured on the inner frame 720 and/or outer frame 750 define their positions to prevent unwanted displacement during delivery. During release, the catheter assembly 800 releases its restraint on the stent 701 within the body, allowing the stent 701 to radially expand and then assume a released state. Unless otherwise specified, descriptions of the shape of the artificial heart valve 700 (such as the inner frame 720 or outer frame 750) in this application refer to that of the artificial heart valve in the released state, and the force of the surrounding tissues is not taken into account.

### Embodiment 1

Referring to Figs. 91a through 91e, an artificial heart valve 700 is provided in one embodiment, including an inner frame 720, leaflets 710 and an outer frame 750. The outer frame 750 is an integrated annular structure, including a plurality of arms 753 arranged circumferentially. The outflow side of each arm 753 is fixedly connected to the inner frame 720, and the inflow side is located around the inner frame 720, and a first gap 751 is formed between the inflow side of the arm 753 and the inner frame 720 for clamping native tissue.

The outflow sides of two adjacent arms 753 are close to each other to form a junction 752. The junction 752 extends radially inward from the outer side of the outer frame 750 to the inner side of the inner frame 720 and is adhered and fixed to the inner wall 1 of the inner frame 720. When withdrawal the stent 701, the sheath tube 806 gradually moves from the outflow side to the inflow side and receives the stent 701. Because the junction 752 is fixed to the inner wall of the inner frame 720 (i.e., the outer circumference of the inner frame 720 does not have any protruding nodes fixed to the outer frame 750), the sheath tube 806 can smoothly transition, avoiding interference between the junction 752 and the end of the sheath tube 806.

In the present application, the diameter of the inner frame 720 gradually decreases and then gradually increases from the outflow side to the inflow side, wherein the diameter of the inner frame 720 at the inflow side is less than that at the outflow side, and the junction 752 is located in the middle position of the inner frame 720, where the diameter of the inner frame 720 is the smallest.

From the outflow side to the inflow side, the arm 753 includes a root portion 7531, a middle portion 7532, and a head portion 7533. In the released state, the circumferential span of the arm 753 gradually narrows from the root portion 7531 to the head portion 7533, and an isolation gap 754 is formed between the middle portion 7532 and the outer circumference of the inner frame 720.

The arm 753 gradually narrows from the root portion 7531 to the head portion 7533. For example, the arm 753 in whole is a U-shaped frame, which can avoid interference with the joint of the two adjacent native leaflets **601,** and facilitate the head portion 7533 to extend into and abut against the bottom of the sinus 602. The isolation gap 754 is formed by the protrusion of the middle portion 7532 toward the outer circumference, which absorbs the deformation of the native leaflet 601 as much as possible in the radial direction to avoid blockage of the coronary artery after excessive deformation of the native leaflet 601.

The head portion 7533 abuts the outer circumference of the inner frame 720, providing a more effective clamping force between the inner frame 720 and the outer frame 750 in the released state. To enhance clamping force, the head portion 7533 can abut against the outer circumference of the inner frame 720 and form a pre-tightening force therebetween.

In this embodiment, the outer frame 750 is formed of frame bars, and the frame bars of the head portion 7533 are undulations. The head portion 7533 is wrapped with a protective film, which can be made of pericardium material to prevent the outer frame 750 from puncturing the blood vessels and the native leaflets 601. The undulations also facilitate the fixation of the protective film to prevent it from slipping.

The protective film is typically secured to the head portion 7533 using wire ties. In one embodiment, the frame bar of the head portion 7533 has anti-slip grooves 7534 provided on its surfaces to facilitate wire ties and positioning. The number of anti-slip grooves 7534 can be adjusted as needed; for example, two anti-slip grooves 7534 are provided on each side of the frame bar of the head portion 7533.

The inner frame 720 has a mesh structure, with each mesh formed by strips. The junction 752 has a bifurcated structure extending toward the outflow side. In this embodiment, the bifurcated structure is specifically bifurcated, with each branch extending along the frame bar of the inner frame 720 and tending to bulge at the ends, for example, forming a T-shaped structure. The node portion of the bifurcated structure is provided with a suture hole 755, which has a positioning function. The inner frame 720 and the outer frame 750 are fixed to each other by binding wires passing through the suture hole 755, and the binding wires are also wrapped and tied to sew each branch and the T-shaped structure at the end.

The outer frame 750 is secured to the inner frame 720 via the bifurcated structure, preventing stress concentration and resulting disconnection at the end joints. Furthermore, the bifurcated structure is easily foldable, eliminating the difficulty of compressing and loading of this area. The ends of the T-shaped structure form a "cross" with the strip of the inner frame 720, facilitating the wrapping of binding wires.

### Embodiment 2

Referring to Figs. 92a-92e, an artificial heart valve 700is provided by another embodiment, including an inner frame 720, leaflets 710, and an outer frame 750. The outer frame 750 is an annular structure integrally formed as one piece, including multiple arms 753 arranged circumferentially. The outflow side of each arm 753 is fixedly connected to the inner frame 720, while the inflow side is located around the inner frame 720. A first gap 751 is formed between the inflow side of the arm 753 and the inner frame 720 for clamping native tissue.

During placement of the artificial heart valve 700 to the lesion, the blood vessel may be curved. Therefore, when the axial length L of the inner frame 720 is long, adjusting the curvature becomes more difficult, requiring a high level of operator skill. To solve this issue, in the released state, the inner frame 720 of this application is cylindrical, with a ratio of the axial length L to the diameter D of (0.8-1.5):1. This results in a shorter overall axial length L, making it easier for the operator to adjust the curvature and center the valve.

In the released state of the present application, the inner frame 720 is roughly in the shape of a straight cylinder.

Both the inner frame 720 and the outer frame 750 are configured with integral connecting lugs, which nest with each other.

The outflow sides of two adjacent arms 753 are adjacent to each other to form a junction 752. The connecting lug 703 of the inner frame 720 is located at the vertex of the outflow side. The connecting lug 702 of the outer frame 750 extends from the junction 752 and is annular in shape, mounted around the connecting lug 703 of the inner frame 720. The annular inner region of the connecting lug 702 of the outer frame 750 matches the shape of the connecting lug 703 of the inner frame 720. For example, in Fig. 92c, the connecting lug 703 of the inner frame 720 is T-shaped, and the corresponding inner shape of the annular region is also T-shaped. Due to the relatively small diameter of the catheter assembly 800, the acceptable depth of the locking slot 807 on the mounting head 805 is limited. In this embodiment, the structure of the connecting lugs can avoid the problem that the thickness is too thick to enter the locking slot 807. During installation, the connecting lugs of the inner frame 720 and the outer frame 750 are nested and then fixed by welding or riveting, making them not easy to fall off.

In this embodiment, the end portion of the connecting lay is bent radially inward, thereby preventing the connecting lug from scratching the blood vessels and causing damage to the blood vessels.

The inner frame 720 has multi-turns of mesh structures arranged along the axial direction. The mesh on the outflow side is concave hexagons. The sides of the concave hexagon facing the outflow side, such as the first frame bar 756 and the second frame bar 757, are V-shaped and concave toward the inflow side. The multi-circles of mesh structures are a closed-loop structure in the circumferential direction, which is more evenly stressed. When the inner frame 720 is released or withdrawn, the leaflets 710 are relatively stable, avoiding the problem of shortened life of the leaflets 710 due to uneven stress. In addition, when the concave hexagon is in a compressed state, the first frame bar 756 and the second frame bar 757 are folded in half and stored inside the concave hexagon, avoiding the simultaneous extension of the axial ends of the mesh after compression, thereby relatively reducing the overall length of the compressed inner frame 720.

The arm 753 includes a root portion 7531, a middle portion 7532 and a head portion 7533 from the outflow side to the inflow side. In the released state, the circumferential span of the arm 753 gradually narrows from the root portion 7531 to the head portion 7533, and the head portion 7533 is arc shaped to avoid injuring the native leaflet 601.

### Embodiment 3

Referring to Figs. 93a to 93g, an artificial heart valve 700 is provided in another embodiment, including an inner frame 720, leaflets 710, and an outer frame 750. The outer frame 750 includes a plurality of arms 753 arranged circumferentially. The outflow side of each arm 753 is fixedly connected to the inner frame 720, while the inflow side is located around the inner frame 720, forming a first gap 751 between the inner frame 720 and the outflow side of each arm 753 for clamping native tissue. The arm 753, from the outflow side to the inflow side, includes a root portion 7531, a middle portion 7532, and a head portion 7533. In the released state, the circumferential span of the arm 753 gradually narrows from the root portion 7531 to the head portion 7533.

In this embodiment, the inner frame 720 and outer frame 750 are integrally formed as one piece, enhancing their structural strength. The inner and outer frames 720 and 750 are connected at the outflow side by an S-shaped transition portion 704, which has a span in both the circumferential direction and the radial direction of the inner frame 720.

The advantage of the S-shape of the transition portion 704 is that the circumferential span prevents interference with the junctions of adjacent native leaflets 601 when clamping the native leaflets **601,** thus preventing the root portion 7531 from expanding too much circumferentially and making it difficult to enter the valve sinus 602. Furthermore, the radial span increases the expansion tendency of each head portion 7533 in the outer frame 750 during the transition state, making it easier to enter the sinus during positioning.

As can be seen in Fig. 93b, the circumferential span of the arm 753 gradually narrows from root portion 7531 to head portion 7533, being U-shaped in whole. Two ends of the U-shaped arm 753 are connected to the inner frame 720 via corresponding transition sections 704 (i.e., S-shaped structures), respectively. For the three arms 753, six S-shaped structures are provided.

For each S-shaped structure, a first span W1 is defined along the circumference of the inner frame 720. Two S-shaped structures are separated on the outflow side between adjacent arms 753, meaning the span between adjacent arms 753 is at least twice the length of W1. To optimize the overall configuration of the arm 753, the central angle γ corresponding to the first span W1 is between 7° and 13°, preferably 9°.

The transition section 704 has a second span W2 along the radial direction of the inner frame 720, ranging from 0.7 mm to 1.6 mm.

The transition sections 704 between two adjacent arms 753, i.e., the two S-shaped structures, intersect each other and further extend toward the outflow side to form a connecting lug 702 that cooperates with the delivery system. In this embodiment, the connecting lug 702 is T-shaped.

The inner frame 720 has a mesh structure, with a plurality of mesh vertices arranged circumferentially on the inflow side of the inner frame 720, wherein at least two mesh vertices have a tendency to further extend axially relative to other mesh vertices on the inflow side.

This can also be understood that the mesh vertices at the inflow side are divided into two groups based on their axial extension lengths, resulting in a two-stage release of the stent when the sheath tube 806 moves, depending on the timing of exposure. For example, during release, as the sheath tube 806 in Fig. 93c moves downward, the shorter group of mesh vertices is released first (the dashed line in Fig. 93c represents the critical state of the two-stage release). As the sheath tube 806 moves further, the longer group of mesh vertices is released later. This two-stage release process avoids the safety hazard of excessive deformation of the inner frame 720 during a single full release, ensuring a more stable posture of the stent 701 during release, preventing damage to the vessel wall, and minimizing interference with the left bundle branch.

To minimize the radial dimension after compression, in one embodiment, the inner frame 720 includes multi-circles of mesh structures arranged axially. Within at least one circle of the mesh structure, two opposing nodes of the same mesh on the circumference of the inner frame 720 are staggered along the axial direction of the inner frame 720. When the nodes of adjacent meshes in the inner frame 720 are compressed, it is difficult to bundle them neatly due to the existence of the branch structure. Therefore, arranging two relative nodes axially staggered along the inner frame 720 can avoid mutual interference, make full use of the circumferential space, and correspondingly reduce the diameter of the inner frame 720 after compression.

When implanting the artificial heart valve 700, the approach path is typically tortuous. For example, during intervention through the aorta 600, the bend in the aortic arch may cause that: the axis of the stent 701is relatively inclined relative to the midline of the native valve annulus when the artificial heart valve 700 is aligned with the native valve. This causes the arms 753 on the inner side of the bend to reach and enter the valve sinus 602 first, while the remaining arms 753 have not yet reached their corresponding positions. As the stent 701 continues to be delivered distally, the arm 753 that has already abutted the bottom of the valve sinus 602 may puncture the sinus floor.

In one embodiment, an improvement over other embodiments is that the axial lengths of the arms 753 are not identical. Based on the actual interventional approach, the operator can rotate the stent to an appropriate angle during centering, so that the arms 753 of varying lengths correspond to their respective native valve leaflets 601. Of course, one arm 753 which has the smallest axial length corresponds to the sinus 602 located on the inner side of the bend aortic arch during the lesion. During surgery, the angle and position of the stent 701 can be determined by combining the directional bend adjusting control of the catheter assembly 800 with real-time imaging equipment.

The root portion 7531 abuts against the outer circumference of the inner frame 720, with the axial length of the abutment portion being at least one-fifth of the total length of the arm 753. In the released position, this abutment portion provides a certain clamp on the native valve leaflets 601, enhancing the stability of the stent 701 during positioning.

### Embodiment 4

Referring to Figs. 94a to 94f, an artificial heart valve 700 is provided in another embodiment, including an inner frame 720, leaflets 710, and an outer frame 750. The outer frame 750 is an annular structure formed integrally, with multiple arms 753 arranged circumferentially. The inflow side of each arm 753 is located around the inner frame 720, forming a first gap 751 between the inner frame 720 and the inflow side of each arm 753 for clamping native tissue. The arm 753, from the outflow side to the inflow side, includes a root portion 7531, a middle portion 7532, and a head portion 7533. The arm 753 is fixed to the inner frame 720 via the middle portion 7532. In the released state, the circumferential span of the arms 753 gradually narrows from the root portion 7531 to the head portion 7533.

The outflow sides of two adjacent arms 753 are close to each other to form a junction 752, which is secured to the inner frame 720 via the junction 752. The outflow side of the junction 752 is provided with a connecting lug 702 for mating with the delivery system. Specifically, the outer frame 750 provides the connecting lugs 702, which can be tilted inward to prevent damage to the blood vessels during release. Furthermore, because the connecting lugs 702 and the junction 752 are integrally formed, the sheath tube moves smoothly from the connecting lugs 702 toward the inflow side during withdrawal, preventing interference between the outer frame 750 and the sheath tube 806.

The root portion 7531 and the head portion 7533 tilt outward relative to the middle portion 7532, with this tilting trend being away from the middle portion 7532 and away from the axis of the inner frame 720. Specifically, in the released state, the root portion 7531 tilts outward as a whole relative to the axis of the inner frame 720, at an angle γ. This tilting trend increases relative to the transition state (in this embodiment, the angle δ between the middle portion 7532 and the root portion 7531 remains constant). Accordingly, the radial spacing between the head portion 7533 and the outer circumference of the inner frame 720 gradually decreases from the transition state to the released state.

During the transitional state, the head portion 7533 is released first. At this state, the root portion 7531 is still radially constrained and roughly parallel to the axis of the inner frame 720. Since the root portion 7531 is everted in the predetermined shape (i.e., the released state), when the root portion 7531 is parallel to the axis of the inner frame 720, the head portion 7533 will be driven to have a more obvious everted tendency, i.e., the head portion 7533 can easily enter the sinus.

With respect to the transition state, after the stent is completely released, the root portion 7531 is released and turned outward, which drives the head portion 7533 to retract inward and further clamp the native leaflet 601.

The junction 752 and the outflow side of the inner frame 720 merge to form a common mesh 760. The inner frame 720 provides the lower half of the common mesh 760, while the junction 752 provides the upper half of the common mesh 760. These upper and lower halves are fixedly connected via nodes along the circumference of the inner frame 720. The connection manner through the nodes increases the overlap between the inner frame 720 and the outer frame 750, ensuring uniform force distribution and improving fatigue resistance. It also avoids the problem of excessive overlap leading to compression difficulties. Of course, the method of fixed connection is not limited in this application, which may be any one of riveting, welding and sewing.

In one embodiment, the nodes on the outflow side of the common mesh 760 are configured with connecting lugs 702 that mate with the conveying system. These connecting lugs 702 can be considered to be provided by the outer frame 750. In the figure, these connecting lugs 702 are T-shaped, and there are three of them evenly distributed along the circumference.

### Embodiment 5

Referring to Figs. 95a to 95e, an artificial heart valve 700 is provided in another embodiment, including an inner frame 720, leaflets 710, and an outer frame 750. The outer frame 750 is an annular structure formed integrally, including multiple arms 753 arranged circumferentially. The outflow side of each arm 753 is fixedly connected to the inner frame 720, while the inflow side is located around the inner frame 720, forming a first gap 751 between the inner frame 720 and the outflow side of each arm 753 for clamping native tissue. The arm 753, from the outflow side to the inflow side, includes a root portion 7531, a middle portion 7532, and a head portion 7533. In the released state, the circumferential span of the arms 753 gradually narrows from the root portion 7531 to the head portion 7533.

The inner frame 720 has multi-circles of mesh structures arranged axially, with the mesh structure on the outflow side being more sparse than that on the inflow side.

The sparseness of the mesh may be fewer meshs per unit area, or the meshs on the outflow side being longer in the axial direction than those on the inflow side. The connecting lug 703 of the present application is provided on the outflow side, and the sparse mesh structure on the outflow side is easy to deform, so the inner frame 720 can be retracted into the sheath tube from this side even when a relatively small force is applied.

The mesh structure of the inner frame 720 on the outflow side consists of two parallelograms arranged side by side with a common edge. The number of parallelograms in the circumferential direction corresponds to the number of connecting lugs 703, both being six. The common edge extends along the axial direction of the inner frame 720. The opposite edges adjacent to this common edge in the circumferential direction of the inner frame 720 extend circumferentially and also have an axial orientation, with the same offset direction.

Referring to Fig. 95e, when the parallelogram and the prism have the same side length L1, opposite edges of the parallelogram adjacent to this common edge in the circumferential direction are offset toward the outflow side, resulting in an elongation of L2 during compression. In contrast, a conventional prism-shaped mesh structure compresses both sides by 2*L2. This parallelogram-shaped mesh reduces the elongation of the inner frame 720 during compression, thereby reducing the shortening rate.

In this embodiment, the mesh structure on the outflow side is more sparse than that on the inflow side. Using a single parallelogram as a reference, there are 12 parallelograms along the circumference, while a circle of rhombuses on the inflow side utilizes 12 rhombuses. In the axial direction of the inner frame 720, the parallelograms are longer and have a larger area than the rhombuses, resulting in an overall sparse structure.

The dense rhombus structure on the inflow side can provide relatively strong support in the radial direction, making the stent 701 at the inflow side less likely to deform and more stable. In addition, the side length of the rhombus structure is relatively short, so the elongation after compression is relatively small, that is, the shortening rate is smaller.

### Embodiment 6

Referring to Figs. 96a to 96g, an artificial heart valve 700 is provided in another embodiment, including an inner frame 720, leaflets 710, and an outer frame 750. The outer frame 750 is an annular structure formed integrally, with multiple arms 753 arranged circumferentially. The outflow side of each arm 753 is fixedly connected to the inner frame 720, while the inflow side is located around the inner frame 720, forming a first gap 751 between the inner frame 720 and the outflow side of the arm 753 for clamping native tissue. The arm 753, from the outflow side to the inflow side, includes a root portion 7531, a middle portion 7532, and a head portion 7533. In the released state, the circumferential span of the arms 753 gradually narrows from the root portion 7531 to the head portion 7533.

The edges of the leaflets 710 include a fixed edge 711 connected to the inner frame 720 and a movable edge 712 that cooperates with the leaflets 710 to control blood flow. The fixed edge 711 is closer to the inflow side than the arm 753. The outflow side of the inner frame 720 has a radially outwardly enlarged portion 761, with the fixed edge 711 located substantially on the inflow side of the enlarged portion 761.

The leaflets 710 are configured as valve-in-ring structures, positioned axially in the middle of the inner frame 720 (resulting in a relatively low height). This shortens the stent 701 and reduces the risk of coronary artery interference. After implantation of the artificial heart valve 700 of this embodiment at the lesion site, the fixed edge 711 is closer to the inflow side than the arm 753, placing the leaflets 710 approximately at the same height as the native leaflets 601, that is, the leaflets 710 replacing the native leaflets 601 at that location without affecting other valve structures.

The inner frame 720 has a mesh structure, with the distance between the bottom of the fixed edge 711 and an edge of the inflow side of the inner frame 720 corresponding to half a mesh. The bottom of the valve is closer to the inflow side of the inner frame 720, further reducing the overall length of the stent 701.

The movable edges 712 of adjacent leaflets 710 intersect at the joint 762 of the inner frame 720. The inner frame 720 also extends at least one mesh on the outflow side of the joint 762, serving as the end mesh 763. The overlap between the inner frame 720 and the outer frame 750 is located on the outflow side of the end mesh 763. A membrane 764 is fixed inside the end mesh 763, which is composed of adjacent leaflets 710 assembled together and sewn onto the end mesh 763 with a thread, further reinforcing the overlapping position of the inner frame 720 and the outer frame 750, reducing the risk of movement and detachment between the inner frame 720 and the outer frame 750.

The inner wall and/or outer wall of the inner frame 720 can be covered with the membrane 764 to enclose the side walls of the inner frame 720, so that when the inner frame 720 and the outer frame 750 cooperate to clamp the native valve leaflets 601, it can reduce the impact on the valve leaflets 710 and increase the service life of the artificial heart valve 700.

The outflow side of the inner frame 720 has multiple pointed corners distributed along the circumference, each of which is fixed with a hole structure 705 for threading the pulling wire. In the figure, the hole structure 705 is evenly distributed in the circumference and has 6 holes. It is easy to release or withdrawal through wire control.

Each arm 753 is U-shaped, with the outflow sides of adjacent arms 753 being adjacent to each other to form a junction 752, wherein the arm is fixe to the inner frame 720 via the junction 752. Specifically, the junction 752 overlaps and fixes the frame bars of the mesh structure, which can better fix the membrane 764.

The vertex of the junction to form a V-shaped structure towards the outflow side. Multiple positioning holes 759 are arranged at the connection of the junction 752 and the arm 753, and corresponding positioning holes 759 are arranged at the corresponding parts of the inner frame 720. The inner frame 720 and the outer frame 750 are fixed to each other by binding wires passing through the positioning holes 759.

The control mechanism of this application can cooperate with each other to control the release or withdrawal process of artificial implants in the body, and the structure has been further optimized and improved. And, the outer frame of the artificial heart valve is an integrated annular structure, which is easy to process and can ensure strength. In addition, improvements have been made to the shape of the inner and outer frames and the connection method between the inner and outer frames, and corresponding technical effects have been obtained.

The technical features of the above embodiments may be combined in any manner. For the sake of brevity, not all possible combinations of the technical features of the above embodiments are described. However, as long as these combinations do not conflict, they should be considered within the scope of this disclosure. When technical features from different embodiments are shown in the same figure, the Fig. should be considered to also disclose examples of combinations of the various embodiments involved.

The above embodiments merely illustrate several implementation methods of this application. While the descriptions are relatively specific and detailed, they should not be construed as limiting the scope of the patent application. It should be noted that a person skilled in the art could make numerous variations and improvements without departing from the spirit of this application, all of which fall within the scope of protection of this application.

## Claims

1. A control mechanism for an artificial implant, comprising:
a base;
a pulling wire having a free end capable of passing through the artificial implant or being detached from the artificial implant;
a locking member movably engaged with the base, the locking member cooperating with the free end of the pulling wire to lock the artificial implant.

2. A control mechanism for an artificial implant, comprising:
a base having a locking region;
a pulling wire having a free end that can pass through the artificial implant or be detached from the artificial implant;
a locking member rotatably engaged with the base, the locking member having a positioning portion that moves into or out of the locking region during rotation, the positioning portion cooperating with the free end of the pulling wire to lock the artificial implant.

3. A control mechanism for an artificial implant, comprising:
a base;
a pulling wire having a free end that can pass through the artificial implant or be detached from the artificial implant;
a locking member rotatably engaged with the base, the locking member cooperating with the free end of the pulling wire to lock the artificial implant;
wherein the artificial implant has an axial direction, one end of the artificial implant in the axial direction is configured as a wire control end, the wire control end defines holes for extending of the pulling wire; and the artificial implant has the following states based on its degree of deformation:
a folding state, in which the wire control end radially contracts toward the base; and
an expanding state, in which the wire control end radially expands away from the base;
wherein a round-trip path of the same pulling wire winding around the artificial implant is not repeated.

4. A control mechanism for an artificial implant, comprising:
a base having a distal region and a proximal region opposite to each other, and an axial direction extending between the distal and proximal regions, wherein an interior of the base defines a first cavity, a first opening communicating with the first cavity is defined in a proximal region side of the base, and a second opening communicating with the first cavity is defined in an outer circumference of the base;
a pulling wire having a free end that can pass through the artificial implant or be detached from the artificial implant, one end of the pulling wire extending proximally out of the base through the first opening, and the other end, i.e., the free end of the pulling wire extending out of the base through the second opening for connecting the artificial implant; and
a locking member rotatably engaged with the base, the locking member cooperating with the free end of the pulling wire to lock the artificial implant.

5. A control mechanism for an artificial implant, comprising:
a base configured with a locking hole oriented in a circumferential direction of the base,
a pulling wire having a free end that can pass through the artificial implant or be detached from the artificial implant; and
a locking member rotatably engaged with the base, the locking member cooperating with the free end of the pulling wire to lock the artificial implant.

6. A control mechanism for an artificial implant, having a distal region and a proximal region opposite to each other and an axial direction extending between the proximal and distal regions, wherein the control mechanism comprises comprises:
an intervention component, comprising a first intervention component and a second intervention component;
a transmission member, a distal region of which is fixedly connected to the first intervention component;
a control handle, connected to and controlling a proximal region of the transmission member; and
a linkage structure, comprising two selectively linked mating portions, one of which is fixed to the second intervention component and the other is fixed to the transmission member, such that a linkage state of the two mating portions is switched during movement of the transmission member.

7. A control mechanism for an artificial implant, having a distal region and a proximal region opposite to each other, comprising:
three shafts, comprising a first shaft, a second shaft, and a third shaft slidably sleeved sequentially from the inside to the outside;
a base connected to a distal region of the third shaft;
a pulling wire connected to a distal region of the second shaft, the pulling wire having a free end that can pass through the artificial implant or be detached from the artificial implant; and
a locking member connected to a distal region of the first shaft, the locking member movably matched with the base, and the locking member cooperating with the free end of the pulling wire to lock the artificial implant.

8. A delivery system, comprising a control handle and a control mechanism as defined in claim 7, the proximal ends of the three shafts are connected to and controlled by the control handle, respectively.

9. A control method for an artificial implant based on wire control, comprising:
providing the control mechanism defined in any one of claims 1-7, wherein the free end of a pulling wire passes through the artificial implant and is restrained to the base by the locking member;
wherein the pulling wire has a control end opposite the free end, when the artificial implant is released, the control end is moved to gradually extend a portion of the pulling wire exposed outside the base, allowing the artificial implant to deform radially;
when the artificial implant has deformed to a desired extent, the cooperation between the locking member and the base is released and the free end is thus unlocked; and
moving the control end to withdraw the free end from the artificial implant.

10. A control handle for operating a catheter assembly to deploy an artificial implant, the control handle having a distal region and a proximal region opposite to each other, and an axial direction extending therebetween; the catheter assembly comprising a sheath tube and an inner shaft assembly that slidably disposed within the sheath tube; the artificial implant before release being coupled to the inner shaft assembly and wrapped by the sheath tube; the artificial implant during release exposed outside the sheath tube; wherein
the control handle comprises:
a first handle for connecting to the sheath tube; and
a second handle for connecting to the inner shaft assembly, a distal region of the second handle being movably inserted into a proximal region of the first handle; wherein
before release of the artificial implant, the control handle has a first length; during release of the artificial implant, the second handle further extends into the interior of the first handle to expose the artificial implant outside the sheath tube, and the control handle accordingly has a second length that is less than the first length.

11. A control handle for operating a catheter assembly to deploy an artificial implant, the control handle having a distal region and a proximal region opposite to each other, and an axial direction extending therebetween; the catheter assembly comprising a sheath tube and an inner shaft assembly that slidably disposed within the sheath tube; the artificial implant before release being coupled to the inner shaft assembly and wrapped by the sheath tube; the artificial implant during release exposed outside the sheath tube; wherein
the control handle comprises:
a first handle connected to the sheath tube; and
a second handle comprising a second support body and a first transmission member, wherein the first transmission member is axially slidably mounted on the first handle, and the distal region of the second support body rotatably matches with and axially links with the first transmission member;
the proximal region of the inner shaft assembly is connected to the second support body, and the distal region of the inner shaft assembly is connected to a wire control assembly for controlling the release of the artificial implant.

12. A control handle for operating a catheter assembly to deploy an artificial implant, the control handle having a distal region and a proximal region opposite to each other, and an axial direction extending therebetween; the catheter assembly comprising a sheath tube and an inner shaft assembly that slidably disposed within the sheath tube; the artificial implant before release being coupled to the inner shaft assembly and wrapped by the sheath tube; the artificial implant during release exposed outside the sheath tube; wherein
the control handle comprises:
a first handle for connecting the sheath tube; and
a second handle for connecting the inner shaft assembly, wherein the distal region of the second handle is configured as an insertion section that is movably inserted into the first handle, when the second handle is moved distally relative to the first handle to a limit position, the insertion section is located within the first handle; the inner shaft assembly comprises a movable shaft that slidably disposed within the second handle, and the proximal region of the movable shaft has a motion path extending into the interior of the insertion section.

13. A control handle for operating a catheter assembly to deploy an artificial implant, the control handle having a distal region and a proximal region opposite to each other, and an axial direction extending therebetween; the catheter assembly comprising a sheath tube and an inner shaft assembly that slidably disposed within the sheath tube; the artificial implant before release being coupled to the inner shaft assembly and wrapped by the sheath tube; the artificial implant during release exposed outside the sheath tube; wherein
the control handle comprises:
a first handle for connecting the sheath tube;
a second handle comprising a second support body and an inner shaft drive mechanism, the second support body comprising a sleeve and a guide rail fixed within the sleeve, the distal region of the sleeve being movably inserted into the interior of the first handle, and a support frame being fixed to the proximal region of the sleeve;
wherein the inner shaft drive mechanism comprises:
a plurality of inner cylinders being nested sequentially from the inside to the outside, each cylinder being rotatably mounted in the radial gap between the sleeve and the guide rail, the proximal region of each inner cylinder extending beyond the sleeve and serving as a force-applying component for driving this inner cylinder, the force-applying components being arranged at intervals along the axial direction through the support frame;
a plurality of second transmission members being axially arranged and slidably mounted on the guide rail, each second transmission member being threadedly engaged with a corresponding inner cylinder, the inner shaft assembly comprising a plurality of movable shafts, and the proximal region of each movable shaft being axially linked to the second transmission member.

14. A control handle for operating a catheter assembly to deploy an artificial implant, the control handle having a distal region and a proximal region opposite to each other, and an axial direction extending therebetween; the catheter assembly comprising a sheath tube and an inner shaft assembly that slidably disposed within the sheath tube; the artificial implant before release being coupled to the inner shaft assembly and wrapped by the sheath tube; the artificial implant during release exposed outside the sheath tube; wherein
the control handle comprises:
a first handle connected to the sheath tube; and
a second handle comprising a second support body and an inner shaft drive mechanism, wherein the second support body comprises a sleeve and a guide rail fixed within the sleeve, and the distal region of the sleeve is movably inserted into the interior of the first handle;
wherein the inner shaft drive mechanism comprises:
an inner shaft, being rotatably mounted in a radial gap between the sleeve and the guide rail, the proximal region of the inner shaft extending beyond the sleeve and serving as a force-applying component for driving the inner shaft;
a second transmission member, being slidably mounted on the guide rail and threadedly engaged with the inner shaft, wherein the inner shaft assembly comprises at least one movable shaft, the proximal region of which is axially linked to the second transmission member.

15. A control handle for operating a catheter assembly to deploy an artificial implant, the control handle having a distal region and a proximal region opposite to each other, and an axial direction extending therebetween; the catheter assembly comprising a sheath tube and an inner shaft assembly that slidably disposed within the sheath tube; the artificial implant before release being coupled to the inner shaft assembly and wrapped by the sheath tube; the artificial implant during release exposed outside the sheath tube; wherein
the control handle comprises:
a support body, the catheter assembly comprises multiple shafts that are slidably mounted on each other from the inside to the outside, the innermost shaft is a first shaft, a transmission sleeve is connected to the proximal region of the first shaft, the transmission sleeve and the first shaft are slidably engaged in the axial direction and constrained in the circumferential direction;
wherein the proximal region of the support body is rotatably mounted with:
a third force-applying component fixed to the transmission sleeve to drive the first shaft to rotate; and
a second force-applying component, in transmission engagement with the proximal region of the first shaft, driving the first shaft to slide axially.

16. A control handle for operating a catheter assembly to deploy an artificial implant, the control handle having a distal region and a proximal region opposite to each other, and an axial direction extending therebetween; the catheter assembly comprising a sheath tube and an inner shaft assembly that slidably disposed within the sheath tube; the artificial implant before release being coupled to the inner shaft assembly and wrapped by the sheath tube; the artificial implant during release exposed outside the sheath tube; wherein
the control handle comprises:
a support body to which the catheter assembly is connected;
a transmission member slidably mounted on the support body and connected to a controlled member of the catheter assembly; and
a drive sleeve rotatably mounted on the support body and configured with internal threads, the transmission member and the internal threads of the drive sleeve being threadedly engaged with each other;
wherein the transmission member is configured with a mounting hole for the controlled member to pass through, a radial side of the mounting hole being open to allow the controlled member to be radially inserted and assembled.

17. A locking structure for rotary mating components in a control handle for an artificial implant, the control handle having a distal region and a proximal region opposite to each other, and an axial direction extending therebetween; the catheter assembly comprising a sheath tube and an inner shaft assembly that slidably disposed within the sheath tube; the artificial implant before release being coupled to the inner shaft assembly and wrapped by the sheath tube; the artificial implant during release exposed outside the sheath tube; wherein
the control handle comprises:
a first component and a second component that are rotatably engaged with each other, and the locking structure comprises:
an annular body connected to the first component and rotatably engaged with the second component, gear teeth being formed on an inner edge of the annular body;
an operating button movably mounted on the second component, the operating button being configured with a locking portion that engages with the gear teeth; and
an elastic member driving the operating button to move toward one side along a radial direction of the annular body, maintaining the engagement and positioning of the locking member and the gear teeth; and the engagement between the locking member and the gear teeth being released when the operating button is pressed to move inward toward another side along the radial direction of the annular body.

18. A control handle for operating a catheter assembly to deploy an artificial implant, the control handle having a distal region and a proximal region opposite to each other, and an axial direction extending therebetween; the catheter assembly comprising a sheath tube and an inner shaft assembly that slidably disposed within the sheath tube; the artificial implant before release being coupled to the inner shaft assembly and wrapped by the sheath tube; the artificial implant during release exposed outside the sheath tube; wherein
the control handle comprises:
a support body;
a bend adjusting drive mechanism connected to the support body; and
a bend adjusting member, one end of which extends to the distal region of the sheath tube and acts on the sheath tube, and the other end of which is controlled by the bend adjusting drive mechanism.
Optionally, the bend adjusting drive mechanism comprises:
a winding wheel rotatably mounted on the support body, the proximal region of the bend adjusting member being made of a flexible material and wound around the winding wheel;
a knob linked with the winding wheel;
a one-way latch structure that interacts with the winding wheel and/or the knob and has relative engaged and disengaged states, in the engaged state, the winding wheel is allowed to rotate in only one direction.

19. A connecting structure between a sheath tube and a control handle, the control handle having a distal region and a proximal region opposite to each other, and an axial direction extending therebetween; the catheter assembly comprising a sheath tube and an inner shaft assembly that slidably disposed within the sheath tube; the artificial implant before release being coupled to the inner shaft assembly and wrapped by the sheath tube; the artificial implant during release exposed outside the sheath tube; wherein
the connecting structure comprises:
a connecting head fixed to the proximal region of the sheath;
an end cap mounted around an outer circumference of the connecting head, anti-rotation structures that cooperate with each other being provided between the end cap and the connecting head; and
a mounting portion located at the distal region of the control handle, the mounting portion defining a mounting channel therein, one radial side of the mounting channel being open, a radial groove being defined on an outer circumference of the mounting portion, the proximal region of the end cap slidably engaged into the radial groove, the proximal region of the sheath tube being engaged into the mounting channel, the mounting portion and the end cap clamping and fixing the connecting heat in the axial direction.

20. A delivery system for operating a catheter assembly to deploy an artificial implant, comprising a control handle and a catheter assembly, the control handle having a distal region and a proximal region opposite to each other, and an axial direction extending therebetween; the proximal region of the catheter assembly being connected to and controlled by the control handle, wherein
the catheter assembly comprises:
a second shaft, a pulling wire being connected to a distal region of the second shaft;
a first shaft, a locking member being linked to a distal region of the first shaft;
a third shaft, a base being fixed to a distal region of the third shaft, the locking member movably mounted to the base, the base having a locking hole configured to engage with the locking member;
the pulling wire winding around the artificial implant through the second shaft and then secured to the base by the locking member, the proximal regions of the second shaft and the first shaft being slidably engaged with the third shaft.

21. An artificial heart valve having an axial direction and an inflow side and an outflow side that are opposed to each other in the axial direction, wherein the artificial heart valve comprises:
an inner frame being configured as a radially deformable cylindrical structure with a blood flow channel defined therein;
valve leaflets, connected to the inner frame to adjust an opening degree of the blood flow channel; and
an outer frame being integrally formed as an annular structure with multiple arms arranged circumferentially, outflow sides of the arm being fixedly connected to the inner frame, inflow sides of the arms being located around the inner frame, first gaps being defined between the inner frame and the inflow sides of the arms for clamping native tissue;
wherein the outflow sides of two adjacent arms approach each other to form a junction, and the junction extends inwardly to an inner side of the inner frame, being adhered and fixed to an inner wall of the inner frame.

22. An artificial heart valve having an axial direction and an inflow side and an outflow side that are opposed to each other in the axial direction, wherein the artificial heart valve comprises:
an inner frame being configured as a radially deformable cylindrical structure with a blood flow channel defined therein;
valve leaflets, connected to the inner frame to adjust an opening degree of the blood flow channel; and
an outer frame being integrally formed as an annular structure with multiple arms arranged circumferentially, outflow sides of the arm being fixedly connected to the inner frame, inflow sides of the arms being located around the inner frame, first gaps being defined between the inner frame and the inflow sides of the arms for clamping native tissue;
wherein the arm comprises, from the outflow side to the inflow side, a root portion, a middle portion, and a head portion, in a released state, a circumferential span of the arm gradually narrows from the root portion to the head portion, and a separation gap is defined between the middle portion and an outer circumference of the inner frame.

23. An artificial heart valve having an axial direction and an inflow side and an outflow side that are opposed to each other in the axial direction, wherein the artificial heart valve comprises:
an inner frame being configured as a radially deformable cylindrical structure with a blood flow channel defined therein;
valve leaflets, connected to the inner frame to adjust an opening degree of the blood flow channel; and
an outer frame configured with multiple arms arranged circumferentially, outflow sides of the arm being fixedly connected to the inner frame, inflow sides of the arms being located around the inner frame, first gaps being defined between the inner frame and the inflow sides of the arms for clamping native tissue;
wherein the inner frame and outer frame are integrally formed, and connected to each other through an S-shaped transition portion at the outflow side, and the transition portion has spans in both the circumferential and radial directions of the inner frame.

24. An artificial heart valve having an axial direction and an inflow side and an outflow side that are opposed to each other in the axial direction, wherein the artificial heart valve comprises:
an inner frame being configured as a radially deformable cylindrical structure with a blood flow channel defined therein;
valve leaflets, connected to the inner frame to adjust an opening degree of the blood flow channel; and
an outer frame being integrally formed as an annular structure with multiple arms arranged circumferentially, outflow sides of the arm being fixedly connected to the inner frame, inflow sides of the arms being located around the inner frame, first gaps being defined between the inner frame and the inflow sides of the arms for clamping native tissue;
wherein the outflow sides of two adjacent arms approach each other to form a junction, the arms are fixed to the inner frame via the junctions, and the outflow side of the junction is provided with a connection lug for cooperating with a delivery system.

25. An artificial heart valve having an axial direction and an inflow side and an outflow side that are opposed to each other in the axial direction, wherein the artificial heart valve comprises:
an inner frame being configured as a radially deformable cylindrical structure with a blood flow channel defined therein;
valve leaflets, connected to the inner frame to adjust an opening degree of the blood flow channel; and
an outer frame being integrally formed as an annular structure with multiple arms arranged circumferentially, outflow sides of the arm being fixedly connected to the inner frame, inflow sides of the arms being located around the inner frame, first gaps being defined between the inner frame and the inflow sides of the arms for clamping native tissue;
wherein the arm comprises, from the outflow side to the inflow side, a root portion, a middle portion, and a head portion, in a released state, the root portion in whole is inclined outward relative to an axis of the inner frame; compared to the transition state, the inclined tendency of the root in the released state increases; and, correspondingly, a radial distance between the head portion and an outer circumference of the inner frame gradually decreases from the transition state to the released state.

26. An artificial heart valve having an axial direction and an inflow side and an outflow side that are opposed to each other in the axial direction, wherein the artificial heart valve comprises:
an inner frame being configured as a radially deformable cylindrical structure with a blood flow channel defined therein;
valve leaflets, connected to the inner frame to adjust an opening degree of the blood flow channel; and
an outer frame being integrally formed as an annular structure with multiple arms arranged circumferentially, outflow sides of the arm being fixedly connected to the inner frame, inflow sides of the arms being located around the inner frame, first gaps being defined between the inner frame and the inflow sides of the arms for clamping native tissue;
wherein the inner frame has multi-turns of mesh structures arranged along the axial direction, the mesh at the outflow side being more sparse than that at the inflow side; and
wherein one turn of mesh structure on the outflow side of the inner frame comprises two parallelograms arranged side by side and sharing a common edge, the common edge extends along the axial direction of the inner frame, opposite edges at two sides of the common edge along a circumferential direction of the inner frame offset toward the outflow side, respectively.
